# EUROPEAN PATENT APPLICATION

(11) **EP 4 725 952 A1**
(43) Date of publication of application: **15.04.2026**
(21) Application number: 24205590.3
(22) Date of filing: 09.10.2024
(51) Int. Cl.: C07K 5/02, C07K 14/245, C12P 21/02, C12N 15/00

(54) **COMPOUNDS COMPRISING NON-CANONICAL AMINO ACIDS AND USES THEREOF**

(71) Applicant: ETH Zurich, 8092 Zurich (CH)
(72) Inventor: LANG, Kathrin, 8049 Zürich (CH); FOTTNER, Maximilian, 8102 Oberengstringen (CH); IYPE, Tarun, 8046 Zürich (CH)

(57) **Abstract**

Provided herein are compounds comprising an isopeptide-linked lysine or an analog thereof. Further provided herein are methods for engineering peptide-binding proteins having increased affinity and/or selectivity for the compound according to the invention, as well as peptide-binding proteins with increased selectivity for the compound according to the invention. Moreover, methods for incorporating non-canonical amino acids into proteins are provided herein.

## Description

### BACKGROUND TO THE INVENTION

Co-translational incorporation of non-canonical amino acids (ncAAs) into proteins via genetic code expansion (GCE) has become a versatile approach to increase the chemical space of the proteome. By leveraging orthogonal aminoacyl-tRNA synthetases (aaRSs), a plethora of diverse functionalities(1) have been incorporated into proteins of interest (POIs) across all domains of life, most commonly in response to the amber nonsense codon (amber suppression). Site-specific encoding of functionalities such as post-translational modifications (PTMs), bioorthogonal handles, crosslinking moieties, spectroscopic probes, and photocaged amino acids offers a wide range of tools for studying and modifying protein functions and generating proteins with potential therapeutic and biotechnological significance.(2, 3)

While GCE holds promise in a wide range of fields, its widespread use in commercial and research applications remains limited due to several challenges, most notably low protein production yields. This stems from insufficient enzymatic activities of orthogonal aaRSs towards many ncAA substrates, as well as unfavorable competition of aminoacylated tRNAs with release factors, causing premature translational termination at introduced nonsense codons(4, 5) In addition, many ncAAs require advanced expertise in chemical synthesis, or are prohibitively expensive, and are used at high millimolar concentrations in typical expression experiments, a factor that is exacerbated by low protein yields.

Significant advances have been made in recent years towards addressing these challenges. Optimization of aaRS/tRNA expression systems combined with novel selection and evolution strategies to improve both orthogonal aaRSs and tRNAs have considerably increased suppression efficiencies(6-10). Efforts to bypass competition with release factors have led to orthogonal ribosomes that read quadruplet codons(11, 12), release factor knockout strains(13, 14) and recoded strains with compressed genetic codes(15, 16), allowing also for sense codon reassignment(17).

Another less investigated factor impacting ncAA incorporation efficiencies lies in their intracellular bioavailability. In typical GCE experiments, chemically synthesized ncAAs are added to expression media and enter cells via passive diffusion, resulting in intracellular concentrations at best equal but most likely severely below those added to media. This specifically reduces aminoacylation efficiencies of aaRS/ncAA combinations that are operating below saturating conditions(4). Furthermore, the reliance on passive diffusion limits the full potential of introducing new-to-nature functionalities, as cell-permeability needs to be considered when designing new ncAAs. For a handful of ncAAs, efforts in introducing and engineering enzymatic pathways to biosynthesize corresponding ncAAs directly within cells have resulted in increased incorporation efficiencies compared to exogenous ncAA addition(18-23). Despite being an attractive prospect for GCE, introducing novel metabolic pathways into new hosts may require significant development. In addition, many ncAA functionalities lack biosynthetic strategies, necessitating substantial advancements before such approaches can be widely applied for increasing ncAA bioavailability and thus genetic encoding.

Engineering membrane transport systems as an alternative strategy for boosting intracellular ncAA uptake, is relatively unexplored, but holds the potential of being widely applicable for increasing expression yields of modified proteins. Previous research in this direction has investigated mutants of a periplasmic leucine binding protein to improve uptake and incorporation of various known ncAAs (24). Another study has used a 'trojan-horse' approach, in which a sulfonic acid moiety attached to a cargo molecule serves as recognition motif for a promiscuous bacterial sulfonate importer. Attaching an impermeant ncAA to a sulfonate carrier, followed by cleavage of the carrier via an engineered enzyme, led to increased cytosolic concentrations of this ncAA, but incorporation into proteins was not shown due to lack of a specific aaRS (25). The use of peptide transporters has also been investigated (26, 27), most notably for transporting phosphotyrosine as a lysine-linked dipeptide (28), which was believed to be a substrate for the dipeptide transporter Dpp. Peptide transporters, especially ATP-binding cassette (ABC) transporters like the Dpp-transporter, are promising candidates for engineering ncAA uptake due to their substrate promiscuity and ability to maintain high concentration gradients across the membrane (29). In addition, attaching ncAAs to peptide carriers is easily achievable via solid-phase peptide synthesis (SPPS) requiring minimal chemical expertise.

Despite these recent progresses, there is still a need in the art for more universal strategies to supply cells with sufficient amounts of non-canonical amino acids.

### SUMMARY OF THE INVENTION

The present invention is characterized in the herein provided embodiments and claims. In particular, the present invention relates, *inter alia,* to the following embodiments:
1. A compound comprising an isopeptide-linked lysine or an analogue thereof, the compound being a compound of formula (I): 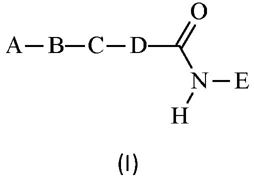 or a salt thereof, wherein:
   A is selected from -NH₂, -OH, -SH, -NH(C₁₋₅ alkyl), -N(C₁₋₅ alkyl)(C₁₋₅ alkyl), -N⁺(C₁₋₅ alkyl)(C₁₋₅ alkyl), -NH-CO-(C₁₋₅ alkyl), -COOH, -SO₃H, -SO₂H, -N₃, and -NO₂, or A is a peptidyl group;
   B is selected from: 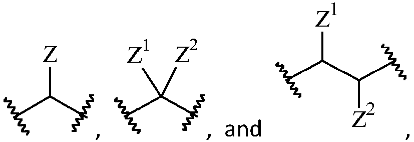 wherein the left empty valence is connected to A and the right empty valence is connected to C, wherein:
      Z is hydrogen or a side chain of an amino acid, in particular wherein Z is selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{Z}, -(C₁₋₆ alkylene)-S-R^{Z}, -(C₁₋₆ alkylene)-N(R^{Z})-R^{ZZ}, -(C₁₋₆ alkylene)-CO-R^{Z}, -(C₁₋₆ alkylene)-COO-R^{Z}, -(C₁₋₆ alkylene)-O-CO-(C₁₋₆ alkyl), -(C₁₋₆ alkylene)-CO-N(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CO-N(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z groups are each optionally substituted with one or more groups selected from -OH, -SH and Hal, further wherein one -CH₂- group in said alkyl may be replaced with 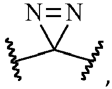 and further wherein each R^{Z} is independently selected from hydrogen and C₁₋₆ alkyl and wherein R^{ZZ} is selected from hydrogen, C₁₋₆ alkyl, -CHO, -CO(C₁₋₅ alkyl), -CO(C₁₋₅ alkenyl), -CO(C₁₋₅ alkynyl), -CO(C₁₋₅ alkylene)-COOH, -CO(C₁₋₅ alkenylene)-COOH, -CO(Co-s alkylene)-carbocyclyl, -CO(Co-s alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S},
      Z¹ and Z² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{z}, -(C₁₋₆ alkylene)-S-R^{Z}, -(C₁₋₆ alkylene)-N(R^{Z})-R^{ZZ}, -(C₁₋₆ alkylene)-COR^{Z}, -(C₁₋₆ alkylene)-COO-R^{z}, -(C₁₋₆ alkylene)-O-CO-(C₁₋₆ alkyl), -(C₁₋₆ alkylene)-CON(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CO-N(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z¹ and Z² groups are each optionally substituted with one or more -OH, -SH or Hal, wherein each R^{Z} is independently selected from hydrogen and C₁₋₆ alkyl, and R^{ZZ} is selected from hydrogen, C₁₋₆ alkyl, -CHO, -CO(C₁₋₅ alkyl), -CO(C₁₋₅ alkenyl), -CO(C₁₋₅ alkynyl), -CO(C₁₋₅ alkylene)-COOH, -CO(C₁₋₅ alkenylene)-COOH, -CO(Co-s alkylene)-carbocyclyl, -CO(C₀₋₅ alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S}, and further wherein one - CH₂- group in said alkyl may be replaced with 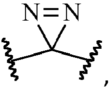 provided that at least one of Z¹ and Z² is not hydrogen, further provided that if Z¹ and Z² are connected to the same carbon atom, then both Z¹ and Z² are not hydrogen,
      or Z¹ and Z² are joined together to form, together with carbon atom(s) that otherwise carry Z¹ and Z², a non-aromatic carbocyclic ring or non-aromatic heterocyclic ring, wherein said non-aromatic carbocyclic ring and said non-aromatic heterocyclic ring are each optionally substituted with one or more R^{S}; -C-D- are defined as follows:
         C is selected from -CO-NH-, -CO-O-, -CO-S- and -CO-N(C₁₋₅ alkyl)-, wherein the left empty valence is connected to B and the right empty valence is connected to D, and
         D is selected from 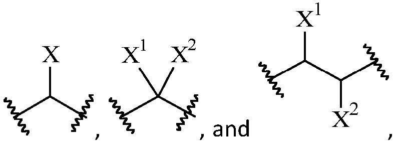 wherein the left empty valence is connected to C and the right empty valence is connected to -CO-NH-, wherein:
            X is a hydrogen or a side chain of an amino acid, in particular wherein X is selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{X}, -(C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(Cᵢ₋₆ alkyl), -(C₁₋₆ alkylene)-CO-N(R^{X})-R^{X}, -(C₁₋₆ alkylene)-N(R^{X})-CO-(C₁₋₆ alkyl), -(C₁₋₆ alkylene)-CO-N(R^{X})-O-R^{X}, -(C₁₋₆ alkylene)-O-CO-N(R^{X})-R^{X}, -(C₁₋₆ alkylene)-N(R^{X})-CO-N(R^{X})-R^{X}, -(C₁₋₆ alkylene)-N(R^{X})-C(=N-R^{X})-N(R^{X})-R^{X}, -(C₁₋₆ alkylene)-SO₃-R^{X}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X groups are each optionally substituted with one or more -OH, -SH or -Hal, wherein each R^{X} is independently selected from hydrogen and C₁₋₆ alkyl, and further wherein one -CH₂- group in said alkyl may be replaced with 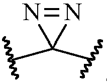
            X¹ and X² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{X}, -(C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(C₁₋₆ alkyl), - (C₁₋₆ alkylene)-CO-N(R^{X})-R^{X}, -(C₁₋₆ alkylene)-N(R^{X})-CO-(C₁₋₆ alkyl), -(C₁₋₆ alkylene)-CO-N(R^{X})-O-R^{X}, -(C₁₋₆ alkylene)-O-CO-N(R^{X})-R^{X}, -(C₁₋₆ alkylene)-N(R^{X})-CO-N(R^{X})- R^{X}, -(C₁₋₆ alkylene)-N(R^{X})-C(=N-R^{X})-N(R^{X})-R^{X}, -(C₁₋₆ alkylene)-SO₃-R^{X}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X¹ and X² groups are each optionally substituted with one or more -OH, -SH or Hal (preferably with one or more -OH), wherein each R^{X} is independently selected from hydrogen and C₁₋₆ alkyl, further wherein one -CH₂- group in said alkyl may be replaced with 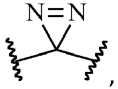 provided that at least one of X¹ and X² is not hydrogen, further provided that if X¹ and X² are connected to the same carbon atom, then both X¹ and X² are not hydrogen, or X¹ and X² are joined together to form, together with carbon atom(s) that otherwise carry X¹ and X², a non-aromatic carbocyclic ring or non-aromatic heterocyclic ring, wherein said non-aromatic carbocyclic ring and said non-aromatic heterocyclic ring are each optionally substituted with one or more R^{S};
      or -C-D- taken together is -CO-(N-heterocycloalkylene)-, wherein said N-heterocycloalkylene is connected to said -CO- group of -C-D- through its nitrogen atom and wherein said N-heterocycloalkylene is optionally substituted with one or more R^{S};
   E is -(C₁₋₆ akylene)-CHY¹Y², wherein Y¹ is selected from hydrogen, -SH, -OH and -NH₂, and wherein Y² is hydrogen or COOH, provided that at least one of Y¹ and Y² is not hydrogen, further wherein said alkylene is optionally substituted with an -OH group, -SH group or Hal, and wherein one -CH₂- group in said alkylene may be replaced with 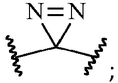 and
   each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-CO-NH(C₁₋₅ alkylene)-CN, -(C₀₋₃ alkylene)-CO-N(C₁₋₅ alkyl)(C₁₋₅ alkylene)-CN, -(C₀₋₃ alkylene)-NH-CO-(C₁₋₅ alkylene)-CN, -(C₀₋₃ alkylene)-N(C₁₋₅ alkyl)-CO-(C₁₋₅ alkylene)-CN, -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -OSO₂F, -B(OH)₂, -PO₄H, -PO₃H, - SO₄H, -SO₃H, -L-(C₀₋₃ alkylene)-carbocyclyl, and -L-(C₀₋₃ alkylene)-heterocyclyl, wherein L is selected from -(C₀₋₃ alkylene)-NH-, -(C₀₋₃ alkylene)-O-, -(C₀₋₃ alkylene)-CO-, -(C₀₋₃ alkylene)-NH-CO-, -(C₀₋₃ alkylene)-CO-NH-, -(C₀₋₃ alkylene)-NH-CO-O-, -(C₀₋₃ alkylene)-O-CO-NH-, -(C₀₋₃ alkylene)-NH-CO-NH- and -(C₀₋₃ alkylene)-NH-CO-NH-, and wherein the carbocyclyl moiety in said -L-(C₀₋₃ alkylene)-carbocyclyl and the heterocyclyl moiety in said -L-(C₀₋₃ alkylene)-heterocyclyl are each optionally substituted with one or more groups independently selected from C₁₋₄ alkyl, halogen, -CN, -NOz, -OH, -O-(C₁₋₄ alkyl), - SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl) and -N(C₁₋₄ alkyl)-CO(C₁₋₄ alkyl).
2. The compound of embodiment 1, wherein A is selected from -NH₂, -OH, -SH, -NH(C₁₋s alkyl), -N(C₁₋s alkyl)(C₁₋₅ alkyl), -NH-CO-(C₁₋s alkyl), -COOH, -SO₃H, -SOzH, -N₃, and -NOz.
3. The compound of embodiment 1 or 2, wherein A is selected from -NH₂, -OH, -SH, -NH(C₁₋₅ alkyl), -N(C₁₋s alkyl)(C₁₋₅ alkyl), and -N₃.
4. The compound of any one of embodiments 1 to 3, wherein A is -NH₂.
5. The compound of any one of embodiments 1 to 4, wherein B is 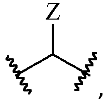 preferably wherein B is 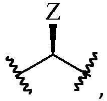 wherein the left empty valence is connected to A and the right empty valence is connected to C.
6. The compound of embodiment 5, wherein Z is selected from hydrogen, -(C₁₋₆ alkylene)-N(R^{Z})-R^{ZZ}, and -(C₁₋₆ alkylene)-N(R^{Z})-CO-(C₁₋₆ alkyl), preferably wherein Z is selected from hydrogen, -CH₂CH₂CH₂CH₂-NH₂ and -CH₂CH₂CH₂CH₂-NH-CO-CH₃.
7. The compound of embodiment 6, wherein Z is hydrogen.
8. The compound of any one of embodiments 1 to 7, wherein C is selected from -CO-NH- and -CO-O-, wherein the left empty valence is connected to B and the right empty valence is connected to D.
9. The compound of embodiment 8, wherein C is -CO-NH-, wherein the left empty valence is connected to B and the right empty valence is connected to D.
10. The compound of any one of embodiments 1 to 9, wherein D is 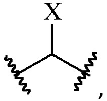 preferably wherein D is 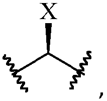 wherein the left empty valence is connected to C and the right empty valence is connected to -CO-NH- group.
11. The compound of embodiment 10, wherein X is selected from C₁₋₆ alkyl, C₂₋₈ alkenyl, C₂₋₈ alkynyl, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-N₃, -(C₁₋₆ alkylene)-Cl, -(C₁₋₆ alkylene)-NH₂, -(C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, wherein said alkyl, said alkenyl, said alkynyl, and any alkylene group in any of the aforementioned groups are each optionally substituted with one or more -OH, and further wherein one -CH₂- group in said alkyl may be replaced with 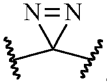
12. The compound of embodiment 11, wherein X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl.
13. The compound of any one of embodiments 1 to 7, wherein -C-D- is a moiety according to formula 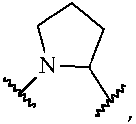 wherein the left empty valence is connected to B, and the right empty valence is connected to -CO-NH- group.
14. The compound of any one of embodiments 1 to 13, wherein E is -CH₂CH₂CH₂CH₂-CH(-NH₂)-COOH.
15. The compound of embodiment 1, wherein the compound is a compound according to formula (II): 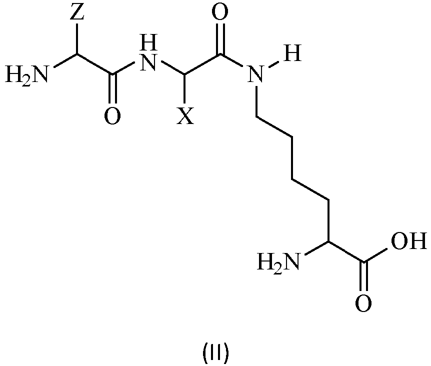
   or its salt, preferably wherein the compound of formula (II) is preferably a compound of formula (IIb): 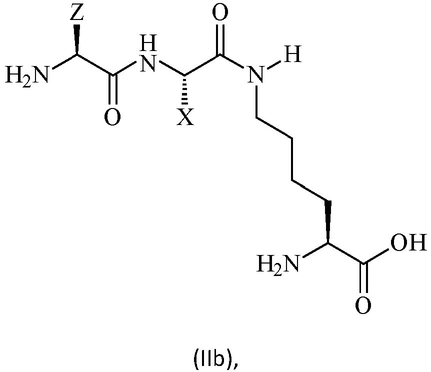
   or its salt, wherein Z and X are as defined in any one of embodiments 1 to 14, preferably wherein Z is selected from hydrogen, -(C₁₋₆ alkylene)-N(R^{Z})-R^{ZZ}, and -(C₁₋₆ alkylene)-N(R^{Z})-CO-(C₁₋₆ alkyl), more preferably wherein Z is selected from hydrogen, - CH₂CH₂CH₂CH₂-NH₂ and -CH₂CH₂CH₂CH₂-NH-CO-CH₃, even more preferably wherein Z is hydrogen,
   preferably wherein X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl;
   or wherein the compound is a compound of formula (III): 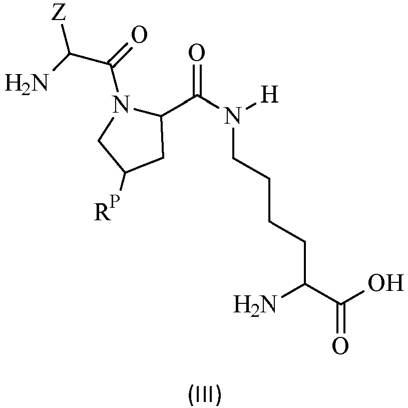
   or its salt, preferably wherein the compound of formula (III) is a compound of formula (Illa): 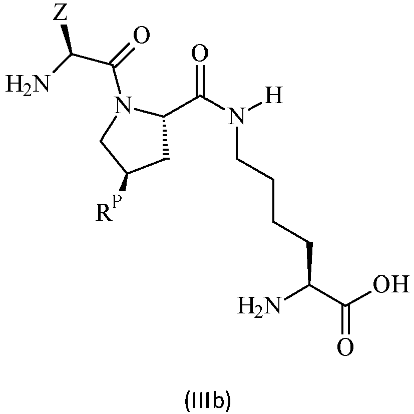
   or its salt, wherein R^{P} is hydrogen or -OH, and wherein Z is as defined in any one of embodiments 1 to 14, preferably wherein is selected from hydrogen, -(C₁₋₆ alkylene)-N(R^{Z})-R^{ZZ}, and -(C₁₋₆ alkylene)-N(R^{Z})-CO-(C₁₋₆ alkyl), more preferably wherein Z is selected from hydrogen, -CH₂CH₂CH₂CH₂-NH₂ and -CH₂CH₂CH₂CH₂-NH-CO-CH₃, even more preferably wherein Z is hydrogen.
16. The compound of embodiment 1, wherein the compound is a compound selected from Table 1, or its salt.
17. A method for identifying an engineered peptide-binding protein having increased affinity and/or specificity for the compound according to any one of embodiments 1 to 16, the method comprising the steps of:
   a) providing a nucleic acid encoding a peptide-binding protein comprising one or more mutations;
   b) expressing the nucleic acid of step (a) in a cell comprising an orthogonal translation system, wherein the orthogonal translation system comprises an orthogonal aminoacyl-tRNA synthetase/tRNA pair specific for an isopeptide-linked lysine or an analog thereof, and a reporter protein gene comprising one or more codons that have been re-allocated for the incorporation of the isopeptide-linked lysine or the analog thereof by the orthogonal aminoacyl-tRNA synthetase/tRNA pair;
   c) contacting the cell of step (b) with a compound according to any one of embodiments 1 to 16, wherein the compound is transported into the cell and hydrolyzed inside the cell to release the isopeptide-linked lysine or an analog thereof;
   d) detecting the expression of the reporter protein gene by the cell, wherein the expression is dependent on the successful uptake of the compound in step (c); and
   e) identifying the engineered peptide-binding protein as having increased affinity and/or specificity for the compound according to any one of embodiments 1 to 16 based on the expression of the reporter protein gene detected in step (d).
18. The method according to embodiment 17, wherein the peptide-binding protein is the peptide-binding protein of an oligopeptide permease (OppA).
19. The method according to embodiment 17 or 18, wherein the peptide-binding protein is the peptide-binding protein of the E. coli oligopeptide permease (OppA; SEQ ID NO:1).
20. The method according to embodiment 19, wherein in step (a) one or more mutations are introduced at positions L78, T173, V193, D221, W222, I303, K307, K333, N337, K371, R439, T429, S460, and/or V482 of SEQ ID NO:1.
21. The method according to any one of embodiments 17 to 20, wherein the mutations introduced in step (a) are introduced using error-prone PCR and/or saturated mutagenesis and/or continuous directed evolution.
22. The method according to any one of embodiments 17 to 21, wherein the cell in step (b) is a prokaryotic cell, in particular an E. coli cell.
23. The method according to any one of embodiments 17 to 22, wherein the orthogonal aminoacyl-tRNA synthetase/tRNA pair is a Pyrrolysyl-tRNA synthetase/tRNA pair.
24. The method according to any one of embodiments 17 to 23, wherein the reporter protein gene in step (b) comprises one or more amber stop codons that have been re-allocated to incorporate the isopeptide-linked lysine or the analog thereof.
25. The method according to any one of embodiments 17 to 24, wherein in step (c), the cell is contacted with a compound according to any one of embodiments 1 to 16 in the presence of a peptide that competes for binding to the peptide-binding protein.
26. The method according to any one of embodiments 17 to 25, wherein the reporter protein is a fluorescent protein.
27. The method according to embodiment 26, wherein the detection of the expression of the reporter protein gene in step (d) is performed using fluorescence-activated cell sorting (FACS).
28. The method according to any one of embodiments 17 to 25, wherein the detection of the expression of the reporter protein gene in step (d) is performed using antibiotic resistance screening and/or using growth-based selection.
29. The method according to any one of embodiments 17 to 28, wherein the isopeptide-linked lysine or the analog thereof is obtained by intracellular cleavage of the compound of any one of embodiments 1 to 16.
30. The method according to any one of embodiments 17 to 29, wherein identifying the engineered peptide-binding protein as having increased specificity for the compound in step (e) comprises a step of determining the sequence of the nucleic acid encoding the peptide-binding protein and/or determining specific mutations in the nucleic acid encoding the peptide-binding protein.
31. The method according to any one of embodiments 17 to 30, wherein the isopeptide-linked lysine or the analog thereof has the structure: 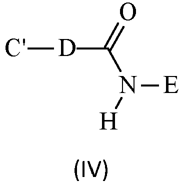 wherein C' is -NH₂ or -OH, and wherein D and E is defined as in any one of embodiments 1 to 16, wherein in D the left empty valence is connected to C'.
32. An engineered variant of an oligopeptide-binding protein OppA from E. coli (SEQ ID NO:1) comprising mutations in one or more of the following positions: L78, T173, V193, D221, W222, I303, K307, K333, N337, K371, R439, T429, S460, and/or V482.
33. The engineered variant of embodiment 32, wherein the one or more mutations are located in positions: T173, V193, D221, W222, I303, N337, R439, and/or S460.
34. The engineered variant of embodiment 32 or 33, wherein the one or more mutations are located in positions: D221, W222, N337, R439, and/or S460.
35. The engineered variant of any one of embodiments 32 to 34, wherein the one or more mutations are located in positions: D221, W222, R439, and/or S460.
36. The engineered variant of any one of embodiments 32 to 35, wherein the one or more mutations are located in positions: R439, and/or S460.
37. A nucleic acid encoding the engineered variant of any one of embodiments 32 to 36.
38. A vector comprising the nucleic acid according to embodiment 37.
39. A cell comprising the nucleic acid according to embodiment 37 or the vector according to embodiment 38.
40. The cell according to embodiment 39, wherein the cell is a prokaryotic cell, in particular an E. coli cell.
41. A method for incorporating a non-canonical amino acid, or an analog thereof, into a protein, the method comprising the steps of:
   a) providing a cell comprising at least one orthogonal translation system, wherein the at least one orthogonal translation system comprises an orthogonal aminoacyl-tRNA synthetase/tRNA pair specific for a non-canonical amino acid, or an analog thereof, comprised in the compound according to any one of embodiments 1 to 16, and a nucleic acid molecule encoding the protein, wherein the nucleic acid molecule encoding the protein comprises one or more codons that have been re-allocated for the incorporation of the non-canonical amino acid or an analog thereof by the orthogonal aminoacyl-tRNA synthetase/tRNA pair;
   b) contacting the cell of step (a) with the compound according to any one of embodiments 1 to 16, wherein the compound is transported into the cell and hydrolyzed inside the cell to release an isopeptide-linked lysine or an analog thereof and, optionally, another non-canonical amino acid or an analog thereof;
   c) incorporating (i) the isopeptide-linked lysine or an analog thereof and/or (ii) the other non-canonical amino acid, or the analog thereof, into the protein in response to the one or more re-allocated codon using the orthogonal translation system.
42. The method according to embodiment 41, wherein the cell is a prokaryotic cell, in particular an E. coli cell.
43. The method according to embodiment 42 or 43, wherein the cell expresses an oligopeptide permease.
44. The method according to embodiment 43, wherein the oligopeptide permease comprises a peptide-binding protein that has been engineered for increased specificity for the compound according to any one of embodiments 1 to 16.
45. The method according to embodiment 44, wherein the engineered peptide-binding protein is OppA of E. coli (SEQ ID NO:1) comprising mutations in one or more of the following positions: L78, T173, V193, D221, W222, I303, K307, K333, N337, K371, R439, T429, 5460, and/or V482.
46. The method according to any one of embodiments 41 to 45, wherein the orthogonal aminoacyl-tRNA synthetase/tRNA pair is a Pyrrolysyl-tRNA synthetase/tRNA pair.
47. The method according to any one of embodiments 41 to 46, wherein the nucleic acid molecule encoding the protein comprises one or more amber stop codons that have been re-allocated to incorporate the non-canonical amino acid or an analog thereof.
48. The method according to any one of embodiments 41 to 47, wherein hydrolysis of the molecule according to any one of embodiments 1 to 16 inside the cell releases a second non-canonical amino acid (ncAA), and wherein the second ncAA is incorporated into the same or a different protein in response to a re-allocated codon.
49. The method according to any one of embodiments 41 to 48, wherein the isopeptide-linked lysine or the analog thereof has the structure: 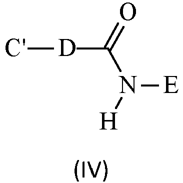 wherein C' is -NH₂ or -OH, and wherein D and E is defined as in any one of embodiments 1 to 16, wherein the left empty valence in D is connected to C'.

Herein the inventors leverage a propeptide-based strategy combined with the engineering of a bacterial ABC transporter system to boost intracellular ncAA concentrations and expression yields of modified proteins. The inventors found that easily synthesized isopeptide-linked tripeptides (G-XisoK) are actively transported into E. coli cells via the oligopeptide permease transporter Opp and are processed in the cytosol to reveal dipeptidic XisoK ncAAs. These isopeptide-linked lysine derivatives allow for the incorporation of various functionalities available for genetic code expansion, with efficiencies rivaling wild type (wt) protein expressions, providing a toolbox of novel ncAAs covering approaches from bioorthogonal labeling over chemical and photocrosslinking to native chemical ligation and chemoenzymatic conjugation. To exploit efficient G-XisoK-uptake for cheap and scalable production of modified proteins, the inventors evolved the periplasmic binding protein of the Opp transporter via a fluorescence-activated cell sorting platform, facilitating the preferential transport of G-XisoK tripeptides over linear tripeptides present in expression media. By introducing the evolved Opp binding protein variant into the E. coli genome, the inventors created a novel E. coli strain that allows high XisoK incorporation efficiencies across a range of target proteins for both single as well as multiple XisoK encoding. Furthermore, the inventors adapted the tripeptide scaffold for incorporation of two different ncAAs in response to two different nonsense codons via their concomitant transport using a single tripeptide, demonstrating the significant impact that active ncAA uptake has on the efficient production of proteins with an expanded alphabet.

In one aspect, the invention relates to a compound comprising an isopeptide-linked lysine or an analogue thereof. The skilled person understands that isopeptide-linked lysine, which also may be referred to as isopeptide-bond linked lysine, is a lysine forming an amide bond with its side chain amino group. To this end, it is to be understood that preferably said amide bond is a peptide bond, i.e., amide bond formed with a peptide. Furthermore, the lysine is otherwise preferably not modified, and its both main chain functional groups, i.e., carboxylic acid group and amino group, are present. Accordingly, a lysine residue forming an isopeptide bond, as referred to herein, is a group according to formula:

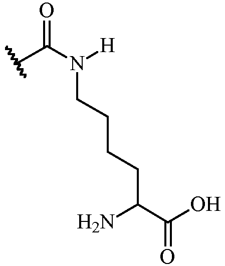

It is preferred that the lysine involved in formation of the isopeptide bond, as referred to herein, is L-lysine.

Thus, preferably, the compound comprising an isopeptide-linked lysine or an analogue thereof is defined as a peptide comprising at least two amino acid residues, and wherein the C-terminal carboxyl group of said C-terminal amino acid residue is modified as -CO-NH-E.

In said -CO-NH-E group, E is -(C₁₋₆ alkylene)-CHY¹Y², wherein Y¹ is selected from hydrogen, - SH, -OH and -NH₂, preferably wherein Y¹ is hydrogen or -NH₂, and wherein Y² is hydrogen or COOH, provided that at least one of Y¹ and Y² is not hydrogen. Accordingly, in one embodiment, Y¹ is -NH₂ and Y² is COOH. In one embodiment, Y¹ is hydrogen and Y² is COOH. In one embodiment, Y¹ is -NH₂ and Y² is hydrogen.

Said alkylene in -(C₁₋₆ alkylene)-CHY¹Y² is further optionally substituted with a group selected from -OH, -SH and Hal. Said alkylene is optionally substituted with an -OH group. Preferably however, said alkylene is not substituted with an -OH group.

Furthermore, one -CH₂- group in said alkylene may be replaced with 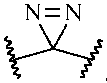 Preferably however, no -CH₂- group in said alkylene is replaced with

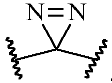

Preferably, said -(C₁₋₆ alkylene)- in E is preferably -(C₃₋₄ alkylene)-. More preferably, said - (C₁₋₆ alkylene)- in E is preferably -(C₄ alkylene)-, such as -CH₂CH₂CH₂CH₂-.

Accordingly, particularly preferred E are selected from -CH₂CH₂CH₂CH₂-CH(-NH₂)COOH, - CH₂CH₂CH₂-CH(-NH₂)COOH, -CH₂CH₂CH₂CH₂CH₂COOH, and -CH₂CH₂CH₂CH₂COOH. More preferably, E is -CH₂CH₂CH₂CH₂-CH(-NH₂)-COOH.

Preferably, in E, the configuration on carbon atom that carries -COOH group and -NH₂ group is the same as in natural L-lysine.

Preferably, in the compound comprising an isopeptide-linked lysine or an analogue thereof, the C-terminal amino acid residue of said peptide is not glycine. Furthermore, it is preferred that the peptide in the compound of the present invention is a dipeptide. It is further preferred that the dipeptide, as referred to herein, is not glycylglycine.

According to the present invention, the compound comprising an isopeptide-linked lysine or an analogue thereof, is a compound of formula (I): 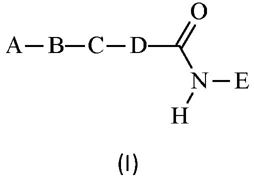 or a salt thereof.

It is to be understood that when referring to the compound comprising an isopeptide-linked lysine or an analogue thereof, which is a compound of formula (I), a reference to a compound of formula (I) is preferably made.

In formula (I), A is selected from -NH₂, -OH, -SH, -NH(C₁₋s alkyl), -N(C₁₋s alkyl)(C₁₋₅ alkyl), - N⁺(C₁₋₅ alkyl)(C₁₋₅ alkyl)(C₁₋₅ alkyl), -NH-CO-(C₁₋₅ alkyl), -COOH, -SO₃H, -SOzH, -N₃, and -NOz, or A is a peptidyl group. It is to be understood that if A is a charged group, a suitable counterion (or suitable counter-charge in the compound of formula (I)) is to be present.

Preferably, A is selected from -NH₂, -OH, -SH, -NH(C₁₋s alkyl), -N(C₁₋s alkyl)(C₁₋₅ alkyl), -NH-CO-(C₁₋₅ alkyl), -COOH, -SO₃H, -SO₂H, -N₃, and -NO₂, or A is a peptidyl group.

More preferably, A is selected from -NH₂, -OH, -SH, -NH(C₁₋s alkyl), -N(C₁₋₅ alkyl)(C₁₋₅ alkyl), - NH-CO-(C₁₋₅ alkyl), -COOH, -SO₃H, -SOzH, -N₃, and -NOz.

Even more preferably, A is selected from -NH₂, -OH, -SH, -NH(C₁₋s alkyl), -N(C₁₋s alkyl)(C₁₋₅ alkyl), -NH-CO-(C₁₋₅ alkyl), -N₃, and -NOz.

Even more preferably, A is selected from -NH₂, -OH, -SH, -NH(C₁₋s alkyl), -N(C₁₋s alkyl)(C₁₋₅ alkyl), and N₃.

Again more preferably, A is selected from -NH₂, -OH, -SH, -NH(C₁₋s alkyl), and -N(C₁₋s alkyl)(C₁₋₅ alkyl).

Still more preferably, A is selected from -NH₂, and -OH.

Most preferably, A is -NH₂.

In formula (I), B is selected from: 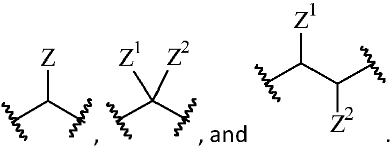 It is to be understood that the left empty valence as depicted herein for B is connected to A, and that the right empty valence as depicted herein for B, is connected to C.

Preferably, B is 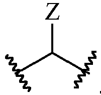 The stereogenic carbon atom carrying Z in B can have both possible stereoconfigurations, as encompassed by the invention. However, preferably B is 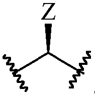 In other words, preferably, the configuration of the stereogenic carbon atom carrying Z in B is the same as the stereoconfiguration of an **α**-carbon atom in a natural L-amino acid residue. It is to be understood that, the left empty valence is connected to A and the right empty valence is connected to C.

In formula (I), Z is hydrogen or a side chain of an amino acid. The present invention does not limit Z to natural amino acid, and accordingly Z can be a side chain of a natural amino acid or a side chain of a nonnatural amino acid. Exemplary preferred side chains of natural and nonnatural amino acid are recited in the following.

Accordingly and preferably, Z is selected from hydrogen, C₁₋₈ alkyl, C₂₋₈ alkenyl, C₂₋₈ alkynyl, - (C₁₋₆ alkylene)-N₃, -(C₁₋₆ alkylene)-CN, -(C₁₋₆ alkylene)-Hal, -(C₁₋₆ alkylene)-O-R^{Z}, -(C₁₋₆ alkylene)-S-R^{Z}, -(C₁₋₆ alkylene)-N(R^{Z})-R^{ZZ}, -(C₁₋₆ alkylene)-CO-R^{Z}, -(C₁₋₆ alkylene)-COO-R^{z}, -(C₁₋₆ alkylene)-O-CO-(Cᵢ₋₆ alkyl), -(C₁₋₆ alkylene)-CO-N(R^{Z})-R^{Z}, -(C₁₋₆ alkylene)-N(R^{Z})-CO-(C₁₋₆ alkyl), - (C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CO-N(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z groups are each optionally substituted with one or more groups selected from -OH, -SH and Hal (preferably with one or more -OH), further wherein one -CH₂- group in said alkyl may be replaced with 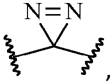 and further wherein each R^{Z} is independently selected from hydrogen and C₁₋₆ alkyl and wherein R^{ZZ} is selected from hydrogen, C₁₋₆ alkyl, -CHO, -CO(C₁₋₅ alkyl), -CO(C₁₋₅ alkenyl), -CO(C₁₋₅ alkynyl), -CO(C₁₋₅ alkylene)-COOH, -CO(C₁₋₅ alkenylene)-COOH, -CO(Co-s alkylene)-carbocyclyl, -CO(C₀₋₅ alkylene)-heterocyclyl, -CO-O-(C₁₋₅ alkyl), -CO-O-(C₁₋₅ alkenyl), -CO-O-(C₁₋₅ alkynyl), - CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S} (preferably selected from hydrogen, C₁₋₆ alkyl, aryl-CHz--O-CO- (such as benzyl-O-CO-) and CH₂=CH-CH₂-O-CO-, more preferably selected from hydrogen, and C₁₋₆ alkyl).

More preferably, Z is selected from hydrogen, C₁₋₈ alkyl, C₂₋₈ alkenyl, C₂₋₈ alkynyl, -(C₁₋₆ alkylene)-N₃, -(C₁₋₆ alkylene)-Hal, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, -(C₁₋₆ alkylene)-NH-CO-NH₂, - (C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned groups are each optionally substituted with one or more -OH, further wherein one -CH₂- group in said alkyl may be replaced with

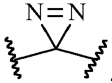

Even more preferably, Z is selected from hydrogen, C₁₋₆ alkyl, C₂₋₈ alkenyl, C₂₋₈ alkynyl, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned groups are each optionally substituted with one or more -OH.

It is particularly preferred that in the compound of formula (I), Z is selected from hydrogen, - (C₁₋₆ alkylene)-N(R^{Z})-R^{ZZ}, and -(C₁₋₆ alkylene)-N(R^{Z})-CO-(C₁₋₆ alkyl). More preferably, Z is selected from hydrogen, -CH₂CH₂CH₂CH₂-NH₂ and -CH₂CH₂CH₂CH₂-NH-CO-CH₃.

Further preferred Z are listed in any one of specific embodiments of the compound of formula (I).

In formula (I), Z¹ and Z² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{Z}, - (C₁₋₆ alkylene)-S-R^{Z}, -(C₁₋₆ alkylene)-N(R^{Z})-R^{ZZ}, -(C₁₋₆ alkylene)-CO-R^{Z}, -(C₁₋₆ alkylene)-COO-R^{Z}, -(C₁₋₆ alkylene)-O-CO-(Cᵢ₋₆ alkyl), -(C₁₋₆ alkylene)-CO-N(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CON(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z¹ and Z² groups are each optionally substituted with one or more -OH, -SH or Hal (preferably with one or more - OH), wherein each R^{Z} is independently selected from hydrogen and C₁₋₆ alkyl, and R^{ZZ} is selected from hydrogen, C₁₋₆ alkyl, -CHO, -CO(C₁₋₅ alkyl), -CO(C₁₋₅ alkenyl), -CO(C₁₋₅ alkynyl), - CO(C₁₋₅ alkylene)-COOH, -CO(C₁₋₅ alkenylene)-COOH, -CO(Co-s alkylene)-carbocyclyl, -CO(Co-s alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S} (preferably selected from hydrogen, C₁₋₆ alkyl, aryl-CH₂--O-CO- (such as benzyl-O-CO-) and CHz=CH-CHz-O-CO-, more preferably selected from hydrogen, and C₁₋₆ alkyl), and further wherein one -CH₂- group in said alkyl may be replaced with 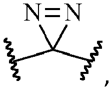 provided that at least one of Z¹ and Z² is not hydrogen, further provided that if Z¹ and Z² are connected to the same carbon atom, then both Z¹ and Z² are not hydrogen,
or Z¹ and Z² are joined together to form, together with carbon atom(s) that otherwise carry Z¹ and Z², a non-aromatic carbocyclic ring or non-aromatic heterocyclic ring, wherein said non-aromatic carbocyclic ring and said non-aromatic heterocyclic ring are each optionally substituted with one or more R^{S}.

Preferably, Z¹ and Z² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{Z}, - (C₁₋₆ alkylene)-S-R^{Z}, -(C₁₋₆ alkylene)-N(R^{Z})-R^{ZZ}, -(C₁₋₆ alkylene)-CO-R^{Z}, -(C₁₋₆ alkylene)-COO-R^{Z}, -(C₁₋₆ alkylene)-O-CO-(C₁₋₆ alkyl), -(C₁₋₆ alkylene)-CO-N(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CON(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z¹ and Z² groups are each optionally substituted with one or more -OH, -SH or Hal (preferably with one or more - OH), wherein each R^{Z} is independently selected from hydrogen and C₁₋₆ alkyl, and R^{ZZ} is selected from hydrogen, C₁₋₆ alkyl, -CHO, -CO(C₁₋₅ alkyl), -CO(C₁₋₅ alkenyl), -CO(C₁₋₅ alkynyl), - CO(C₁₋₅ alkylene)-COOH, -CO(C₁₋₅ alkenylene)-COOH, -CO(Co-s alkylene)-carbocyclyl, -CO(Co-s alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S} (preferably selected from hydrogen, C₁₋₆ alkyl, aryl-CH₂--O-CO- (such as benzyl-O-CO-) and CHz=CH-CHz-O-CO-, more preferably selected from hydrogen, and C₁₋₆ alkyl), and further wherein one -CH₂- group in said alkyl may be replaced with 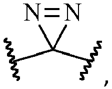 provided that at least one of Z¹ and Z² is not hydrogen, further provided that if Z¹ and Z² are connected to the same carbon atom, then both Z¹ and Z² are not hydrogen.

More preferably, Z¹ and Z² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-Hal, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, - (C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned Z¹ and Z² groups are each optionally substituted with one or more -OH, further wherein one -CH₂- group in said alkyl may be replaced with 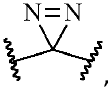 provided that at least one of Z¹ and Z² is not hydrogen, further provided that if Z¹ and Z² are connected to the same carbon atom, then both Z¹ and Z² are not hydrogen.

Even more preferably, Z¹ and Z² are each independently selected from hydrogen, C₁₋₈ alkyl, C₂₋₈ alkenyl, C₂₋₈ alkynyl, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said - (C₁₋₆ alkylene)-heterocycloalkyl are each optionally substituted with one or more groups R^{S}, and further wherein said alkyl, said alkenyl, said alkynyl, and any alkylene group in any of the aforementioned Z¹ and Z² groups are each optionally substituted with one or more -OH, provided that at least one of Z¹ and Z² is not hydrogen, further provided that if Z¹ and Z² are connected to the same carbon atom, then both Z¹ and Z² are not hydrogen.

Further preferred Z¹ and Z² groups are listed in any one of specific embodiments of the compound of formula (I).

In formula (I), -C-D- are defined as follows:
C is selected from -CO-NH-, -CO-O-, -CO-S- and -CO-N(C₁₋₅ alkyl)-, wherein the left empty valence is connected to B and the right empty valence is connected to D, and
D is selected from 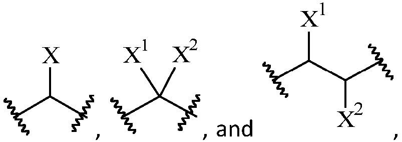 wherein the left empty valence is connected to C and the right empty valence is connected to -CO-NH- (i.e., -CO-NH- connected to -E),
or -C-D- taken together is -CO-(N-heterocycloalkylene)-, wherein said N-heterocycloalkylene is connected to said -CO- group of -C-D- through its nitrogen atom and wherein said N-heterocycloalkylene is optionally substituted with one or more R^{S}.

Preferably, C is selected from -CO-NH-, -CO-O-, -CO-S- and -CO-N(C₁₋₅ alkyl)-, wherein the left empty valence is connected to B and the right empty valence is connected to D, and D is selected from 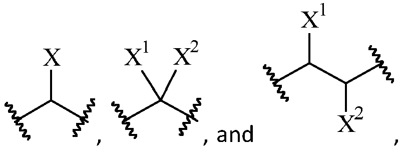 wherein the left empty valence is connected to C and the right empty valence is connected to -CO-NH-.

Accordingly, and preferably, C is selected from -CO-NH- and -CO-O-, wherein the left empty valence is connected to B and the right empty valence is connected to D.

More preferably, C is -CO-NH-, wherein the left empty valence is connected to B and the right empty valence is connected to D.

Alternatively, C is -CO-O-, wherein the left empty valence is connected to B and the right empty valence is connected to D. In particular, it is preferred that if A is not -NH₂, then C is - CO-O-, as described herein, i.e., wherein the left empty valence is connected to B and the right empty valence is connected to D.

In formula (I), preferably D is 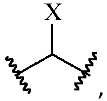 wherein the left empty valence is connected to C and the right empty valence is connected to -CO-NH- group. It is to be understood that both configurations at the stereogenic carbon atom carrying X are possible, as encompassed by the present invention. Preferably however, D is 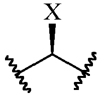 In other words, preferably, the configuration of the stereogenic carbon atom carrying X in D is the same as the stereoconfiguration of an **α**-carbon atom in a natural L-amino acid residue.

In formula (I), X is hydrogen or a side chain of an amino acid. The present invention does not limit X to natural amino acid, and accordingly C can be a side chain of a natural amino acid or a side chain of a nonnatural amino acid. Exemplary preferred side chains of natural and nonnatural amino acid are recited in the following.

Preferably, X is selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{X}, -(C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(C₁₋₆ alkyl), -(C₁₋₆ alkylene)-CO-N(R^{X})-R^{X}, -(C₁₋₆ alkylene)-N(R^{X})-CO-(C₁₋₆ alkyl), -(C₁₋₆ alkylene)-CON(R^{X})-O-R^{X}, -(C₁₋₆ alkylene)-O-CO-N(R^{X})-R^{X}, -(C₁₋₆ alkylene)-N(R^{X})-CO-N(R^{X})-R^{X}, -(C₁₋₆ alkylene)-N(R^{X})-C(=N-R^{X})-N(R^{X})-R^{X}, -(C₁₋₆ alkylene)-SO₃-R^{X}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X groups are each optionally substituted with one or more -OH, -SH or -Hal (preferably with one or more -OH), wherein each R^{X} is independently selected from hydrogen and C₁₋₆ alkyl, and further wherein one -CH₂-group in said alkyl may be replaced with

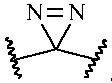

More preferably, X is selected from hydrogen, C₁₋₈ alkyl, C₂₋₈ alkenyl, C₂₋₈ alkynyl, -(C₁₋₆ alkylene)-N₃, -(C₁₋₆ alkylene)-Hal, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, -(C₁₋₆ alkylene)-NH-CO-NH₂, - (C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned groups are each optionally substituted with one or more -OH, further wherein one -CH₂- group in said alkyl may be replaced with

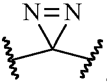

Even more preferably, X is selected from hydrogen, C₁₋₈ alkyl, C₂₋₈ alkenyl, C₂₋₈ alkynyl, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned groups are each optionally substituted with one or more -OH.

It is particularly preferred that in the compound of formula (I), X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl.

Further preferred X are listed in any one of specific embodiments of the compound of formula (I).

In formula (I), X¹ and X² are each independently selected from hydrogen, C₁₋₆ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{x}, - (C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(Cᵢ₋₆ alkyl), -(C₁₋₆ alkylene)-CO-N(R^{X})-R^{X}, -(C₁₋₆ alkylene)-N(R^{X})-CO-(C₁₋₆ alkyl), -(C₁₋₆ alkylene)-CO-N(R^{X})-O-R^{X}, -(C₁₋₆ alkylene)-O-CO-N(R^{X})-R^{X}, -(C₁₋₆ alkylene)-N(R^{X})-CON(R^{X})- R^{X}, -(C₁₋₆ alkylene)-N(R^{X})-C(=N-R^{X})-N(R^{X})-R^{X}, -(C₁₋₆ alkylene)-SO₃-R^{X}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X¹ and X² groups are each optionally substituted with one or more -OH, -SH or Hal (preferably with one or more - OH), wherein each R^{X} is independently selected from hydrogen and C₁₋₆ alkyl, further wherein one -CH₂- group in said alkyl may be replaced with 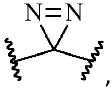 provided that at least one of X¹ and X² is not hydrogen, further provided that if X¹ and X² are connected to the same carbon atom, then both X¹ and X² are not hydrogen, or X¹ and X² are joined together to form, together with carbon atom(s) that otherwise carry X¹ and X², a non-aromatic carbocyclic ring or non-aromatic heterocyclic ring, wherein said non-aromatic carbocyclic ring and said non-aromatic heterocyclic ring are each optionally substituted with one or more R^{S}.

More preferably, X¹ and X² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-Hal, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, - (C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NHz, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, and further wherein said alkyl, said alkenyl, said alkynyl, and any alkylene group in any of the aforementioned X¹ and X² groups are each optionally substituted with one or more -OH, further wherein one -CH₂- group in said alkyl may be replaced with 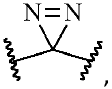 provided that at least one of X¹ and X² is not hydrogen, further provided that if X¹ and X² are connected to the same carbon atom, then both X¹ and X² are not hydrogen.

Even more preferably, X¹ and X² are each independently selected from hydrogen, C₁₋₈ alkyl, C₂₋₈ alkenyl, C₂₋₈ alkynyl, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said - (C₁₋₆ alkylene)-heterocycloalkyl are each optionally substituted with one or more groups R^{S}, and further wherein said alkyl, said alkenyl, said alkynyl, and any alkylene group in any of the aforementioned X¹ and X² groups are each optionally substituted with one or more -OH, provided that at least one of X¹ and X² is not hydrogen, further provided that if X¹ and X² are connected to the same carbon atom, then both X¹ and X² are not hydrogen.

Further preferred X¹ and X² groups are listed in any one of specific embodiments of the compound of formula (I).

In formula (I), E is as defined herein, including any preferred definition of E and any specific embodiment of E.

In formula (I), each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-CO-NH(C₁₋₅ alkylene)-CN, -(C₀₋₃ alkylene)-CO-N(C₁₋₅ alkyl)(C₁₋₅ alkylene)-CN, -(C₀₋₃ alkylene)-NH-CO-(C₁₋₅ alkylene)-CN, -(C₀₋₃ alkylene)-N(C₁₋₅ alkyl)-CO-(C₁₋₅ alkylene)-CN, -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SOz-NHz, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -OSO₂F, -B(OH)₂, -PO₄H, -PO₃H, -SO₄H, -SO₃H, -L-(C₀₋₃ alkylene)-carbocyclyl, and -L-(C₀₋₃ alkylene)-heterocyclyl, wherein L is selected from -(C₀₋₃ alkylene)-NH-, -(C₀₋₃ alkylene)-O-, -(C₀₋₃ alkylene)-CO-, -(C₀₋₃ alkylene)-NH-CO-, -(C₀₋₃ alkylene)-CO-NH-, -(C₀₋₃ alkylene)-NH-CO-O-, -(C₀₋₃ alkylene)-O-CO-NH-, -(C₀₋₃ alkylene)-NH-CO-NH- and -(C₀₋₃ alkylene)-NH-CO-NH-, and wherein the carbocyclyl moiety in said -L-(C₀₋₃ alkylene)-carbocyclyl and the heterocyclyl moiety in said -L-(C₀₋₃ alkylene)-heterocyclyl are each optionally substituted with one or more groups independently selected from C₁₋₄ alkyl, halogen, -CN, -NO₂, -OH, -O-(C₁₋₄ alkyl), -SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), - NHCO(C₁₋₄ alkyl) and -N(C₁₋₄ alkyl)-CO(C₁₋₄ alkyl).

Preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl, wherein the carbocyclyl moiety in said -(C₀₋₃ alkylene)-carbocyclyl and the heterocyclyl moiety in said -(C₀₋₃ alkylene)-heterocyclyl are each optionally substituted with one or more groups independently selected from C₁₋₄ alkyl, halogen, -CN, -NOz, -OH, -O-(C₁₋₄ alkyl), -SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl) and -N(C₁₋₄ alkyl)-CO(C₁₋₄ alkyl).

More preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl.

More preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), and -(C₀₋₃ alkylene)-SO-(C₁₋₅ alkyl).

Even more preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), and -(C₀₋₃ alkylene)-SO-(C₁₋₅ alkyl).

Still more preferably, each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), and -(C₀₋₃ alkylene)-O-CO-N(C₁₋₅ alkyl)-(C₁₋₅ alkyl).

Again more preferably, each R^{S} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-O(C₁₋₅ alkyl), halogen, -(C₁₋₅ haloalkyl), -O-(C₁₋₅ haloalkyl), -CN, -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-COO-(C₁₋₅ alkyl), -N(C₁₋₅ alkyl)-CO-O-(C₁₋₅ alkyl), -O-CO-NH-(C₁₋₅ alkyl), and -O-CO-N(C₁₋₅ alkyl)-(C₁₋₅ alkyl).

Still more preferably, each R^{S} is independently selected from C₁₋₅ alkyl, C₂₋₅ alkenyl, C₂₋₅ alkynyl, -OH, -O(C₁₋₅ alkyl), -O(C₁₋₅ alkylene)-OH, -SH, -S(C₁₋₅ alkyl), -S(C₁₋₅ alkylene)-SH, -NH₂, -NH(C₁₋₅ alkyl), -N(C₁₋₅ alkyl)(C₁₋₅ alkyl), halogen, -(C₁₋₅ haloalkyl), -O-(C₁₋₅ haloalkyl), -CN, -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), and -N(C₁₋₅ alkyl)-CO-(C₁₋₅ alkyl).

Even more preferably, each R^{S} is independently selected from C₁₋₅ alkyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, -(C₁₋₅ haloalkyl), -O-(C₁₋₅ haloalkyl), -CN, and -COOH.

Again more preferably, each R^{S} is independently selected from -OH, -SH, -NH₂, halogen, -CN, and -COOH.

Most preferably, each R^{S} is independently selected from -OH, halogen, and -CN.

Preferably, the compound according to formula (I), as provided herein, is a compound according to formula (II): 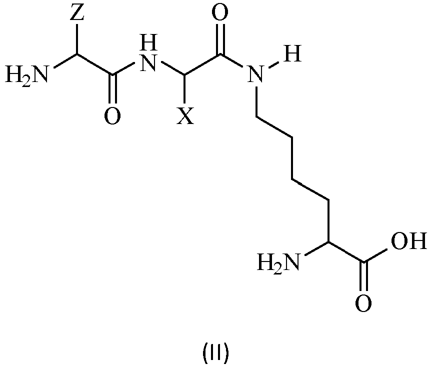 or its salt. In formula (II), Z and X are as defined in formula (I).

As mentioned before, it is preferred that lysine residue engaged in formation of an isopeptide bond is characterized by L configuration at its stereogenic centre. Accordingly, the compound of formula (II) is preferably a compound of formula (Ila): 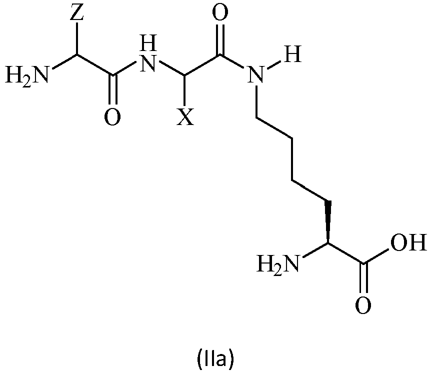 or its salt. In formula (Ila), Z and X are as defined in formula (I), including any preferred definition and any specific embodiment of formula (I).

In one preferred embodiment, the compound of formula (Ila) is preferably a compound of formula (IIb): 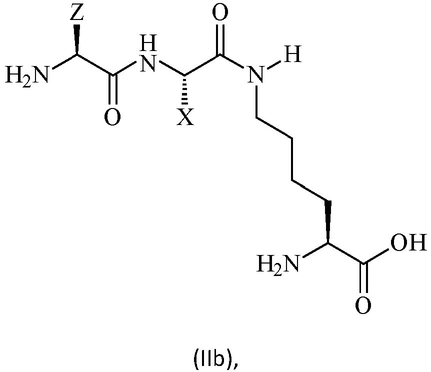 or its salt. In formula (Ilb), Z and X are as defined in formula (I), including any preferred definition and any specific embodiment of formula (I).

In one preferred embodiment, the compound of formula (Ila) is preferably a compound of formula (IIc): 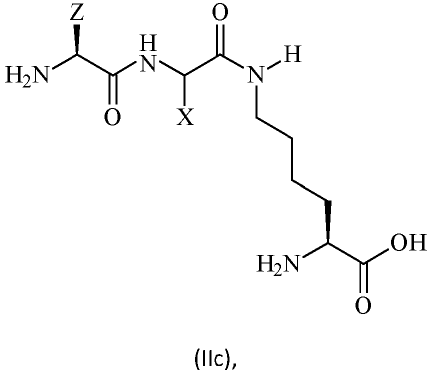 or its salt. In formula (IIc), Z and X are as defined in formula (I), including any preferred definition and any specific embodiment of formula (I).

Preferably, in formula (II) (or formula (IIa) (IIb) or (IIc)), Z is selected from hydrogen, -(C₁₋₆ alkylene)-N(R^{Z})-R^{ZZ}, and -(C₁₋₆ alkylene)-N(R^{Z})-CO-(C₁₋₆ alkyl). More preferably, Z is selected from hydrogen, -CH₂CH₂CH₂CH₂-NH₂ and -CH₂CH₂CH₂CH₂-NH-CO-CH₃. Even more preferably, Z is hydrogen.

Preferably, in formula (II) (or formula (IIa), (IIb) or (IIc)), X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl.

In one embodiment of the present invention, the compound of formula (I) is a compound of formula (III): 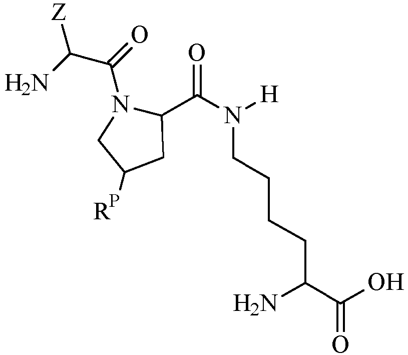 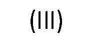 or its salt. In formula (III), R^{P} is hydrogen or -OH, preferably R^{P} is hydrogen. Further in formula (III), Z is as defined for formula (I), including any preferred definition and any specific embodiment of formula (I).

Preferably, the compound of formula (III) is a compound of formula (Illa): 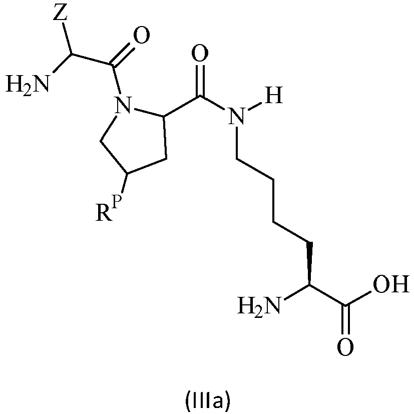 or its salt. or its salt. In formula (IIIa), R^{P} is hydrogen or -OH, preferably R^{P} is hydrogen. Further in formula (Illa), Z is as defined for formula (I), including any preferred definition and any specific embodiment of formula (I).

Preferably, the compound of formula (Illa) is a compound of formula (Illb): 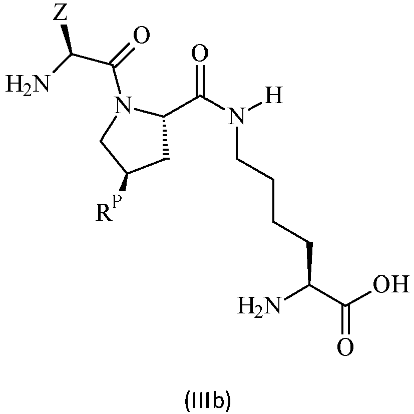 or its salt. In formula (IIIb), R^{P} is hydrogen or -OH, preferably R^{P} is hydrogen. Further in formula (IIIb), Z is as defined for formula (I), including any preferred definition and any specific embodiment of formula (I).

Preferably, in formula (III) (including formula (IIIa) and (IIIb), Z is selected from hydrogen, -(C₁₋₆ alkylene)-N(R^{Z})-R^{ZZ}, and -(C₁₋₆ alkylene)-N(R^{Z})-CO-(C₁₋₆ alkyl). More preferably, Z is selected from hydrogen, -CH₂CH₂CH₂CH₂-NH₂ and -CH₂CH₂CH₂CH₂-NH-CO-CH₃, even more preferably Z is hydrogen.

Particularly preferred compounds of formula (I) are listed in Table 1. Accordingly, it is preferred that the compound of formula (I) is a compound selected from the compounds listed in Table 1, or its salt.

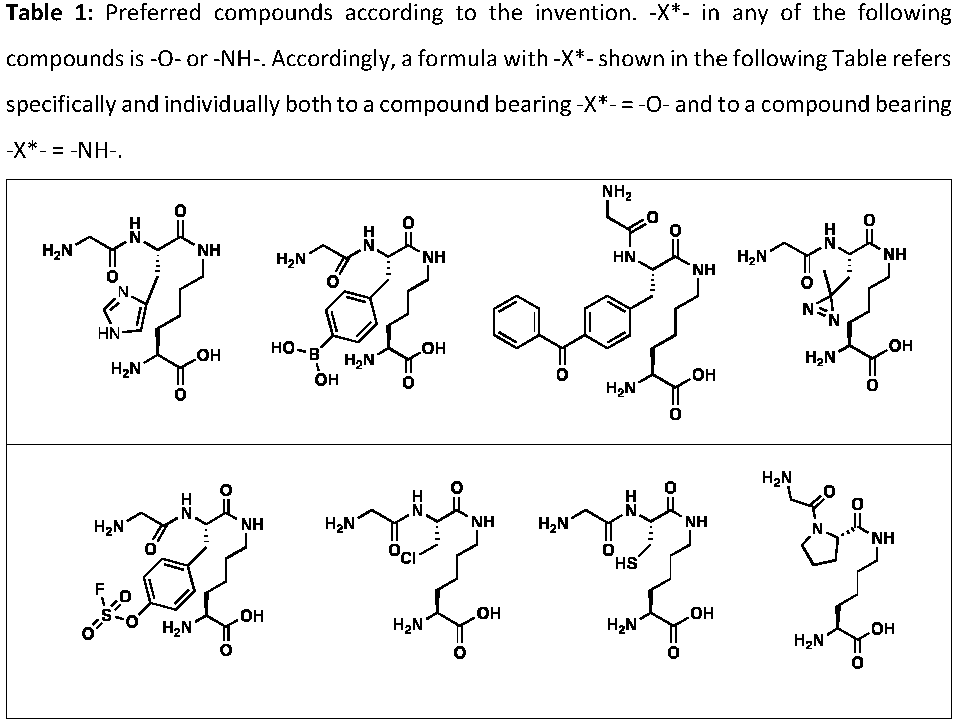

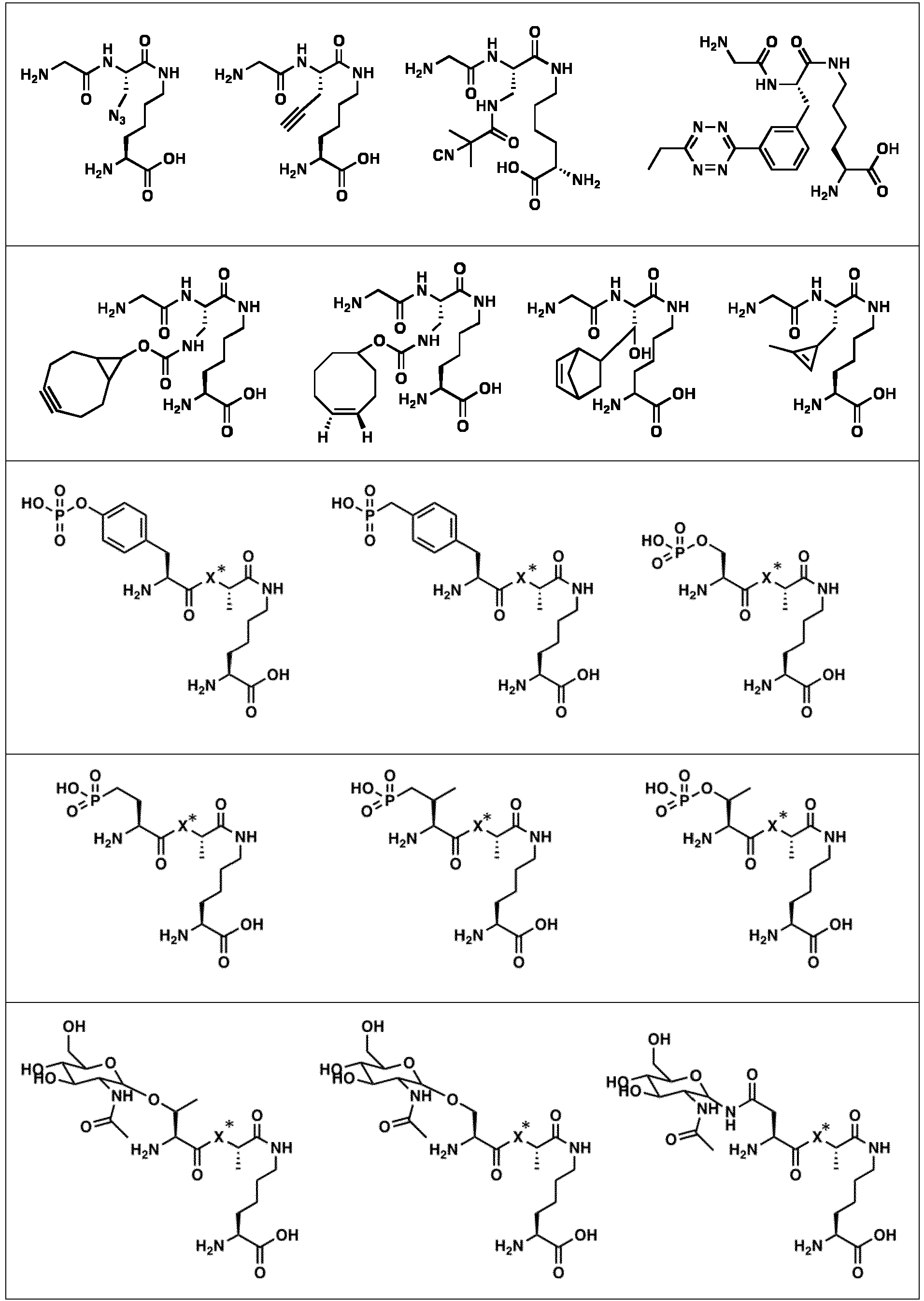

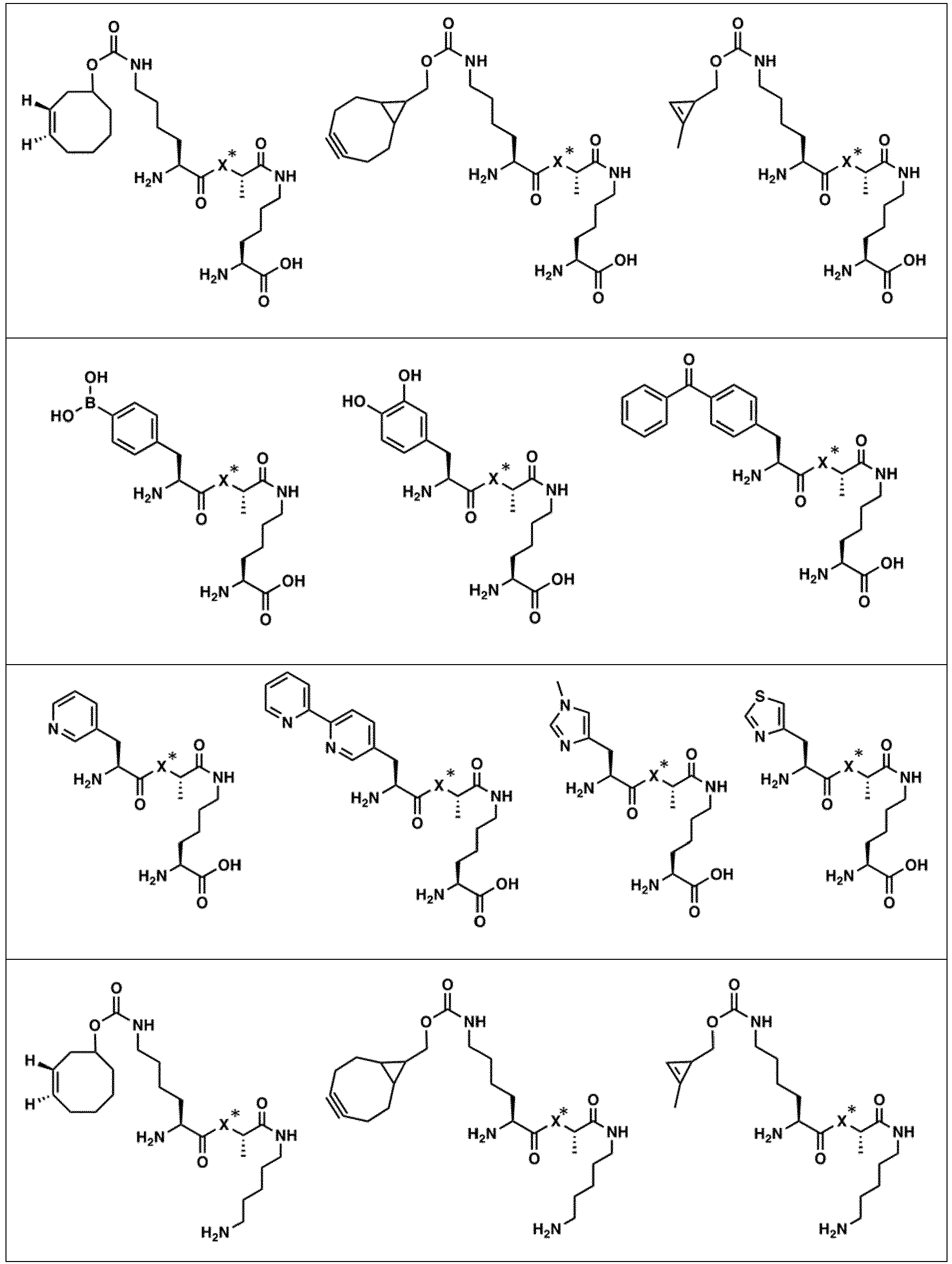

In the following specific embodiments, preferred definitions of the amino acid side chain are defined. Accordingly, the expression "any one of Z, Z¹, Z², X, X¹ and X²" is to be understood as encompassing individualized reference to each and every of variables any one of Z, Z¹, Z², X, X¹ and X², as referred to herein, as well as any combinations thereof. These variables are descriptive of amino acid sidechains in the compound of formula (I). Thus, as provided herein, any one of Z, Z¹, Z², X, X¹ and X² may refer to Z. Alternatively, any one of Z, Z¹, Z², X, X¹ and X² may refer to Z¹, Z² or Z¹ and Z². Alternatively, any one of Z, Z¹, Z², X, X¹ and X² may refer to X. Alternatively, any one of Z, Z¹, Z², X, X¹ and X² may refer to X¹, X² or X¹ and X². In other words, the expression "any one of Z, Z¹, Z², X, X¹ and X²" may also be understood as referring to an amino acid side chain, when discussing any amino acid side chain in the compound of the present invention. Accordingly and preferably, the expression "any one of Z, Z¹, Z², X, X¹ and X²", includes a specific and individual reference to Z, and a specific and individual reference to X.

In a first specific embodiment of the present invention, any one of Z, Z¹, Z², X, X¹ and X² is C₁₋₆ alkyl. Particularly preferred C₁₋₆ alkyl groups are methyl, ethyl, n-propyl, isopropyl, n-butyl, isobutyl, sec-butyl, and tert-butyl. Thus, in this embodiment any one of Z, Z¹, Z², X, X¹ and X² can be selected from methyl, ethyl, n-propyl, isopropyl, n-butyl, isobutyl, sec-butyl, and tert-butyl. Preferably, any one of Z, Z¹, Z², X, X¹ and X² is selected from methyl, isopropyl, isobutyl, and sec-butyl. Even more preferably, any one of Z, Z¹, Z², X, X¹ and X² is isopropyl.

In a second specific embodiment of the present invention, any one of Z, Z¹, Z², X, X¹ and X² is selected from phenyl and -(C₁₋₆ alkylene)-phenyl, preferably any one of Z, Z¹, Z², X, X¹ and X² is selected from phenyl, benzyl, and phenethyl. More preferably, any one of Z, Z¹, Z², X, X¹ and X² is benzyl.

In a third specific embodiment of the present invention, any one of Z, Z¹, Z², X, X¹ and X² is a side chain of an amino acid, preferably of an α-amino acid (particularly a naturally occurring α-amino acid, including a proteinogenic α-amino acid or a non-proteinogenic α-amino acid). Examples of any one of Z, Z¹, Z², X, X¹ and X² being a side chain of an amino acid are illustrated in the following table.

**Table 2: Examples of a side chain of an amino acid which can be any one of Z, Z¹, Z², X, X¹ and X².**

| **Z, Z¹, Z², X, X¹ or X²** | **Amino acid (side chain)** |
|---|---|
| hydrogen | Glycine |
| methyl | Alanine |
| ethyl | homoalanine |
| n-propyl | norvaline |
| isopropyl | valine |
| n-butyl | norleucine |
| isobutyl | leucine |
| sec-butyl | isoleucine |
| tert-butyl | terleucine |
| n-pentyl | homonorleucine |
| n-hexyl | 2-aminocaprylic acid |
| -C(-CH₃)(-OH)-CH₃ | β-hydroxyvaline |
| -CH(-OH)-CH(-CH₃)-CH₃ | β-hydroxyleucine |
| -CH₂-CH=CH-CH₃ | 2-amino-4-hexenoic acid |
| -CH₂-C≡CH | propargylglycine |
| -CH(-OH)-C≡CH | β-ethynylserine |
| phenyl | phenylglycine |
| 4-hydroxyphenyl | 4-hydroxyphenylglycine |
| 3,5-dihydroxyphenyl | 3,5-dihydroxyphenylglycine |
| -CH₂-phenyl | phenylalanine |
| -CH₂-(4-hydroxyphenyl) | tyrosine |
| -CH₂-(3,4-dihydroxyphenyl) | DOPA |
| -CH₂-(3-nitro-4-hydroxyphenyl) | 3-nitrotyrosine |
| -CH₂-(4-methoxyphenyl) | O-methyltyrosine |
| -CH₂-(3-hydroxy-4-methoxy-5-methylphenyl) | 3-OH-5-Me-OMe-tyrosine |
| -CH₂-(4-prenyloxyphenyl) | O-prenyltyrosine |
| -CH₂CH₂-phenyl | homophenylalanine |
| -CH₂CH₂-(4-hydroxyphenyl) | Homotyrosine |
| -CH(-OH)-CH₂-(4-nitrophenyl) | β-OH-p-NO₂-homophenylalanine |
| -CH₂-(1H-indol-3-yl) | tryptophan |
| -CH(-CH₃)-(1H-indol-3-yl) | β-methyl-tryptophan |
| -CH₂-(5-hydroxy-1H-indol-3-yl) | 5-hydroxytryptophan |
| -CH₂-(1-acetyl-6-hydroxy-1H-indol-3-yl) | 6-hydroxy-N-acetyltryptophan |
| -CH₂-(1-(1,1-dimethylallyl)-1H-indol-3-yl) | N-dimethylallyltryptophan |
| -CH(-CH₃)-(2-oxo-indolin-3-yl) | oxo-β-methyl-tryptophan |
| -CH₂-(4-nitro-1H-indol-3-yl) or -CH₂-(5-nitro-1H-indol-3-yl) | nitrated tryptophan |
| -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), - CH₂-(7-chloro-1H-indol-3-yl), or -CH₂-(7-bromo-1H-indol-3-yl) | halogenated tryptophan |
| -CH₂-(1H-imidazol-4-yl) | histidine |
| -CH₂-(5-mercapto-1-methyl-1H-imidazol-4-yl) | ovothiol |
| -CH₂-(3-furanyl) | 3-(3-furyl)-alanine |
| pyrrolidin-2-yl | tambroline |
| -CH₂-OH | serine |
| -CH₂CH₂-OH | homoserine |
| -CH₂CH₂-O-CH₃ | O-methyl-homoserine |
| -CH₂CH₂-O-CH₂CH₃ | O-ethyl-homoserine |
| -CH(-CH₃)-OH | threonine |
| -CH₂-SH | cysteine |
| -CH₂CH₂-SH | homocysteine |
| -CH₂CH₂-S-CH₃ | methionine |
| -CH₂CH₂-S-CH₂CH₃ | ethionine |
| -CH₂-SO₃H | cysteate |
| -CH₂-COOH | aspartate |
| -CH₂CH₂-COOH | glutamate |
| -CH₂-CO-NH₂ | asparagine |
| -CH₂CH₂-CO-NH₂ | glutamine |
| -(CH₂)₃-NH-CO-NH₂ | citrulline |
| -(CH₂)₂-CO-NH-OH | glutamate-γ-monohydroxamate |
| -CH₂-NH₂ | 2,3-diamino-propionic acid (Dap) |
| -(CH₂)₂-NH₂ | homo-Dap |
| -(CH₂)₃-NH₂ | ornithine |
| -(CH₂)₄-NH₂ | lysine |
| -CH(-CH₃)-(CH₂)₂-NH₂ | β-methylornithine |
| -C(-OH)-(CH₂)₃-NH₂ | 3-hydroxylysine |
| -CH₂-C(-OH)-(CH₂)₂-NH₂ | 4-hydroxylysine |
| -(CH₂)₂-C(-OH)-C(-CH₂-OH)-NH₂ | diamino-dihydroxy-heptanoic acid (DADH) |
| -(CH₂)₃-NH-C(=NH)-NH₂ | arginine |
| -CH(-CH₃)-(CH₂)₂-NH-C(=NH)-NH₂ | β-methylarginine |
| -C(-OH)-(CH₂)₂-NH-C(=NH)-NH₂ | 3-hydroxyarginine |
| -CH₂-C(-OH)-CH₂-NH-C(=NH)-NH₂ | 4-hydroxyarginine |
| -C(-OH)-C(-OH)-CH₂-NH-C(=NH)-NH₂ | 3,4-dihydroxyarginine |
| -(CH₂)₄-NH-C(=NH)-NH₂ | homoarginine |
| -C(-OH)-(CH₂)₃-NH-C(=NH)-NH₂ | 3-hydroxyhomoarginine |
| -CH₂-C(-OH)-CH₂CH₂-NH-C(=NH)-NH₂ | 4-hydroxyhomoarginine |
| -CH(-OH)-CH(-OH)-CH₂-O-CO-NH₂ | carbamoylpolyoxamic acid |

In a fourth specific embodiment, any one of Z, Z¹, Z², X, X¹ and X² is selected from hydrogen, methyl, ethyl, n-propyl, isopropyl, n-butyl, isobutyl, sec-butyl, tert-butyl, n-pentyl, n-hexyl, - CH₂-CH=CH-CH₃, -CH₂-C≡CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH_{z}-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, -CH₂-(2-nitrocycloprop-1-yl), -CH₂-OH, -CH₂CH₂-OH, -CH₂-O-CH₃, -CH₂CH₂-O-CH₃, -CH₂CH₂-O-CH₂CH₃, -CH(-CH₃)-OH, -C(-CH₃)(-OH)-CH₃, -CH(-OH)-CH(-CH₃)-CH₃, -CH(-OH)-CH(-CH₃)-CH₂-CH=CH-CH₃, -CH(-OH)-C≡CH, -CH₂-SH, -CH₂CH₂-SH, -CH₂CH₂-S-CH₃, -CH₂CH₂-S-CH₂CH₃, -CH₂-SO₃H, -CH₂-COOH, -CH₂CH₂-COOH, -CH(-OH)-COOH, -CH(-OH)-CH₂-COOH, -CH(-CH₃)-COOH, -CH(-CH₃)-CH₂-COOH, -CH₂-CO-NH₂, -CH₂CH₂-CO-NH₂, -CH(-OH)-CO-NH₂, -CH₂-NH-CO-NH₂, -(CH₂)₂-NH-CO-NH₂, -(CH₂)₃-NH-CO-NH₂, -(CH₂)₂-CO-NH-OH, -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, -(CH₂)₂-C(-OH)-C(-CH₂-OH)-NH₂, -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, -CH₂-C(-OH)-CH₂CH₂-NH-C(=NH)-NH₂, and -CH(-OH)-CH(-OH)-CH₂-O-CO-NH₂.

In a fifth specific embodiment, any one of Z, Z¹, Z², X, X¹ and X² is selected from hydrogen, methyl, ethyl, n-propyl, isopropyl, n-butyl, isobutyl, sec-butyl, tert-butyl, n-pentyl, n-hexyl, - CH₂-CH=CH-CH₃, -CH₂-C≡CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, and -CH₂-(2-nitrocycloprop-1-yl).

In a sixth specific embodiment of the present invention, any one of Z, Z¹, Z², X, X¹ and X² is hydrogen, which corresponds to a side chain of glycine.

In a seventh specific embodiment, any one of Z, Z¹, Z², X, X¹ and X² is selected from C₁₋s alkyl. Preferably, any one of Z, Z¹, Z², X, X¹ and X² is selected from methyl, ethyl, n-propyl, isopropyl, n-butyl, isobutyl, sec-butyl, tert-butyl, n-pentyl, and n-hexyl. In certain embodiments of the present invention, any one of Z, Z¹, Z², X, X¹ and X² is methyl, which corresponds to a side chain of alanine. In a particular embodiment of the present invention, any one of Z, Z¹, Z², X, X¹ and X² is ethyl, which corresponds to a side chain of homoalanine. In a further particular embodiment of the present invention, any one of Z, Z¹, Z², X, X¹ and X² is n-propyl, which corresponds to a side chain of norvaline. In a further particular embodiment of the present invention, any one of Z, Z¹, Z², X, X¹ and X² is isopropyl, which corresponds to a side chain of valine. In a further particular embodiment of the present invention, any one of Z, Z¹, Z², X, X¹ and X² is n-butyl, which corresponds to a side chain of norleucine. In a further particular embodiment of the present invention, any one of Z, Z¹, Z², X, X¹ and X² is isobutyl, which corresponds to a side chain of leucine. In a further particular embodiment of the present invention, any one of Z, Z¹, Z², X, X¹ and X² is sec-butyl, which corresponds to a side chain of isoleucine. In a further particular embodiment of the present invention, any one of Z, Z¹, Z², X, X¹ and X² is tert-butyl, which corresponds to a side chain of terleucine. In a further particular embodiment of the present invention, any one of Z, Z¹, Z², X, X¹ and X² is n-pentyl, which corresponds to a side chain of homonorleucine. In a further particular embodiment of the present invention, any one of Z, Z¹, Z², X, X¹ and X² is n-hexyl, which corresponds to a side chain of 2-aminocaprylic acid.

In an eighth specific embodiment of the present invention, any one of Z, Z¹, Z², X, X¹ and X² is selected from C₂₋₈ alkenyl and C₂₋₈ alkynyl. Preferably, any one of Z, Z¹, Z², X, X¹ and X² is selected from -CH₂-CH=CH-CH₃ and -CH₂-C≡CH. In a particular embodiment of the present invention, any one of Z, Z¹, Z², X, X¹ and X² is -CH₂-CH=CH-CH₃, which corresponds to a side chain of 2-amino-4-hexenoic acid. In a further particular embodiment of the present invention, any one of Z, Z¹, Z², X, X¹ and X² is -CH₂-C≡CH, which corresponds to a side chain of propargylglycine.

In a ninth specific embodiment of the present invention, any one of Z, Z¹, Z², X, X¹ and X² is phenyl or -(C₁₋₆ alkylene)-phenyl, wherein said phenyl and the phenyl group in said -(C₁₋₆ alkylene)-phenyl are each optionally substituted with one or more groups R^{S}. Preferably, any one of Z, Z¹, Z², X, X¹ and X² is selected from phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), and -CH(-OH)-CH₂-(4-nitrophenyl).

In a particular embodiment of the present invention, any one of Z, Z¹, Z², X, X¹ and X² is phenyl, which corresponds to a side chain of phenylglycine. In a further particular embodiment of the present invention, any one of Z, Z¹, Z², X, X¹ and X² is 4-hydroxyphenyl, which corresponds to a side chain of 4-hydroxyphenylglycine. In a further particular embodiment of the present invention, anyone of Z, Z¹, Z², X, X¹ and X² is 3,5-dihydroxyphenyl, which corresponds to a side chain of 3,5-dihydroxyphenylglycine. In a further particular embodiment of the present invention, any one of Z, Z¹, Z², X, X¹ and X² is -CH₂-phenyl, which corresponds to a side chain of phenylalanine. In a further particular embodiment of the present invention, any one of Z, Z¹, Z², X, X¹ and X² is -CH₂-(4-hydroxyphenyl), which corresponds to a side chain of tyrosine. In a further particular embodiment of the present invention, any one of Z, Z¹, Z², X, X¹ and X² is - CH₂-(3,4-dihydroxyphenyl), which corresponds to a side chain of DOPA. In a further particular embodiment of the present invention, any one of Z, Z¹, Z², X, X¹ and X² is -CH₂-(3-nitro-4-hydroxyphenyl), which corresponds to a side chain of 3-nitrotyrosine. In a further particular embodiment of the present invention, any one of Z, Z¹, Z², X, X¹ and X² is -CH₂-(4-methoxyphenyl) which corresponds to a side chain of O-methyltyrosine. In a further particular embodiment of the present invention, any one of Z, Z¹, Z², X, X¹ and X² is -CH₂-(3-hydroxy-4-methoxy-5-methylphenyl) which corresponds to a side chain of 3-OH-5-Me-OMe-tyrosine. In a further particular embodiment of the present invention, any one of Z, Z¹, Z², X, X¹ and X² is -CH₂-(4-prenyloxyphenyl) which corresponds to a side chain of O-prenyltyrosine. In a further particular embodiment of the present invention, any one of Z, Z¹, Z², X, X¹ and X² is -CH₂CH₂-phenyl which corresponds to a side chain of homophenylalanine. In a further particular embodiment of the present invention, any one of Z, Z¹, Z², X, X¹ and X² is -CH₂CH₂-(4-hydroxyphenyl) which corresponds to a side chain of homotyrosine. In a further particular embodiment of the present invention, any one of Z, Z¹, Z², X, X¹ and X² is -CH(-OH)-CH₂-(4-nitrophenyl) which corresponds to a side chain of β-OH-p-NO₂-homophenylalanine.

In a tenth specific embodiment of the present invention, any one of Z, Z¹, Z², X, X¹ and X² is -(C₀₋₆ alkylene)-heterocyclyl, wherein said heterocyclyl is optionally substituted with one or more groups R^{S}. Preferably, the heterocyclyl in said -(C₀₋₆ alkylene)-heterocyclyl is 1H-indol-3-yl. Further preferably, any one of Z, Z¹, Z², X, X¹ and X² is selected from -CH₂-(1H-indol-3-yl), - CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH_{z}-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), and -CH₂-(6,7-dichloro-1H-indol-3-yl). Thus, in a particular embodiment of the present invention, any one of Z, Z¹, Z², X, X¹ and X² is -CH₂-(1H-indol-3-yl) which corresponds to a side chain of tryptophan. In a further particular embodiment of the present invention, any one of Z, Z¹, Z², X, X¹ and X² is -CH(-CH₃)-(1H-indol-3-yl) which corresponds to a side chain of β-methyl-tryptophan. In a further particular embodiment of the present invention, any one of Z, Z¹, Z², X, X¹ and X² is - CH₂-(5-hydroxy-1H-indol-3-yl) which corresponds to a side chain of 5-hydroxytryptophan. In a further particular embodiment of the present invention, any one of Z, Z¹, Z², X, X¹ and X² is - CH₂-(1-acetyl-6-hydroxy-1H-indol-3-yl) which corresponds to a side chain of 6-hydroxy-N-acetyltryptophan. In a further particular embodiment of the present invention, any one of Z, Z¹, Z², X, X¹ and X² is -CH₂-(1-(1,1-dimethylallyl)-1H-indol-3-yl) which corresponds to a side chain of N-dimethylallyltryptophan. In a further particular embodiment of the present invention, any one of Z, Z¹, Z², X, X¹ and X² is -CH(-CH₃)-(2-oxo-indolin-3-yl) which corresponds to a side chain of oxo-β-methyl-tryptophan. In a further particular embodiment of the present invention, any one of Z, Z¹, Z², X, X¹ and X² is -CH₂-(4-nitro-1H-indol-3-yl) or -CHz-(5-nitro-1H-indol-3-yl) which corresponds to a side chain of nitrated tryptophan. In a further particular embodiment of the present invention, any one of Z, Z¹, Z², X, X¹ and X² is -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), or -CH₂-(7-bromo-1H-indol-3-yl), which correspond to a side chain of halogenated tryptophan.

In an eleventh specific embodiment of the present invention, any one of Z, Z¹, Z², X, X¹ and X² is -(C₀₋₆ alkylene)-heterocyclyl, wherein said heterocyclyl is optionally substituted with one or more groups R^{S}, any one of Z, Z¹, Z², X, X¹ and X² is preferably selected from -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), and 2-iminohexahydropyrimidin-4-yl. In a particular embodiment of the present invention, any one of Z, Z¹, Z², X, X¹ and X² is -CH₂-(1H-imidazol-4-yl) which corresponds to a side chain of histidine. In a further particular embodiment of the present invention, any one of Z, Z¹, Z², X, X¹ and X² is -CH₂-(5-mercapto-1-methyl-1H-imidazol-4-yl) which corresponds to a side chain of ovothiol. In a further particular embodiment of the present invention, any one of Z, Z¹, Z², X, X¹ and X² is -CH₂-(3-furanyl) which corresponds to a side chain of 3-(3-furyl)-alanine. In a further particular embodiment of the present invention, any one of Z, Z¹, Z², X, X¹ and X² is pyrrolidin-2-yl which corresponds to a side chain of tambroline.

In a twelfth specific embodiment of the present invention, any one of Z, Z¹, Z², X, X¹ and X² is cycloalkyl or -(C₁₋₆ alkylene)-cycloalkyl, wherein said cycloalkyl and the cycloalkyl group in said -(C₁₋₆ alkylene)-cycloalkyl are each optionally substituted with one or more groups R^{S}. Preferably, any one of Z, Z¹, Z², X, X¹ and X² is cycloalkyl, preferably a cyclopropyl, which may be optionally substituted with one or more groups R^{S}. Preferably, any one of Z, Z¹, Z², X, X¹ and X² is selected from 1-methylcycloprop-1-yl, and -CH₂-(2-nitrocycloprop-1-yl).

In a thirteenth specific embodiment, any one of Z, Z¹, Z², X, X¹ and X² is selected from C₁₋₈ alkyl, C₂₋₈ alkenyl, C₂₋₈ alkynyl, -(C₁₋₆ alkylene)-OH and -(C₁₋₆ alkylene)-O-( C₁₋₆ alkyl), wherein said alkyl, said alkenyl, said alkynyl, and said alkylene group are each optionally substituted with one or more -OH. Preferably, any one of Z, Z¹, Z², X, X¹ and X² is selected from -CH₂-OH, -CH₂CH₂-OH, -CH₂-O-CH₃, -CH₂CH₂-O-CH₃, -CH₂CH₂-O-CH₂CH₃, -CH(-CH₃)-OH, -C(-CH₃)(-OH)-CH₃, -CH(-OH)-CH(-CH₃)-CH₃, -CH(-OH)-CH(-CH₃)-CH₂-CH=CH-CH₃, and -CH(-OH)-C≡CH. In a particular embodiment of the present invention, any one of Z, Z¹, Z², X, X¹ and X² is -C(-CH₃)(-OH)-CH₃, which corresponds to a side chain of β-hydroxyvaline. In a further particular embodiment of the present invention, any one of Z, Z¹, Z², X, X¹ and X² is -CH(-OH)-CH(-CH₃)-CH₃, which corresponds to a side chain of β-hydroxyleucine. In a further particular embodiment of the present invention, any one of Z, Z¹, Z², X, X¹ and X² is -CH(-OH)-C≡CH, which corresponds to a side chain of β-ethynylserine. In a further particular embodiment of the present invention, any one of Z, Z¹, Z², X, X¹ and X² is -CH₂-OH which corresponds to a side chain of serine. In a further particular embodiment of the present invention, any one of Z, Z¹, Z², X, X¹ and X² is -CH₂CH₂-OH which corresponds to a side chain of homoserine. In a further particular embodiment of the present invention, any one of Z, Z¹, Z², X, X¹ and X² is -CH₂CH₂-O-CH₃ which corresponds to a side chain of O-methyl-homoserine. In a further particular embodiment of the present invention, any one of Z, Z¹, Z², X, X¹ and X² is -CH₂CH₂-O-CH₂CH₃ which corresponds to a side chain of O-ethyl-homoserine. In a further particular embodiment of the present invention, any one of Z, Z¹, Z², X, X¹ and X² is -CH(-CH₃)-OH which corresponds to a side chain of threonine.

In a fourteenth specific embodiment, any one of Z, Z¹, Z², X, X¹ and X² is -(C₁₋₆ alkylene)-SH or -(C₁₋₆ alkylene)-S-(C₁₋₆ alkyl). Preferably, any one of Z, Z¹, Z², X, X¹ and X² is selected from -CH₂-SH, -CH₂CH₂-SH, -CH₂CH₂-S-CH₃, and -CH₂CH₂-S-CH₂CH₃. In a particular embodiment of the present invention, any one of Z, Z¹, Z², X, X¹ and X² is -CH₂-SH which corresponds to a side chain of cysteine. In a further particular embodiment of the present invention, any one of Z, Z¹, Z², X, X¹ and X² is -CH₂CH₂-SH which corresponds to a side chain of homocysteine. In a further particular embodiment of the present invention, any one of Z, Z¹, Z², X, X¹ and X² is -CH₂CH₂-S-CH₃ which corresponds to a side chain of methionine. In a further particular embodiment of the present invention, any one of Z, Z¹, Z², X, X¹ and X² is -CH₂CH₂-S-CH₂CH₃ which corresponds to a side chain of ethionine.

In a fifteenth specific embodiment of the present invention, any one of Z, Z¹, Z², X, X¹ and X² is -(C₁₋₆ alkylene)-SO₃H or -(C₁₋₆ alkylene)-SO₃-(C₁₋₆ alkyl). Preferably, any one of Z, Z¹, Z², X, X¹ and X² is -CH₂-SO₃H. In a particular embodiment of the present invention, any one of Z, Z¹, Z², X, X¹ and X² is -CH₂-SO₃H which corresponds to a side chain of cysteate.

In a sixteenth specific embodiment of the present invention, any one of Z, Z¹, Z², X, X¹ and X² is -(C₁₋₆ alkylene)-COOH or -(C₁₋₆ alkylene)-COO-(C₁₋₆ alkyl), wherein said alkyl, and said alkylene group are each optionally substituted with one or more -OH. Preferably, any one of Z, Z¹, Z², X, X¹ and X² is -(C₁₋₆ alkylene)-COOH. Preferably, in certain embodiments any one of Z, Z¹, Z², X, X¹ and X² is selected from -CH₂-COOH, -CH₂CH₂-COOH, -CH(-OH)-COOH, -CH(-OH)-CH₂-COOH, -CH(-CH₃)-COOH, -CH(-CH₃)-CH₂-COOH. In a particular embodiment of the present invention, any one of Z, Z¹, Z², X, X¹ and X² is -CH₂-COOH, which corresponds to a side chain of aspartate.

In a seventeenth specific embodiment of the present invention, any one of Z, Z¹, Z², X, X¹ and X² is -(C₁₋₆ alkylene)-CO-NH₂, -(C₁₋₆ alkylene)-CO-N(C₁₋₆ alkyl)H or -(C₁₋₆ alkylene)-CO-N(C₁₋₆ alkyl)-(C₁₋₆ alkyl), wherein said alkyl, and said alkylene group are each optionally substituted with one or more -OH. Preferably, any one of Z, Z¹, Z², X, X¹ and X² is -(C₁₋₆ alkylene)-CO-NH₂. Preferably, in certain embodiments any one of Z, Z¹, Z², X, X¹ and X² is selected from -CH₂-CO-NH₂, -CH₂CH₂-CO-NH₂, -CH(-OH)-CO-NH₂, and -CH₂-NH-CO-NH₂.

In an eighteenth specific embodiment of the present invention, any one of Z, Z¹, Z², X, X¹ and X² is selected from -(CH₂)₂-NH-CO-NH₂, -(CH₂)₃-NH-CO-NH₂, and -(CH₂)₂-CO-NH-OH.

In a nineteenth specific embodiment of the present invention, any one of Z, Z¹, Z², X, X¹ and X² is -(C₁₋₆ alkylene)-NH₂, -(C₁₋₆ alkylene)-N(C₁₋₆ alkyl)-H or -(C₁₋₆ alkylene)-N(C₁₋₆ alkyl)-(C₁₋₆ alkyl), and wherein said alkylene group is optionally substituted with one or more -OH. Preferably, any one of Z, Z¹, Z², X, X¹ and X² is -(C₁₋₆ alkylene)-NH₂. Preferably, any one of Z, Z¹, Z², X, X¹ and X² is selected from -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, and -(CH₂)₂-C(-OH)-C(-CH₂-OH)-NH₂. In a particular embodiment of the present invention, any one of Z, Z¹, Z², X, X¹ and X² is -(CH₂)₃-NH₂, which corresponds to the side chain of ornithine. In a further particular embodiment of the present invention, any one of Z, Z¹, Z², X, X¹ and X² is -(CH₂)₄-NH₂, which corresponds to the side chain of lysine.

In a twentieth specific embodiment of the present invention, any one of Z, Z¹, Z², X, X¹ and X² is selected from -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, and -CH₂-C(-OH)-CH₂CH₂-NH-C(=NH)-NH₂. In a particular embodiment, any one of Z, Z¹, Z², X, X¹ and X² is -(CH₂)₃-NH-C(=NH)-NH₂, which corresponds to the side chain of arginine.

In a twenty-first specific embodiment, any one of Z, Z¹, Z², X, X¹ and X² is -CH(-OH)-CH(-OH)-CH₂-O-CO-NH₂.

In a twenty-second specific embodiment, any one of Z, Z¹, Z², X, X¹ and X² is C₁₋₈ alkyl, wherein one -CH₂- group in said alkyl is replaced with 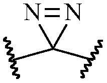 Preferably, in this specific embodiment, any one of Z, Z¹, Z², X, X¹ and X² is (3-methyl-diazirin-3-yl)methyl.

In a twenty-third specific embodiment, X, which can be a side chain of a natural or a nonnatural amino acid, is selected from any one of the following groups:

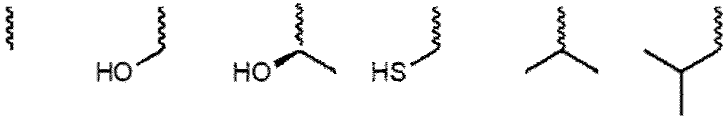

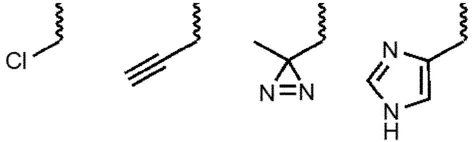

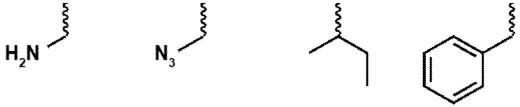

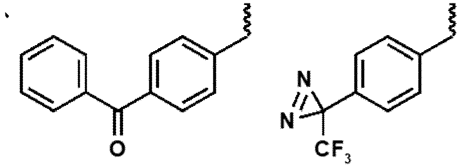

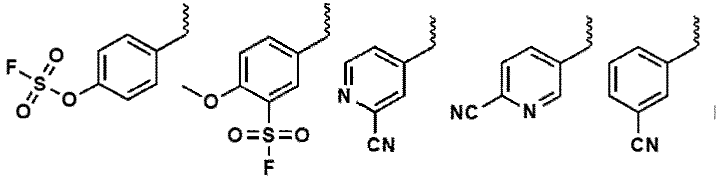

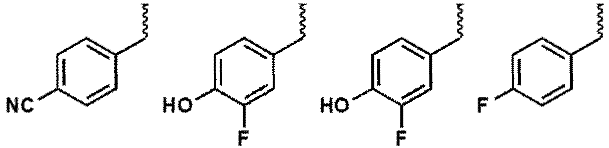

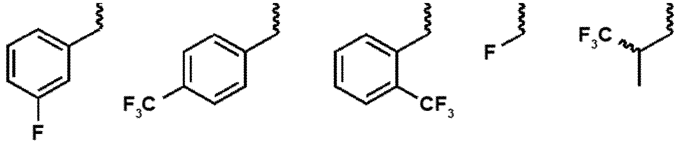

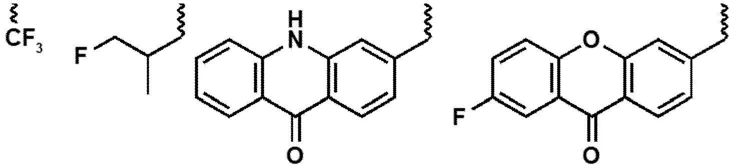

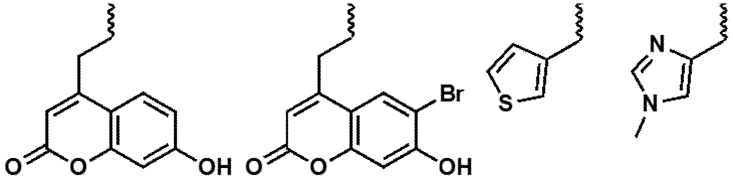

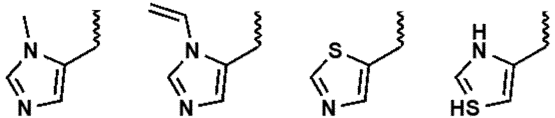

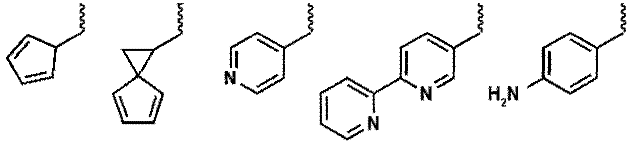

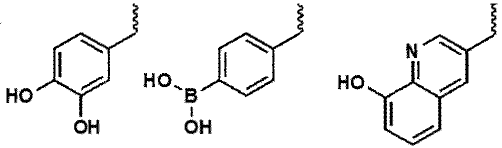

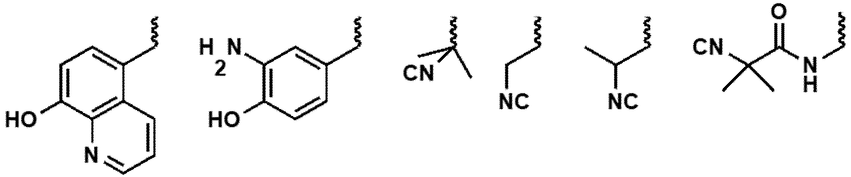

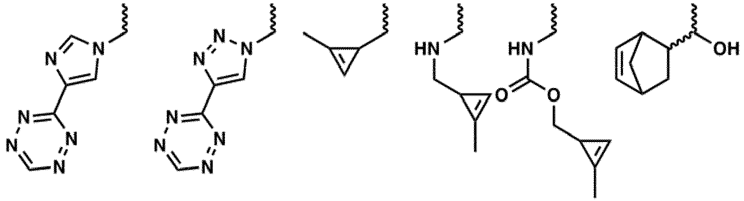

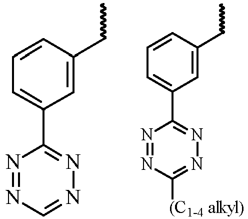

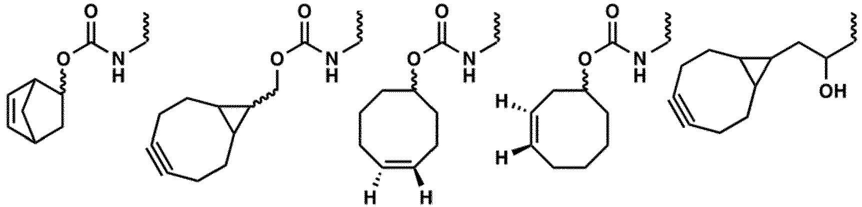

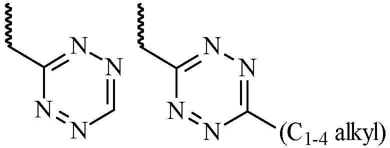

As understood herein, the wavy bond indicates the point of attachment of the monovalent group.

In a twenty-fourth specific embodiment, Z, which can be a side chain of a natural or a nonnatural amino acid, is selected from any one of the following groups:

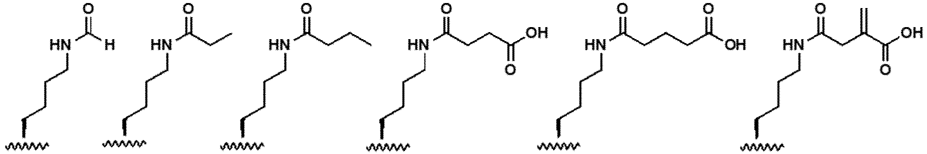

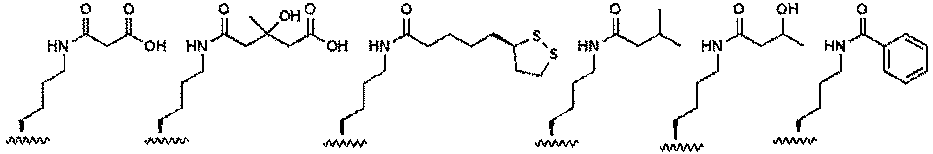

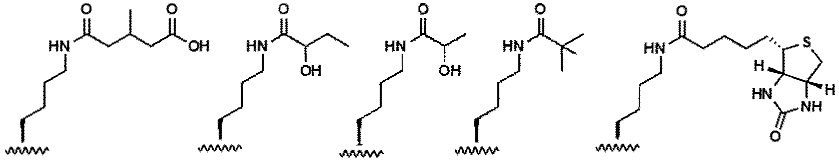

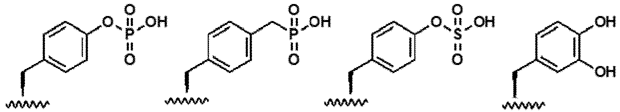

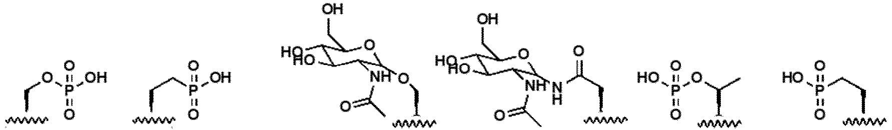

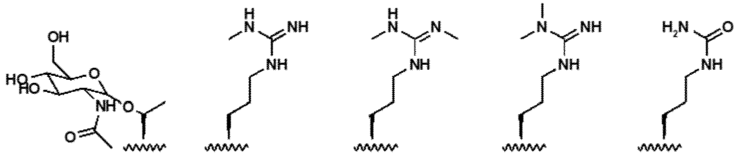

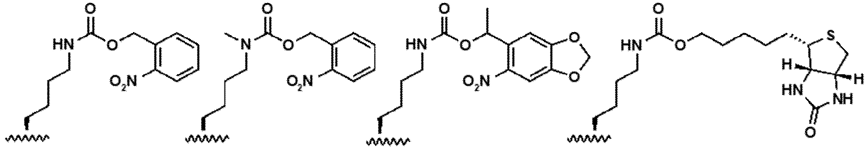

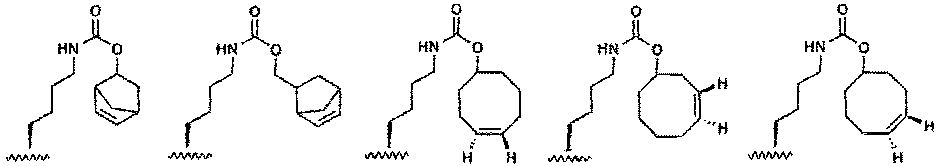

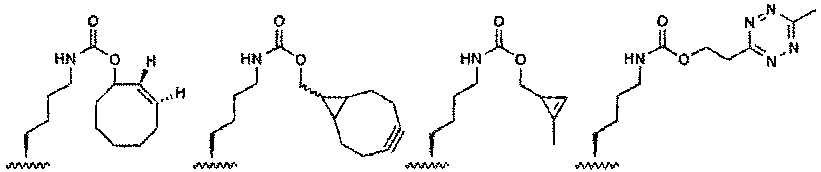

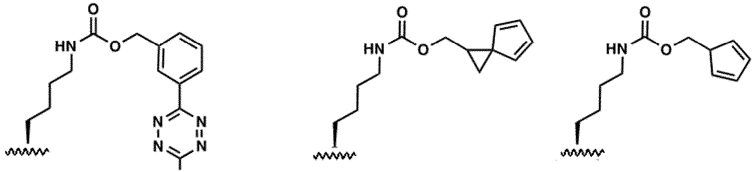

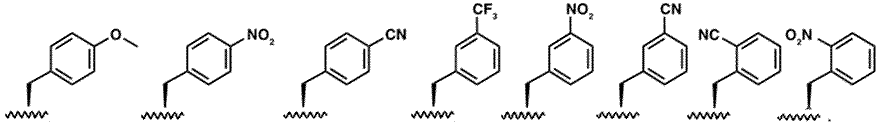

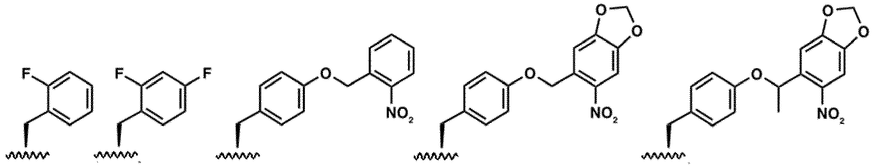

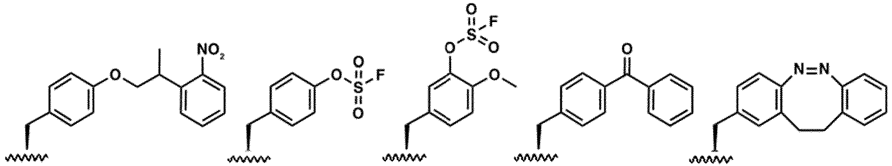

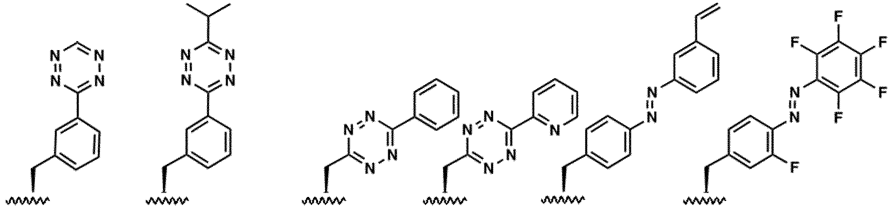

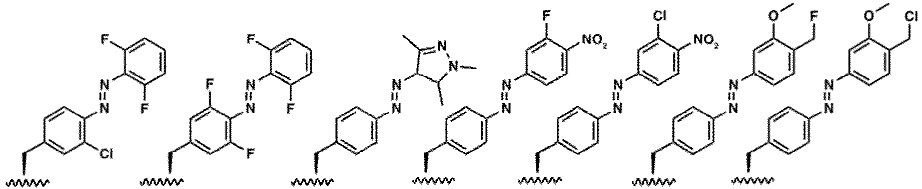

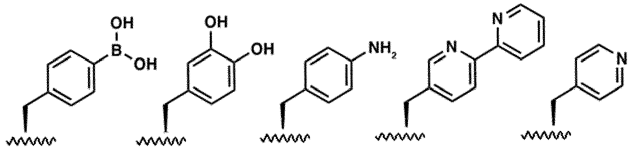

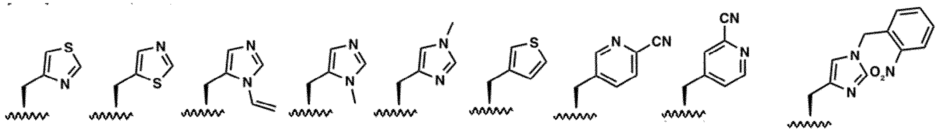

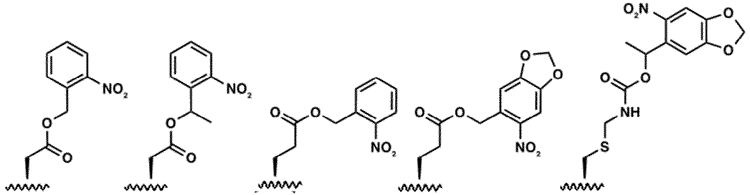

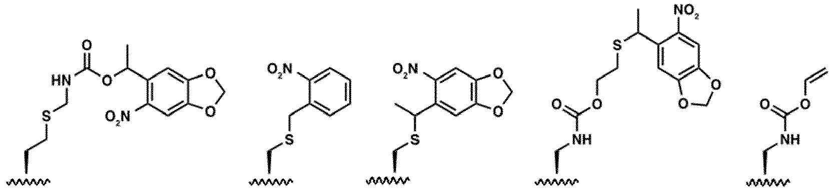

In a twenty-fifth specific embodiment of the compound of formula (I), B is selected from: 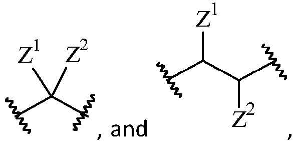 wherein the left empty valence is connected to A and the right empty valence is connected to C.

In a twenty sixth specific embodiment of the compound of formula (I), -C-D- taken together is -CO-(N-heterocycloalkylene)-, wherein said N-heterocycloalkylene is connected to said -CO-group of -C-D- through its nitrogen atom and wherein said N-heterocycloalkylene is optionally substituted with one or more R^{S}. Preferably, in this specific embodiment, -C-D- is a moiety according to formula 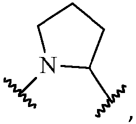 wherein the left empty valence is connected to B, and the right empty valence is connected to -CO-NH- group.

In a twenty seventh specific embodiment of the compound of formula (I), X is -(C₁₋₆ alkylene)-Hal, in particular -(C₁₋₆ alkylene)-Br or -(C₁₋₆ alkylene)-Cl.

The following definitions apply throughout the present specification and the claims, unless specifically indicated otherwise.

The term "hydrocarbon group" refers to a group consisting of carbon atoms and hydrogen atoms.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₆ alkenyl" denotes an alkenyl group having 2 to 6 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₆ alkynyl" denotes an alkynyl group having 2 to 6 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₆ alkylene" denotes an alkylene group having 1 to 6 carbon atoms, and the term "C₀₋₆ alkylene" indicates that a covalent bond (corresponding to the option "C₀ alkylene") or a C₁₋₆ alkylene is present. Preferred exemplary alkylene groups are methylene (-CH₂-), ethylene (e.g., -CH₂-CH₂-or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkenyl or cycloalkyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkenyl or heterocycloalkyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members (e.g., cyclopropyl or cyclohexyl).

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As understood herein, the term "heterocycloalkylene" refers to a heterocycloalkyl group, as defined herein above, but having two points of attachment, i.e. a divalent saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkylene" may, e.g., refer to aziridinylene, azetidinylene, pyrrolidinylene, imidazolidinylene, pyrazolidinylene, piperidinylene, piperazinylene, azepanylene, diazepanylene (e.g., 1,4-diazepanylene), oxazolidinylene, isoxazolidinylene, thiazolidinylene, isothiazolidinylene, morpholinylene, thiomorpholinylene, oxazepanylene, oxiranylene, oxetanylene, tetrahydrofuranylene, 1,3-dioxolanylene, tetrahydropyranylene, 1,4-dioxanylene, oxepanylene, thiiranylene, thietanylene, tetrahydrothiophenylene (i.e., thiolanylene), 1,3-dithiolanylene, thianylene, 1,1-dioxothianylene, thiepanylene, decahydroquinolinylene, decahydroisoquinolinylene, or 2-oxa-5-aza-bicyclo[2.2.1]hept-5-ylene. Unless defined otherwise, "heterocycloalkylene" preferably refers to a divalent 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkylene" refers to a divalent 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "N-heterocycloalkylene" refers to the heterocycloalkylene group as defined hereinabove wherein said heterocycloalkylene includes at least one nitrogen atom which serves as an attachment point of said heterocycloalkylene.

As used herein, the term "halogen" refers to fluoro (-F), chloro (-CI), bromo (-Br), or iodo (-I). The terms "Hal" and "halogen" can be used interchangeably.

As used herein, the term "nitro" refers to a group -NO₂.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

The term "peptide" refers to a polymer of two or more amino acids linked via amide bonds that are formed between an amino group of one amino acid and a carboxylic acid group of another amino acid. The amino acids comprised in the peptide or protein, which are also referred to as amino acid residues, may be selected from the 20 standard proteinogenic α-amino acids (i.e., Ala, Arg, Asn, Asp, Cys, Glu, Gln, Gly, His, Ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), labionin, or an alanine or glycine that is substituted at the side chain with a cyclic group such as, e.g., cyclopentylalanine, cyclohexylalanine, phenylalanine, naphthylalanine, pyridylalanine, thienylalanine, cyclohexylglycine, or phenylglycine) as well as β-amino acids (e.g., β-alanine), γ-amino acids (e.g., γ-aminobutyric acid, isoglutamine, or statine) and δ-amino acids. Preferably, the amino acid residues comprised in the peptide or protein are selected from α-amino acids, more preferably from the 20 standard proteinogenic α-amino acids (which can be present as the L-isomer or the D-isomer, and are preferably all present as the L-isomer). The peptide may be unmodified or may be modified, e.g., at its N-terminus, at its C-terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PG & Greene TW, Greene's protective groups in organic synthesis, John Wiley & Sons, 2006. Such modifications may also include the covalent attachment of one or more polyethylene glycol (PEG) chains (forming a PEGylated peptide), the glycosylation and/or the acylation with one or more fatty acids (e.g., one or more C8-30 alkanoic or alkenoic acids; forming a fatty acid acylated peptide or protein). Moreover, such modified peptides or proteins may also include peptidomimetics, provided that they contain at least two amino acids that are linked via an amide bond (formed between an amino group of one amino acid and a carboxyl group of another amino acid). The amino acid residues comprised in the peptide or protein may, e.g., be present as a linear molecular chain (forming a linear peptide) or may form one or more rings (corresponding to a cyclic peptide). The peptide may also form oligomers consisting of two or more identical or different molecules. The term "peptide" may also refer to a monovalent radical derived from a "peptide" as described herein above, wherein preferably an amino group or a carboxylic acid group, in particular the N-terminal amino group or the C-terminal carboxylic acid group, serves as point of attachment, preferably through an amide bond. Thus, the term "peptide" may refer to a peptidyl moiety attached to the rest of the molecule through a -CO- group (formed from a carboxylic acid group, e.g., from its C-terminal carboxylic acid group).

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

The scope of the invention embraces all salts, in particular all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a fumarate salt, a maleate salt, an oxalate salt, a malate salt, a tartrate salt, and a mesylate salt.

The present invention also specifically relates to the compound of formula (I), including any one of the specific compounds of formula (I) described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates and non-racemic mixtures). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

Throughout the present application, reference is made to an isopeptide-linked lysine or an analog thereof. This isopeptide-linked lysine or analog thereof is released when the covalent bond C of the compound of formula (I) according to the invention is hydrolyzed inside the cell. Accordingly, the isopeptide-linked lysine or analog thereof has the following structure according to formula (IV): 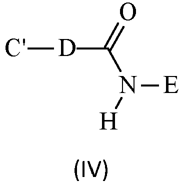
wherein C' is -NH₂ or -OH,
D is as defined for formula (I) (with the difference that its left empty valence is connected to C' instead of C), in particular D is selected from 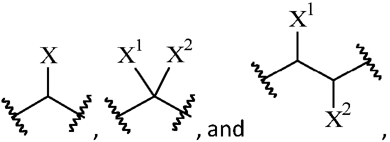 wherein the left empty valence is connected to C' and the right empty valence is connected to -CO-NH-, wherein X, X¹ and X² are as defined for formula (I), including any preferred definition and any specific embodiment of the compound of formula (I), and
wherein E is as defined for formula (I), in particular wherein E is -(C₁₋₆ alkylene)-CHY¹Y², wherein Y¹ is -NH₂ and Y² is COOH, preferably wherein the configuration on the carbon atom that carries Y¹ and Y² is the same as in L-lysine.

In another aspect, the invention relates to a method for identifying an engineered peptide-binding protein that promotes uptake of the compound according to the present invention, the method comprising the steps of:
a) providing a nucleic acid encoding a peptide-binding protein comprising one or more mutations;
b) expressing the nucleic acid of step (a) in a cell comprising an orthogonal translation system, wherein the orthogonal translation system comprises an orthogonal aminoacyl-tRNA synthetase/tRNA pair specific for an isopeptide-linked lysine or an analog thereof, and a reporter protein gene comprising one or more codons that have been re-allocated for the incorporation of the isopeptide-linked lysine or the analog thereof by the orthogonal aminoacyl-tRNA synthetase/tRNA pair;
c) contacting the cell of step (b) with a compound according to the invention, wherein the compound is transported into the cell and hydrolyzed inside the cell to release the isopeptide-linked lysine or the analog thereof;
d) detecting the expression of the reporter protein gene by the cell, wherein the expression is dependent on the successful uptake of the compound in step (c); and
e) identifying the engineered peptide-binding protein that promotes uptake of the compound according to the invention based on the expression of the reporter protein gene detected in step (d).

That is, another aspect of the invention relates to a method for engineering a peptide-binding protein to promote the uptake of the compound according to the invention. It has been shown in the appended experimental Examples that engineering of the peptide-binding protein OppA from E. coli resulted in higher affinity and selectivity for the compounds according to the invention, i.e., compounds comprising an isopeptide-linked lysine residue or an analog thereof, which resulted in increased cellular uptake of these compounds; see Example 4.

Herein, an engineered peptide-binding protein is defined as promoting the uptake of the compound of the invention if, when expressed by a cell, it leads to increased intracellular accumulation of the compound compared to a cell expressing the non-engineered counterpart. Intracellular levels of the compound may be measured, for example, by HPLC as described herein.

Alternatively or in addition, the method of the invention may be used to identify an engineered peptide-binding protein having increased affinity and/or selectivity for the compound according to the present invention.

Thus, in certain embodiments, the invention relates to a method for identifying an engineered peptide-binding protein having increased affinity and/or selectivity for the compound according to the present invention, the method comprising the steps of:
a) providing a nucleic acid encoding a peptide-binding protein comprising one or more mutations;
b) expressing the nucleic acid of step (a) in a cell comprising an orthogonal translation system, wherein the orthogonal translation system comprises an orthogonal aminoacyl-tRNA synthetase/tRNA pair specific for an isopeptide-linked lysine or an analog thereof, and a reporter protein gene comprising one or more codons that have been re-allocated for the incorporation of the isopeptide-linked lysine or the analog thereof by the orthogonal aminoacyl-tRNA synthetase/tRNA pair;
c) contacting the cell of step (b) with a compound according to any one of claims 1 to 16, wherein the compound is transported into the cell and hydrolyzed inside the cell to release the isopeptide-linked lysine or the analog thereof;
d) detecting the expression of the reporter protein gene by the cell, wherein the expression is dependent on the successful uptake of the compound in step (c); and
e) identifying the engineered peptide-binding protein as having increased affinity and/or selectivity for the compound according to any one of claims 1 to 16 based on the expression of the reporter protein gene detected in step (d).

A peptide-binding protein is defined to have increased affinity for the compound of the invention if it demonstrates a stronger binding interaction with the compound compared to its non-engineered counterpart. This increased affinity can be quantified by measuring the dissociation constant (Kd) for the compound of the invention. A lower Kd value indicates a higher affinity, as it reflects a tighter binding interaction between the peptide-binding protein and the compound.

A peptide-binding protein is defined to have increased selectivity for the compound of the invention if its preference for the compound compared to linear peptides is greater than that of a non-engineered counterpart. This increased selectivity can be quantified by comparing the dissociation constants (Kd) for the compound of the invention and the linear peptides, and calculating the selectivity ratio (SR). The selectivity ratio is defined as the ratio of the Kd for the linear peptides to the Kd for the compound of the invention (SR = Kd (linear peptide) / Kd (compound according to the invention)). A higher selectivity ratio indicates greater selectivity for the compound of the invention, demonstrating that the engineered protein has a stronger preference for the compound compared to its non-engineered counterpart.

A higher selectivity ratio indicates greater selectivity for the compound of the invention. For instance, if the affinity for the compound of the invention remains the same while the affinity for linear peptides decreases, the selectivity ratio will increase, demonstrating that the engineered protein has a stronger preference for the compound of the invention.

That is, a higher selectivity ratio for the compound according to the invention may be achieved by engineering the peptide-binding protein such that its affinity for the compound of the invention is maintained or increased, while its affinity for linear peptides is decreased. This approach ensures that the protein preferentially binds to the compound of the invention over other similar peptides, thereby enhancing its specificity.

Alternatively, a higher selectivity ratio for the compound according to the invention may also be achieved by engineering the peptide-binding protein to increase its affinity for the compound of the invention while maintaining or decreasing its affinity for linear peptides. This strategy similarly enhances the protein's preference for the target compound, ensuring that it binds more effectively and selectively to the compound of the invention compared to linear peptides.

In certain embodiments, an engineered peptide-binding protein is determined to have a higher affinity for the compound according to the invention, if the affinity for the compound according to the invention is at least 10%, 20%, 30%, 40%, 50%, 60%, 70%, 80%, 90%, 100%, 200%, 300%, 400% or 500% higher than that of the non-engineered counterpart.

In certain embodiments, an engineered peptide-binding protein is determined to have a higher selectivity for the compound according to the invention, if the selectivity ratio for the compound according to the invention is at least 10%, 20%, 30%, 40%, 50%, 60%, 70%, 80%, 90%, 100%, 200%, 300%, 400% or 500% higher than that of the non-engineered counterpart.

To standardize the determination of the affinity and the selectivity ratio, the same compounds as in Example 4 may be used. That is, as an example for the compound according to the invention, the compound G-SlsoK may be used and as an example for a linear peptide, the peptide GSK may be used. These compounds differ only in that in G-SlsoK, the lysine residue is attached to the serine residue via an isopeptide bond formed between the carboxyl group of the serine and the amino group in the side chain of the lysine.

The skilled person is aware of methods to determine the affinity of a protein for a ligand. For example, microscale thermophoresis may be used as described in appended Example 4. Alternative methods known to the person skilled in the art include, without limitation, isothermal titration calorimetry, surface plasmon resonance, fluorescence anisotropy, and equilibrium dialysis. These techniques provide reliable measurements of binding constants (Kd), allowing for the accurate assessment of protein-ligand interactions and the determination of binding affinities.

The starting point for the method of the invention is a nucleic acid encoding a peptide-binding protein. A peptide-binding protein is a type of protein that has the ability to specifically recognize and bind to peptide molecules. These proteins typically contain binding sites that interact with the peptide's amino acid residues through various non-covalent interactions, such as hydrogen bonds, ionic interactions, and hydrophobic effects.

The peptide-binding protein may be any peptide-binding protein from any organism. However, it is preferred herein, that the peptide-binding protein is the substrate-binding protein of an ABC transporter, in particular a prokaryotic ABC transporter. An ATP-binding cassette (ABC) transporter is a type of membrane protein complex that utilizes the energy derived from the hydrolysis of adenosine triphosphate (ATP) to transport various molecules across cellular membranes. ABC transporters are characterized by their ATP-binding domains (nucleotide-binding domains or NBDs) and transmembrane domains (TMDs) that form the pathway for substrate transport. A critical component of many ABC transporters, particularly in prokaryotes, is the substrate-binding protein (SBP). The SBP specifically binds to the substrate (e.g., a peptides) with high affinity and delivers it to the transmembrane components of the transporter. This interaction ensures the specificity and efficiency of the transport process, allowing the substrate to be translocated into the cell in an ATP-dependent manner. In bacteria, the substrate-binding protein is typically a periplasmic protein (in Gramnegative bacteria) or an extracellular protein that is tethered to the outer membrane (in Gram-positive bacteria).

That is, in certain embodiments, the peptide-binding protein is preferably a peptide-binding protein of a prokaryotic peptide transporter. In a particular embodiment, the invention relates to the method according to the invention, wherein the peptide-binding protein is the peptide-binding protein of a dipeptide permease (DppA),an oligopeptide permease (OppA) or a murein peptide-binding protein (MppA).

Dipeptide permease is a type of ABC transporter system found in bacteria, such as E. coli, that is specialized in the uptake and transport of dipeptides across the cell membrane. The system comprises several components, including a periplasmic dipeptide-binding protein (DppA), which specifically binds dipeptides in the periplasmic space. Once bound, the dipeptide is delivered to the transmembrane components of the system (DppBCDF), which facilitate its transport into the cytoplasm using the energy derived from ATP hydrolysis. The Dpp system plays a crucial role in nutrient acquisition, allowing the bacterium to utilize dipeptides as sources of amino acids and nitrogen for growth and metabolism.

Dipeptide permease (Dpp) and oligopeptide permease (Opp) are types of ATP-binding cassette (ABC) transporter systems found in bacteria, such as E. coli, that specialize in the uptake and transport of peptides across the cell membrane. The Dpp system is responsible for the transport of dipeptides, while the Opp system handles oligopeptides (short chains of amino acids). Both systems consist of several key components, including a periplasmic peptide-binding protein (DppA for dipeptides and OppA for oligopeptides), which specifically binds the respective peptides in the periplasmic space. Once bound, the peptides are delivered to the transmembrane components of the systems (DppBCDF for dipeptides and OppBCDF for oligopeptides), which facilitate their transport into the cytoplasm using the energy derived from ATP hydrolysis. These permease systems are essential for nutrient acquisition, enabling the bacterium to utilize peptides as sources of amino acids and nitrogen, thereby supporting growth and metabolic processes.

MppA is a periplasmic substrate-binding protein that is part of the murein tripeptide permease system in bacteria. MppA specifically binds to murein tripeptides, which are degradation products of peptidoglycan, in the periplasmic space. Once bound, MppA delivers the murein tripeptides to the transmembrane components of the Opp system (OppBCDF), which then transport the peptides into the cytoplasm using the energy derived from ATP hydrolysis. The Mpp system, utilizing the OppBCDF components, plays a crucial role in recycling cell wall components and in nutrient acquisition, allowing the bacterium to utilize murein tripeptides as sources of amino acids and nitrogen for growth and metabolism.

In a preferred embodiment, the starting point for the method according to the invention is the peptide-binding protein OppA. It has been demonstrated in Example 4 that OppA can be engineered to have a higher specificity for the compounds according to the invention. The OppA may be derived from any organism. More preferably, the OppA is a prokaryotic OppA. In a most preferred embodiment, the OppA is derived from E. coli. Thus, in a particular embodiment, the invention relates to the method according to the invention, wherein the peptide-binding protein is the peptide-binding protein of the E. coli oligopeptide permease (OppA; SEQ ID NO:1).

In a first step of the method of the invention, a nucleic acid encoding a peptide-binding protein comprising one or more mutations is provided. The one or more mutations may be introduced into the nucleic acid encoding the peptide-binding protein by any suitable method known in the art. The skilled person is aware of such methods.

Mutations may be introduced into the nucleic acid in a random or site-directed manner. Random mutagenesis techniques, such as error-prone PCR, can be used to generate a diverse library of variants by introducing mutations throughout the entire gene. Alternatively, site-directed mutagenesis allows for the precise introduction of specific mutations at predetermined positions within the gene, enabling the study of the effects of particular amino acid changes on the protein's function.

In a particular embodiment, the invention relates to the method according to the invention, wherein the mutations introduced in step (a) are introduced using error-prone PCR and/or saturated mutagenesis and/or continuous directed evolution.

Error-prone PCR is a molecular biology technique used to introduce random mutations into a specific DNA sequence during the amplification process. Unlike standard PCR, which aims to correctly replicate the DNA template, error-prone PCR employs modified reaction conditions, such as the use of a low-fidelity DNA polymerase, imbalanced nucleotide concentrations, or the inclusion of mutagenic agents, to increase the error rate of DNA synthesis. This results in the generation of a diverse library of mutant DNA sequences, each containing various point mutations. Error-prone PCR is particularly useful in directed evolution experiments, where the goal is to create a wide array of genetic variants that can be screened for desirable traits. Error-prone PCT may be used to identify positions in a protein that have an impact on the functionality of the protein. For example, error-prone PCR may be used to identify positions in a peptide-binding protein that are involved in binding the compound of the invention.

Saturated mutagenesis is a molecular biology technique used to generate a comprehensive library of genetic variants by introducing all possible nucleotide substitutions at specific positions within a target DNA sequence. This method involves systematically mutating one or more codons to include every possible amino acid residue, thereby creating a diverse set of protein variants. Saturated mutagenesis is typically performed using synthetic oligonucleotides that contain degenerate codons at the desired mutation sites, which are then incorporated into the target gene through techniques such as PCR or site-directed mutagenesis. This approach allows researchers to explore the functional impact of every possible amino acid change at specific positions, making it a valuable tool for studying structure-function relationships, optimizing protein properties, and engineering proteins with enhanced or novel functionalities. Saturated mutagenesis may be used to mutagenize positions in a peptide-binding protein that have previously been identified by error-prone PCR to have an impact on peptide-binding.

Continuous directed evolution is a powerful technique used to evolve proteins or nucleic acids in a continuous, iterative manner to achieve desired traits or functions. Unlike traditional directed evolution methods that rely on discrete rounds of mutagenesis and selection, continuous directed evolution integrates these processes into a single, ongoing cycle. This is typically achieved by coupling the evolutionary process to the replication or survival of the host organism, often using systems such as phage-assisted continuous evolution (PACE) or yeast surface display. In these systems, genetic variants that exhibit improved or desired characteristics are preferentially replicated or survive better under selective conditions, thereby enriching the population for beneficial mutations over time. Continuous directed evolution allows for the rapid and efficient optimization of biomolecules, enabling the development of proteins with enhanced activities, specificities, or stabilities, as well as the discovery of novel functions.

To identify peptide-binding proteins with high specificity for the compound of the invention, an initial round of mutagenesis may be conducted using error-prone PCR. This technique introduces random mutations throughout the peptide-binding protein, allowing for the identification of positions potentially involved in peptide binding. Following this, a second round of mutagenesis may be performed using saturated mutagenesis at the positions identified by error-prone PCR. This approach systematically introduces all possible amino acid substitutions at these specific sites, enabling the verification of their involvement in peptide binding and the identification of preferred amino acid residues. Additionally, multi-site saturation mutagenesis can be employed to explore the combinatorial effects of mutations at multiple positions, further refining the binding specificity and affinity of the peptide-binding protein for the compound of the invention. This iterative process of mutagenesis and selection facilitates the development of highly specific and optimized peptide-binding proteins.

In Example 4, the inventors have identified positions in E. coli OppA (SEQ ID NO:1) by error-prone PCR that potentially play a role in binding of the compound of the invention. These positions involve: L78, T173, V193, D221, W222, I303, K307, K333, N337, K371, R439, T429, S460, and/or V482 of SEQ ID NO:1.

Thus, in a particular embodiment, the invention relates to the method according to the invention, wherein in step (a) one or more mutations are introduced at positions L78, T173, V193, D221, W222, I303, K307, K333, N337, K371, R439, T429, S460, and/or V482 of SEQ ID NO:1.

Positions of particular interest in E. coli OppA (SEQ ID NO:1) for binding the compound of the invention are R439 and S460. Mutations in these positions appeared more than once when error-prone PCR was used to identify mutations that can confer higher specificity to the compound of the invention. Similarly, mutations in position D221 and W222 were identified in the error-prone PCR screen, indicating that these neighboring positions are involved in binding of the compound of the invention.

Thus, in a preferred embodiment, the invention relates to the method according to the invention, wherein in step (a) one or more mutations are introduced at positions D221, W222, R439, and/or S460 of SEQ ID NO:1.

In certain embodiments, the nucleic acid encoding the peptide-binding protein comprising one or more mutations is comprised in a plasmid, in particular an expression plasmid comprising a promoter for expression of the peptide-binding protein comprising one or more mutations. The term "plasmid" as used herein refers to a small, circular, double-stranded DNA molecule that is distinct from a cell's chromosomal DNA. Plasmids are capable of autonomous replication within a host cell and are commonly found in bacteria, although they can also be present in archaea and eukaryotic organisms. In the context of genetic engineering and biotechnology, plasmids are often used as vectors to introduce foreign genetic material into a host cell. They typically contain an origin of replication, selectable marker genes, and multiple cloning sites, which facilitate the insertion and expression of target genes.

In certain embodiments, the nucleic acid encoding the peptide-binding protein comprising one or more mutations is comprised in a library of nucleic acids, preferably a plasmid library, wherein each member of the library preferably encodes a peptide-binding protein comprising one or more mutation.

The nucleic acid encoding the peptide-binding protein and comprising one or more mutations may then be expressed in a cell. To achieve this, the nucleic acid, or a plurality of nucleic acids, first needs to be introduced into the cells. The skilled person is aware of methods for introducing a nucleic acid into a cell, including, but not limited to, transformation, transfection, electroporation, and viral transduction. These techniques facilitate the uptake and integration of the nucleic acid into the host cell, enabling the subsequent expression of the engineered peptide-binding protein for further analysis and characterization.

The cell may be any type of cell. Preferably, the cell is a prokaryotic cell, more preferably An E. coli cell. Thus, in a particular embodiment, the invention relates to the method according to the invention, wherein the cell in step (b) is a prokaryotic cell, in particular an E. coli cell.

In a preferred embodiment, the nucleic acid encoding the peptide-binding protein comprising one or more mutations is a plasmid that is introduced into a cell, preferably a prokaryotic cell, using transformation and/or electroporation.

The term "transformation" refers to the process by which a cell takes up and expresses foreign DNA, such as a plasmid, from its surrounding environment. Transformation can be facilitated by various methods, including chemical treatment to make the cell membrane more permeable or by applying an electric field in a process known as electroporation, which creates temporary pores in the cell membrane through which the plasmid DNA can enter. These techniques enable the efficient introduction of the plasmid into the host cell, allowing for the expression of the engineered peptide-binding protein and subsequent analysis of its properties.

The skilled person is aware of the conditions necessary to achieve the expression of the peptide-binding protein comprising one or more mutations. Specifically, this involves the induction of the promoter that controls the expression of the mutated peptide-binding protein. These conditions may include the appropriate temperature, pH, and the presence of specific inducers or cofactors that activate the promoter. Additionally, the skilled person understands how to optimize these conditions to maximize protein expression, taking into account factors such as the choice of host cell, the strength of the promoter, and the efficiency of the translation and transcription machinery.

In certain embodiments, expression of the peptide-binding protein comprising one or more mutations is driven by an inducible or a constitutive promoter. Inducible promoters require specific conditions or the presence of certain molecules to initiate transcription. For example, the pBAD promoter is inducible by the sugar arabinose, allowing for controlled expression of the target gene. In contrast, constitutive promoters drive continuous expression of the gene regardless of environmental conditions. Other non-limiting examples of inducible promoters include the lac promoter, which is induced by the presence of lactose or its analog IPTG (isopropyl β-D-1-thiogalactopyranoside), and the tac promoter, which is a hybrid of the trp and lac promoters and is also inducible by IPTG.

In contrast, constitutive promoters drive continuous expression of the gene regardless of environmental conditions. These promoters are always active and do not require any external inducers. Examples of constitutive promoters that can be used in bacteria include the lacUV5 promoter, which is a mutant form of the lac promoter with higher activity, the T7 promoter, which is recognized by T7 RNA polymerase for high-level expression, and the rpsL promoter, which is derived from the ribosomal protein S12 gene and provides strong, consistent expression. By selecting the appropriate promoter and optimizing the induction conditions, the desired levels of expression of the peptide-binding protein can be reliably achieved.

The cell used for identifying peptide-binding proteins with increased affinity and/or selectivity for the compound of the invention comprises an orthogonal translation system that enables the incorporation of an isopeptide-linked lysine or an analog thereof into a protein of interest. This incorporation serves as a readout for the efficiency with which the compound of the invention is transported into the cell by peptide transporters. By quantifying the incorporation of the isopeptide-linked lysine or its analog into the protein of interest, the performance of peptide-binding proteins, and their mutants, in mediating the uptake of the target compound can be assessed, thereby identifying those with enhanced affinity and/or selectivity and transport efficiency.

Cells expressing peptide-binding proteins with increased affinity and/or selectivity for the compound of the invention will accumulate higher concentrations of the compound inside the cell. Once inside, the compound is hydrolyzed to release an isopeptide-linked lysine or an analog thereof. The availability of the isopeptide-linked lysine or its analog within the cell for the orthogonal translation system directly correlates with the specificity of the peptide-binding protein for the compound of the invention. Therefore, the incorporation of the isopeptide-linked lysine or its analog into the protein of interest serve as effective indicators of the peptide-binding protein's affinity and/or selectivity for the compound.

The term "orthogonal translation system" as used herein refers to a specialized translation machinery within a cell that operates independently of the cell's native translation system. This orthogonal system typically includes an orthogonal aminoacyl-tRNA synthetase/tRNA pair that is specific for a non-canonical amino acid, such as an isopeptide-linked lysine or an analog thereof. The orthogonal aminoacyl-tRNA synthetase charges the orthogonal tRNA with the non-canonical amino acid, which is then incorporated into a protein of interest at designated codon positions that have been re-allocated for this purpose. This system allows for precise control over the incorporation of non-standard amino acids into proteins, enabling the study and engineering of proteins with novel properties and functions. In the context of the invention, the orthogonal translation system is used to monitor the uptake and incorporation of the compound of the invention, providing a readout for the efficiency and specificity of peptide-binding proteins in transporting the target compound into the cell.

The orthogonal aminoacyl-tRNA synthetase may be any aminoacyl-tRNA synthetase that is capable of attaching an isopeptide-linked lysine or an analog thereof to an orthogonal tRNA.

However, it is preferred herein that the aminoacyl-tRNA synthetase is a pyrrolysyl-tRNA synthetase (PyIRS). Thus, in a particular embodiment, the invention relates to the method according the invention, wherein the orthogonal aminoacyl-tRNA synthetase/tRNA pair is a Pyrrolysyl-tRNA synthetase/tRNA pair.

Pyrrolysyl-tRNA synthetases are specific for their cognate tRNA and the non-canonical amino acid pyrrolysine. However, it has been demonstrated in the past that pyrrolysyl-tRNA synthetases also recognize other lysine-derivatives than pyrrolysine. For example, it was demonstrated in Example 3, that a PylRS from *Methanosarcina barkeri* (MbPyIRS; SEQ ID NO:2) can be used to incorporate various isopeptide linked lysins (XlsoK) into proteins. In addition, engineered variants of MbPyIRS were shown in Example 3 to have increased specificity for isopeptide linked lysins.

An overview of orthogonal PyIRS/tRNA^{Pyl} pairs are provided by Koch et al. (Int J Mol Sci. 2021 Oct 17;22(20):11194), Koch and Budisa (Chem Rev. 2024 Aug 28;124(16):9580-9608), and more recently by Dunkelmann and Chin (https://doi.org/10.1021/acs.chemrev.4c00243), which are incorporated herein in their entirety.

In certain embodiments, the PylRS is an archaeal PyIRS. In certain embodiments, the PylRS is derived from a *Methanosarcina* species, such as *Methanosarcina barkeri* or *Methanosarcina mazei.* In certain embodiments, the PylRS is derived from a *Methanomethylophilus* species, such as *Methanomethylophilus alvus.*

Thus, in a particular embodiment, the invention relates to the method according to the invention, wherein the pyrrolysyl-tRNA synthetase is the pyrrolysyl-tRNA synthetase from *Methanosarcina barkeri* (MbPyIRS; SEQ ID NO:2) or a variant thereof. In certain embodiments, the MbPylRS comprises one or more mutations at the following positions: Y271, and/or C313.

In certain embodiments, the pyrrolysyl-tRNA synthetase is the pyrrolysyl-tRNA synthetase from *Methanosarcina barkeri* (MbPyIRS) as set forth in SEQ ID NO:2.

In certain embodiments, the pyrrolysyl-tRNA synthetase is the pyrrolysyl-tRNA synthetase from *Methanosarcina barkeri* (MbPyIRS) as set forth in SEQ ID NO:3 comprising the mutation C313V.

In certain embodiments, the pyrrolysyl-tRNA synthetase is the pyrrolysyl-tRNA synthetase from *Methanosarcina barkeri* (MbPyIRS) as set forth in SEQ ID NO:4 comprising the mutations Y271L and C313T. In a particular embodiment, the invention relates to a protein comprising the amino acid set forth in SEQ ID NO:4.

In certain embodiments, the pyrrolysyl-tRNA synthetase is the pyrrolysyl-tRNA synthetase from *Methanomethylophilus alvus* (MaPyIRS) as set forth in SEQ ID NO:6 comprising the mutations H227I and Y228P.

In certain embodiments, the pyrrolysyl-tRNA synthetase is the pyrrolysyl-tRNA synthetase from *Methanosarcina barkeri* (MbPyIRS) as set forth in SEQ ID NO:2 comprising one or more mutations at positions: M241, M365, L266, A267, L270, Y271, L274, I287, M309, N311, C313, M315, V348, Y349, S364, V366, V370, L372, I378, W382, and/or G386. These positions of MbPyIRS have been reported to be involved in substrate binding.

In certain embodiments, the pyrrolysyl-tRNA synthetase is the pyrrolysyl-tRNA synthetase from *Methanosarcina mazei* (MmPyIRS) as set forth in SEQ ID NO:7 comprising one or more mutations at positions: M276, M300, L301, A302, L305, Y306, L309, I322, M344, N346, C348, M350, V383, Y384, S399, V401, I405, L407, I413, W417, and/or G421. These positions of MmPyIRS have been reported to be involved in substrate binding.

In certain embodiments, the pyrrolysyl-tRNA synthetase is the pyrrolysyl-tRNA synthetase from *Methanomethylophilus alvus* (MaPyIRS) as set forth in SEQ ID NO:8 comprising one or more mutations at positions: M96, L121, A122, L125, Y126, M129, I142, N166, V168, M170, V205, Y206, S221, A223, H227, L229, V235, W239, and/or G243. These positions of MaPyIRS have been reported to be involved in substrate binding.

In certain embodiments, the amino acyl-tRNA synthetase is a tyrosyl-tRNA synthetase from *Methanocaldococcus janaschii* (*Mj*TyrRS) as set forth in SEQ ID NO:9 comprising one or more mutations at positions: Y32, L65, H70, F108, Q109, D158, I159, and/or L162. These positions of *Mj*TyrRS have been reported to be involved in substrate binding.

In certain embodiments, the amino acyl-tRNA synthetase is a tyrosyl-tRNA synthetase from *Archaeoglobus fulgidus* (*Af*TyrRS) as set forth in SEQ ID NO:10 comprising one or more mutations at positions: Y36, L69, H74, Q116, D165, and/or I166. These positions of *Af*TyrRS have been reported to be involved in substrate binding.

In certain embodiments, the amino acyl-tRNA synthetase is a phosphoseryl-tRNA synthetase from *Methanococcus maripaludis (MmSep*RS) as set forth in SEQ ID NO:11 comprising one or more mutations at positions: E412, E414, P495, I496, and/or F529. These positions of *MmSep*RS have been reported to be involved in substrate binding.

With the teaching provided herein, the skilled person would have no difficulty in identifying a pyrrolysyl-tRNA synthetase (PyIRS) that is suitable for attaching an isopeptide-linked lysine, or an analog thereof, to its cognate tRNA. Furthermore, based on the teaching provided herein, identifying additional PylRS variants capable of attaching an isopeptide-linked lysine, or an analog thereof, to their cognate tRNA would not pose an undue burden on the skilled person. In this regard, it is worth noting that the process of identifying or evolving a new PylRS variant for a certain isopeptide-linked lysine amino acid is greatly facilitated by the higher concentration of the amino acid inside the cell due to the increased uptake of the compound of the invention. The methods and criteria for selecting and optimizing such PylRS variants are well within the expertise of those skilled in the art, ensuring efficient and accurate incorporation of the desired non-canonical amino acids into proteins.

The orthogonal translation system further comprises an orthogonal tRNA that is recognized by the aminoacyl-tRNA synthetase and can be loaded with the isopeptide-linked lysine or the analog thereof. The skilled person is aware of cognate aminoacyl-tRNA synthetase/tRNA pairs.

The orthogonal tRNA preferably comprises an engineered anticodon loop to enable incorporation of the isopeptide-linked lysine or the analog thereof in response to a re-allocated codon. For example, where the re-allocated codon is the amber stop codon TAG, the anticodon loop is engineered to comprise the anticodon CUA.

The orthogonal translation system is used to incorporate one or more isopeptide-linked lysins or the analogs thereof into a reporter protein in response to one or more re-allocated codons comprised in the gene encoding the reporter protein.

The term "reporter protein" as used herein refers to a protein that is used as a marker to monitor and measure biological processes, gene expression, or cellular events. Reporter proteins produce a detectable signal, such as fluorescence, luminescence, or enzymatic activity, which can be easily quantified using various analytical techniques. Preferably, the reporter protein is a fluorescent protein which allows for real-time visualization and quantification of protein expression.

Thus, in a particular embodiment, the invention relates to the method according to the invention, wherein the reporter protein is a fluorescent protein.

The term "fluorescent protein" as used herein refers to a type of protein that exhibits fluorescence, meaning it can absorb light at a specific wavelength and subsequently emit light at a longer wavelength. This property makes fluorescent proteins valuable tools in molecular and cellular biology for visualizing and tracking biological processes in real-time. Fluorescent proteins are often used as reporter proteins to monitor gene expression, protein localization, and interactions within living cells. The most well-known fluorescent protein is green fluorescent protein (GFP), originally derived from the jellyfish *Aequorea victoria.* Variants of GFP and other naturally occurring fluorescent proteins have been engineered to emit light in different colors, such as blue (BFP), cyan (CFP), yellow (YFP), and red (RFP), expanding their utility in multicolor imaging and multiplexed assays.

However, the reporter protein can also be an antibiotic resistance marker or an auxotrophic selection marker.

Antibiotic resistance markers, such as β-lactamase or neomycin phosphotransferase, confer resistance to specific antibiotics, enabling the selection of successfully modified cells by allowing them to grow in the presence of the antibiotic, while cells that do not express the antibiotic resistance marker are inhibited or killed.

Auxotrophic selection markers, on the other hand, involve genes that complement a nutritional deficiency in the host cell. For example, a cell that is auxotrophic for a particular amino acid or nucleotide can only grow if the medium is supplemented with that nutrient. By introducing a gene that restores the ability to synthesize the missing nutrient, only the cells that successfully express the gene can grow in minimal media lacking the supplemented nutrient.

In the context of this invention, reporter proteins are utilized to indicate the successful incorporation of non-canonical amino acids, such as isopeptide-linked lysine or its analogs, into a protein of interest. The expression and activity of the reporter protein serve as a readout for the efficiency and specificity of the underlying biological process, facilitating the identification and characterization of peptide-binding proteins with enhanced affinity and/or selectivity for the compound of the invention.

To enable the expression of the reporter protein gene, the gene comprises one or more codons that have been re-allocated for the incorporation of the isopeptide-linked lysine or an analog thereof by the orthogonal aminoacyl-tRNA synthetase/tRNA pair. Several codons have been described in the art that can be re-allocated for the incorporation of non-canonical amino acids. The most prominent example is the amber stop codon (TAG/UAG). In its native context, the amber stop codon signals the termination of protein synthesis. However, in an orthogonal translation system, the amber stop codon can be repurposed to incorporate non-canonical amino acids, such as isopeptide-linked lysine, into the protein of interest.

Thus, in a particular embodiment, the invention relates to the method according to the invention, wherein the reporter protein gene in step (b) comprises one or more amber stop codons that have been re-allocated to incorporate the isopeptide-linked lysine or the analog thereof.

In embodiments where the re-allocated codon is the amber stop codon, the cell preferably lacks release factor 1 (RF1) to improve incorporation of the non-canonical amino acid in response to the amber stop codon.

Besides the amber stop codon other codons may be re-allocated for the incorporation of the isopeptide-linked lysine or an analog thereof. For example, the re-allocated codon may be the ochre stop codon (TAA/UAA), a quadruplet codon (as described for example by Guo and Niu (J Mol Biol. 2022 Apr 30; 434(8): 167346)) or a liberated sense codon (as described by Robertson et al., (Science 372,1057-1062(2021).DOI:10.1126/science.abg3029)).

It is to be noted that the cell may also comprise two or more mutually orthogonal translation systems and the reporter protein gene may comprise two or more codons re-allocated for the incorporation of non-canonical amino acids. This is demonstrated in Example 5, where a first orthogonal translation system is used to incorporate a non-canonical amino acid in response to a first re-allocated codon (TAA) and a second orthogonal translation system is used to incorporate an isopeptide-linked lysine in response to a second re-allocated codon (TAG).

In a next step, cells comprising an orthogonal translation system and expressing a peptide-binding protein comprising one or more mutations are contacted with the compound according to the invention.

Whether and how efficiently the compound according to the invention can be taken up by the cell depends on the affinity and/or selectivity of the peptide-binding protein for the compound according to the invention. That is, if the compound according to the invention is selectively bound by the peptide-binding protein, it will be efficiently transported into the cell. Inside the cell, the compound according to the invention will then by hydrolyzed to release the isopeptide-linked lysine or the analog thereof, which can subsequently by incorporated into the reporter protein.

The term "contact" as used herein refers to the process of bringing two or more substances or entities into close proximity or direct interaction with each other. In the context of this invention, "contact" typically involves exposing a cell to a compound, such as the compound of the invention, under conditions that facilitate the interaction between the cell and the compound. This may include incubating the cell with the compound in a suitable medium, under appropriate temperature, pH, and other environmental conditions that support the uptake, binding, or reaction of the compound with cellular components. The term "contact" encompasses both direct physical interaction and indirect interactions facilitated by the surrounding environment, ensuring that the desired biological or chemical processes can occur effectively.

In certain embodiments, cells are contacted with the compound according to the invention in a growth medium. In certain embodiments, the growth medium is a defined growth medium. In certain embodiments, the defined growth medium contain no or little amounts of peptides that can compete with the compound according to the invention for binding to the peptide-binding protein.

However, if the aim of the method is to further increase the specificity of the peptide-binding protein for the compound according to the invention, the method may be repeated several times and the concentration of competing peptides in the medium may be increased in every reiteration. As such, peptide-binding proteins may be obtained that are highly specific for compounds comprising isopeptide-linked lysins or analogs thereof, but have only limited affinity to linear peptides.

Thus, in a particular embodiment, the invention relates to the method according to the invention, wherein in step (c), the cell is contacted with a compound according to the invention in the presence of a peptide that competes for binding to the peptide-binding protein.

As mentioned above, the compound according to the invention is hydrolyzed inside the cell to release the isopeptide-linked lysine or an analog thereof. Thus, in a particular embodiment, the invention relates to the method according to the invention, wherein the isopeptide-linked lysine or the analog thereof is obtained by intracellular cleavage of the compound of the invention.

If the isopeptide-linked lysine or its analog is linked to an amino acid residue via a peptide bond, the release can be catalyzed by intracellular peptidases. As demonstrated in the appended Examples, E. coli possesses various endogenous peptidases capable of catalyzing this reaction. Alternatively, the cell may be engineered to express a recombinant peptidase known for its promiscuous activity, thereby enhancing the efficiency of hydrolysis and ensuring the effective release of the isopeptide-linked lysine or its analog.

In certain embodiments, the cell may recombinantly express one or more peptidase selected from: pepA (SEQ ID NO:55), pepB (SEQ ID NO:56), pepT (SEQ ID NO:57), pepN (SEQ ID NO:58), ddpX (SEQ ID NO:59), ydpE (SEQ ID NO:60), ypdF (SEQ ID NO:61), frvX (SEQ ID NO:62), or sgcX (SEQ ID NO:63).

Of note, certain peptidases require the presence of a free N-terminal amino group to hydrolyze the peptide bond. Thus, it is preferred herein that when the bond C of the compound according to the invention is a peptide bond (CO-NH), then the group A is an amino group (-NH₂).

In certain embodiments, the isopeptide-linked lysine or its analog may be linked to an amino acid residue via an ester bond. In such embodiments, the release can be catalyzed by intracellular esterases. Alternatively, the cell may be engineered to express a recombinant esterase known for its promiscuous activity, thereby enhancing the efficiency of hydrolysis and ensuring the effective release of the isopeptide-linked lysine or its analog.

In certain embodiments, the cell may recombinantly express an esterase from Bacillus subtilis (SEQ ID NO:64).

It is preferred herein that when the group A of the compound according to the invention is not NH₂, the bond C is preferably an ester bond (i.e., C is -CO-O-, as defined herein), since esterases do not require the presence of an N-terminal amino group to recognize their targets.

In a next step, expression of the reporter protein gene is detected and, preferably, quantified in the cell. As explained herein above, the expression level of the reporter protein gene correlates with the efficiency with which the isopeptide-linked lysine, or the analog thereof, is incorporated into the reporter protein, which in turn corresponds with the efficiency with which the compound according to the invention is taken up by the cell, mediated by the engineered peptide-binding protein. Thus, the affinity and/or specificity of the peptide-binding protein for the compound according to the invention correlates with the expression of the reporter protein gene.

How the expression of the reporter protein gene can be detected and/or quantified depends on the type of reporter protein. If the reporter gene is a fluorescent protein, as described herein, expression of the reporter protein gene is preferably detected and quantified by flow cytometry. Flow cytometry is a powerful analytical technique used to measure and analyze the physical and chemical characteristics of individual cells or particles as they flow in a fluid stream through a beam of light, typically a laser. This technique allows for the rapid quantification of various parameters, such as cell size, granularity, and fluorescence intensity, which can be indicative of specific cellular properties or the presence of particular biomolecules. Flow cytometry is widely used in research and clinical settings for applications such as cell sorting, biomarker detection, and the assessment of cellular health and function.

A preferred embodiment of flow cytometry is fluorescence-activated cell sorting (FACS). FACS is a specialized type of flow cytometry that not only analyzes but also sorts cells based on their fluorescence characteristics. In FACS, cells are labeled with fluorescent markers. As the cells pass through the laser beam, their fluorescence is detected and measured. Based on the fluorescence intensity and other parameters, the cells are then sorted into different populations using an electrostatic deflection system. FACS allows for the precise isolation of specific cell types from heterogeneous mixtures.

Thus, in a particular embodiment, the invention relates to the method according to the invention, wherein the detection of the expression of the reporter protein gene is performed using fluorescence-activated cell sorting (FACS).

The skilled person is capable of isolating cells expressing high levels of the reporter protein gene by flow cytometry. In particular, the skilled person is capable of applying gating strategies to select cells that exhibit high fluorescence levels, effectively isolating those with elevated expression of the reporter protein gene.

Alternatively, selection strategies may be applied to detect and/or quantify the expression of the reporter protein gene. For example, the reporter protein gene may be an antibiotic resistance gene, and successful incorporation of the isopeptide-linked lysine or an analog thereof will result in the expression of this gene. In such embodiments, the level of expression of the antibiotic resistance gene directly correlates with the cell's ability to tolerate higher concentrations of the corresponding antibiotic. Therefore, cells expressing higher levels of the reporter protein gene will survive and proliferate in the presence of increasing antibiotic concentrations, allowing for the effective detection and quantification of gene expression.

When using antibiotic resistance markers as the reporter protein gene, multiple rounds of mutagenesis may be performed, with increasing concentrations of the antibiotic applied in each round. This iterative selection process ensures the enrichment of cells that exhibit enhanced incorporation of the isopeptide-linked lysine or its analog. By progressively challenging the cells with higher antibiotic concentrations, the selection pressure drives the evolution of peptide-binding proteins with improved specificity and efficiency for the compound of the invention.

Alternatively, growth-based selection strategies may be applied to detect and/or quantify the expression of the reporter protein gene. For example, the reporter protein gene may be an auxotrophic marker gene that complements a nutritional deficiency in the host cell. Successful incorporation of the isopeptide-linked lysine or an analog thereof will result in the expression of the auxotrophic marker gene. In such embodiments, cells expressing the reporter protein gene will be able to grow in minimal media lacking the supplemented nutrient that the auxotrophic marker gene restores. The higher the expression of the auxotrophic marker gene, the better the growth and survival of the cells under these selective conditions.

Thus, in a particular embodiment, the invention relates to the method according to the invention, wherein the detection of the expression of the reporter protein gene is performed using antibiotic resistance screening and/or using growth-based selection.

In the final step, cells that have been detected and isolated based on their ability to express the reporter protein gene are determined to express a peptide-binding protein having increased affinity and/or selectivity for the compound of the invention. These isolated cells may be further analyzed to verify that the enhanced expression of the reporter protein gene correlates with the improved binding characteristics of the peptide-binding protein. This evaluation may include additional biochemical assays, binding studies, or functional tests to ensure that the peptide-binding protein exhibits the desired specificity and affinity for the target compound.

Preferably, identifying an engineered peptide-binding protein as having increased affinity and/or selectivity for the compound according to the invention involves a step of determining the nucleic acid sequence of the gene encoding the engineered peptide-binding protein, and/or identifying specific mutations in the gene encoding the engineered peptide-binding protein.

That is, cells expressing high levels of the reporter protein gene may be isolated and the gene encoding the engineered peptide-binding protein may be sequenced to identify the nucleic acid sequence encoding an engineered peptide-binding protein having increased affinity and/or selectivity for the compound according to the invention. Sanger sequencing, as known in the art, may be used to identify the nucleic acid sequence encoding an engineered peptide-binding protein.

Alternatively, a plurality of cells that have been identified to express engineered peptide-binding proteins with increased affinity and/or selectivity for the compound of the invention may be subjected to deep sequencing. This approach allows for the comprehensive analysis of the genetic diversity within the population, enabling the identification of specific positions and/or mutations in the peptide-binding protein that are critical for binding the compound. By examining the frequency and enrichment of particular mutations, the genetic changes that contribute to the enhanced binding characteristics can be pinpointed. The skilled person is aware of methods to perform deep sequencing and subsequent bioinformatic analyses to identify such positions and/or mutations. This detailed genetic information provides valuable insights into the molecular basis of the improved binding properties, facilitating the rational design and further optimization of peptide-binding proteins for the compound of the invention.

Thus, in a particular embodiment, the invention relates to the method according to the invention, wherein identifying the engineered peptide-binding protein as having increased specificity for the compound comprises a step of determining the sequence of the nucleic acid encoding the peptide-binding protein and/or determining specific positions and/or mutations in the nucleic acid encoding the peptide-binding protein.

The method described herein above was successfully applied by the inventors to identify engineered variants of the peptide-binding protein OppA from E. coli having increased affinity and selectivity for the compound according to the invention. Thus, another aspect of the invention relates to an engineered variant of an oligopeptide-binding protein OppA from E. coli (SEQ ID NO:1) comprising mutations in one or more of the following positions: L78, T173, V193, D221, W222, 1303, K307, K333, N337, K371, R439, T429, 5460, and/or V482.

That is, in certain embodiments, the engineered variant of an oligopeptide-binding protein OppA from E. coli (SEQ ID NO:1) comprises a mutation at position L78. An improved variant of OppA was identified using the method of the invention comprising the mutation L78I. Thus, in certain embodiments, the engineered variant of an oligopeptide-binding protein OppA from E. coli (SEQ ID NO:1) comprises a mutation at position L78, preferably wherein the leucine at position 78 is replaced by an isoleucine or another hydrophobic amino acid residue such as methionine, alanine or valine.

In certain embodiments, the engineered variant of an oligopeptide-binding protein OppA from E. coli (SEQ ID NO:1) comprises a mutation at position T173. An improved variant of OppA was identified using the method of the invention comprising the mutation T173N. Thus, in certain embodiments, the engineered variant of an oligopeptide-binding protein OppA from E. coli (SEQ ID NO:1) comprises a mutation at position T173, preferably wherein the threonine at position 173 is replaced by an asparagine or another polar amino acid residue such as serine or glutamine.

In certain embodiments, the engineered variant of an oligopeptide-binding protein OppA from E. coli (SEQ ID NO:1) comprises a mutation at position V193. An improved variant of OppA was identified using the method of the invention comprising the mutation V193A. Thus, in certain embodiments, the engineered variant of an oligopeptide-binding protein OppA from E. coli (SEQ ID NO:1) comprises a mutation at position V193, preferably wherein the valine at position 193 is replaced by an alanine or another hydrophobic amino acid residue such as methionine, leucine, or isoleucine.

In certain embodiments, the engineered variant of an oligopeptide-binding protein OppA from E. coli (SEQ ID NO:1) comprises a mutation at position D221. Improved variants of OppA were identified using the method of the invention comprising the mutation D221G, D221L or D221M. Thus, in certain embodiments, the engineered variant of an oligopeptide-binding protein OppA from E. coli (SEQ ID NO:1) comprises a mutation at position D221, preferably wherein the aspartate at position 221 is replaced by a glycine. In certain embodiments, the engineered variant of an oligopeptide-binding protein OppA from E. coli (SEQ ID NO:1) comprises a mutation at position D221, preferably wherein the aspartate at position 221 is replaced by a leucine or methionine or another hydrophobic amino acid residue such as alanine, valine or isoleucine.

In certain embodiments, the engineered variant of an oligopeptide-binding protein OppA from E. coli (SEQ ID NO:1) comprises a mutation at position W222. Improved variants of OppA were identified using the method of the invention comprising the mutations W222R and W222A. Thus, in certain embodiments, the engineered variant of an oligopeptide-binding protein OppA from E. coli (SEQ ID NO:1) comprises a mutation at position W222, preferably wherein the tryptophan at position 222 is replaced by an arginine or another positively charged amino acid residue such as histidine or lysine. In certain embodiments, the engineered variant of an oligopeptide-binding protein OppA from E. coli (SEQ ID NO:1) comprises a mutation at position W222, preferably wherein the tryptophan at position 222 is replaced by an alanine or another hydrophobic amino acid residue such as valine, methionine, leucine or isoleucine.

In certain embodiments, the engineered variant of an oligopeptide-binding protein OppA from E. coli (SEQ ID NO:1) comprises a mutation at position I303. An improved variant of OppA was identified using the method of the invention comprising the mutation I303V. Thus, in certain embodiments, the engineered variant of an oligopeptide-binding protein OppA from E. coli (SEQ ID NO:1) comprises a mutation at position I303, preferably wherein the isoleucine at position 303 is replaced by a valine or another hydrophobic amino acid residue such as methionine, leucine, or alanine.

In certain embodiments, the engineered variant of an oligopeptide-binding protein OppA from E. coli (SEQ ID NO:1) comprises a mutation at position K307. An improved variant of OppA was identified using the method of the invention comprising the mutation K307R. Thus, in certain embodiments, the engineered variant of an oligopeptide-binding protein OppA from E. coli (SEQ ID NO:1) comprises a mutation at position K307, preferably wherein the lysine at position 307 is replaced by an arginine or another positively charged amino acid residue such as histidine.

In certain embodiments, the engineered variant of an oligopeptide-binding protein OppA from E. coli (SEQ ID NO:1) comprises a mutation at position K333. An improved variant of OppA was identified using the method of the invention comprising the mutation K333N. Thus, in certain embodiments, the engineered variant of an oligopeptide-binding protein OppA from E. coli (SEQ ID NO:1) comprises a mutation at position K333, preferably wherein the lysine at position 333 is replaced by an asparagine or another polar amino acid residue such as serine, threonine or glutamine.

In certain embodiments, the engineered variant of an oligopeptide-binding protein OppA from E. coli (SEQ ID NO:1) comprises a mutation at position N337. An improved variant of OppA was identified using the method of the invention comprising the mutation N337D. Thus, in certain embodiments, the engineered variant of an oligopeptide-binding protein OppA from E. coli (SEQ ID NO:1) comprises a mutation at position N337, preferably wherein the asparagine at position 337 is replaced by an aspartate or another negatively charged amino acid residue such as glutamate.

In certain embodiments, the engineered variant of an oligopeptide-binding protein OppA from E. coli (SEQ ID NO:1) comprises a mutation at position K371. An improved variant of OppA was identified using the method of the invention comprising the mutation K371E. Thus, in certain embodiments, the engineered variant of an oligopeptide-binding protein OppA from E. coli (SEQ ID NO:1) comprises a mutation at position K371, preferably wherein the lysine at position 371 is replaced by a glutamate or another negatively charged amino acid residue such as aspartate.

In certain embodiments, the engineered variant of an oligopeptide-binding protein OppA from E. coli (SEQ ID NO:1) comprises a mutation at position R439. Improved variants of OppA were identified using the method of the invention comprising the mutations R439H, R439Q, R439L or R439A. Thus, in certain embodiments, the engineered variant of an oligopeptide-binding protein OppA from E. coli (SEQ ID NO:1) comprises a mutation at position R439, preferably wherein the arginine at position 439 is replaced by a histidine or another positively charged amino acid residue such as lysine. In certain embodiments, the engineered variant of an oligopeptide-binding protein OppA from E. coli (SEQ ID NO:1) comprises a mutation at position R439, preferably wherein the arginine at position 439 is replaced by a glutamine or another polar amino acid residue such as serine, threonine or asparagine. In certain embodiments, the engineered variant of an oligopeptide-binding protein OppA from E. coli (SEQ ID NO:1) comprises a mutation at position R439, preferably wherein the arginine at position 439 is replaced by a leucine or alanine or another hydrophobic amino acid residue such as methionine, isoleucine or valine.

In certain embodiments, the engineered variant of an oligopeptide-binding protein OppA from E. coli (SEQ ID NO:1) comprises a mutation at position T429. An improved variant of OppA was identified using the method of the invention comprising the mutation T429A. Thus, in certain embodiments, the engineered variant of an oligopeptide-binding protein OppA from E. coli (SEQ ID NO:1) comprises a mutation at position T429, preferably wherein the threonine at position 429 is replaced by an alanine or another hydrophobic amino acid residue such as methionine, leucine, isoleucine or valine.

In certain embodiments, the engineered variant of an oligopeptide-binding protein OppA from E. coli (SEQ ID NO:1) comprises a mutation at position S460. Improved variants of OppA were identified using the method of the invention comprising the mutations S460N, S460H or S460C. Thus, in certain embodiments, the engineered variant of an oligopeptide-binding protein OppA from E. coli (SEQ ID NO:1) comprises a mutation at position S460, preferably wherein the serine at position 460 is replaced by an asparagine or another polar amino acid residue such as threonine or glutamine. In certain embodiments, the engineered variant of an oligopeptide-binding protein OppA from E. coli (SEQ ID NO:1) comprises a mutation at position S460, preferably wherein the serine at position 460 is replaced by a histidine or another positively charged amino acid residue such as lysine or arginine. In certain embodiments, the engineered variant of an oligopeptide-binding protein OppA from E. coli (SEQ ID NO:1) comprises a mutation at position S460, preferably wherein the serine at position 460 is replaced by a cysteine or another polar amino acid residue such as serine, threonine, asparagine or glutamine.

In certain embodiments, the engineered variant of an oligopeptide-binding protein OppA from E. coli (SEQ ID NO:1) comprises a mutation at position V482. An improved variant of OppA was identified using the method of the invention comprising the mutation V482A. Thus, in certain embodiments, the engineered variant of an oligopeptide-binding protein OppA from E. coli (SEQ ID NO:1) comprises a mutation at position V482, preferably wherein the valine at position 482 is replaced by an alanine or another hydrophobic amino acid residue such as methionine, leucine, or isoleucine.

In certain embodiments, the engineered OppA variant according to the invention comprises at least one mutation in a position selected from the group consisting of: T173, V193, D221, W222, 1303, N337, R439, and S460. Thus, in a particular embodiment, the invention relates to the engineered variant of the invention, wherein the one or more mutations are located in positions: T173, V193, D221, W222, 1303, N337, R439, and/or 5460.

In certain embodiments, the engineered OppA variant according to the invention comprises at least one mutation in a position selected from the group consisting of: D221, W222, N337, R439, and S460. Thus, in a particular embodiment, the invention relates to the engineered variant of the invention, wherein the one or more mutations are located in positions: D221, W222, N337, R439, and/or 5460.

In certain embodiments, the engineered OppA variant according to the invention comprises at least one mutation in a position selected from the group consisting of: D221, W222, R439, and S460. Thus, in a particular embodiment, the invention relates to the engineered variant of the invention, wherein the one or more mutations are located in positions: D221, W222, R439, and/or 5460.

In certain embodiments, the engineered OppA variant according to the invention comprises at least one mutation in a position selected from the group consisting of: R439, and S460. Thus, in a particular embodiment, the invention relates to the engineered variant of the invention, wherein the one or more mutations are located in positions: R439, and/or 5460.

The mutations are preferably any of the mutations listed herein above. That is, in certain embodiments, the invention relates to an engineered OppA variants comprising one or more of mutations: L78I, T173N, V193A, D221G, D221L, D221M, W222R, W222A, I303V, K307R, K333N, N337D, K371E, R439H, R439Q, R439L, R439A, T429A, S460N, S460H, S460C, and/or V482A.

In certain embodiments, the invention relates to an engineered OppA variants comprising one or more of mutations: T173N, V193A, D221G, D221L, D221M, W222R, W222A, I303V, N337D, R439H, R439Q, R439L, R439A, S460N, S460H, and/or S460C.

In certain embodiments, the invention relates to an engineered OppA variants comprising one or more of mutations: D221G, D221L, D221M, W222R, W222A, N337D, R439H, R439Q, R439L, R439A, S460N, S460H, and/or S460C.

In certain embodiments, the invention relates to an engineered OppA variants comprising one or more of mutations: D221G, D221L, D221M, W222R, W222A, R439H, R439Q, R439L, R439A, S460N, S460H, and/or S460C.

In certain embodiments, the invention relates to an engineered OppA variants comprising one or more of mutations: R439H, R439Q, R439L, R439A, S460N, S460H, and/or S460C.

In certain embodiments, the invention relates to an engineered OppA variants comprising one or more of mutations: R439Q and/or S460C.

Further encompassed are variants of the E. coli OppA protein having at least 70%, at least 75%, at least 80%, at least 85%, at least 90%, or at least 95 sequence identity to SEQ ID NO:1, provided that they comprise one or more of the mutations disclosed herein above and show increased affinity and/or selectivity for the compound according to the invention compared to wild type OppA (SEQ ID NO:1).

The present invention further relates to a nucleic acid encoding the engineered OppA variants disclosed herein. Thus, in a particular embodiment, the invention relates to a nucleic acid encoding the engineered variant according to the invention.

The skilled person will recognize that these nucleic acids can be synthesized using standard molecular biology techniques and can be introduced into suitable host cells for expression. These nucleic acids may include regulatory elements such as promoters, enhancers, and terminators to ensure efficient transcription and translation of the engineered OppA variants.

Additionally, the nucleic acids can be incorporated into various vectors, including plasmids, for ease of manipulation and transfer into host cells. Thus, in a particular embodiment, the invention relates to a vector comprising the nucleic acid according to the invention.

By encoding the specific mutations identified in the engineered OppA variants, these nucleic acids enable the production of peptide-binding proteins with enhanced affinity and/or selectivity for the compound of the invention.

The nucleic acid and/or vector encoding the engineered OppA variants may be introduced into a host cell. Thus, in a particular embodiment, the invention relates to a cell comprising the nucleic acid according to the invention or the vector according to the invention.

That is, the cell may comprise a vector or plasmid encoding the engineered OppA variant. The skilled person is aware of methods to introduce a vector or plasmid into a cell and maintain it inside the cell, such as transformation, transfection and electroporation. These techniques ensure the stable presence and expression of the engineered OppA variant within the host cell.

Alternatively, the nucleic acid encoding the engineered OppA variant may be integrated into the genome of the host cell. This can be achieved using methods such as the Datsenko-Wanner method, which allows for precise genomic integration through homologous recombination (Proc Natl Acad Sci USA. 2000 Jun 6;97(12):6640-5) or viral transduction. By integrating the nucleic acid into the host genome, stable and long-term expression of the engineered OppA variant can be achieved, providing a reliable system for studying and utilizing the enhanced peptide-binding properties of the protein.

The host cell may be any type of cell. It is, however, preferred herein that the host cell is a prokaryotic cell. The term "prokaryotic cell" as used herein refers to a type of cell that lacks a membrane-bound nucleus and other membrane-bound organelles. Prokaryotic cells are characterized by their relatively simple structure, with genetic material organized in a single, circular chromosome located in a region called the nucleoid. Additionally, prokaryotic cells may contain plasmids, which are small, circular DNA molecules that replicate independently of the chromosomal DNA. Prokaryotic cells include organisms from the domains Bacteria and Archaea. Preferably, the cell according to the invention is a bacterial cell. Even more preferably, the cell according to the invention is an E. coli cell. Thus, in a particular embodiment, the invention relates to the cell according to the invention, wherein the cell is a prokaryotic cell, in particular an E. coli cell.

Another aspect of the invention relates to a method for incorporating a non-canonical amino acid into a protein, wherein the non-canonical amino acid is derived from the compound according to the invention.

Thus, in a particular embodiment, the invention relates to a method for incorporating a non-canonical amino acid, or an analog thereof, into a protein, the method comprising the steps of:
a) providing a cell comprising at least one orthogonal translation system, wherein the at least one orthogonal translation system comprises an orthogonal aminoacyl-tRNA synthetase/tRNA pair specific for a non-canonical amino acid comprised in the compound according to the invention, and a nucleic acid molecule encoding the protein, wherein the nucleic acid molecule encoding the protein comprises one or more codons that have been re-allocated for the incorporation of the non-canonical amino acid by the orthogonal aminoacyl-tRNA synthetase/tRNA pair;
b) contacting the cell of step (a) with the compound according to the invention, wherein the compound is transported into the cell and hydrolyzed inside the cell to release an isopeptide-linked lysine or an analog thereof and, optionally, another non-canonical amino acid or an analog thereof;
c) incorporating (i) the isopeptide-linked lysine or an analog thereof and/or (ii) the other non-canonical amino acid or an analog thereof into the protein in response to the one or more re-allocated codon using the orthogonal translation system.

It has been convincingly demonstrated herein that the compound according to the invention, which comprises an isopeptide-linked lysine or an analog thereof, can be efficiently taken up by a cell through endogenous peptide transporters or engineered variants thereof. Notably, the import of the compound according to the invention has been shown to be more efficient than the uptake of isopeptide-linked lysine, or its analog, alone. This enhanced uptake efficiency underscores the effectiveness of the compound in leveraging the cellular transport mechanisms, thereby facilitating its intracellular delivery and subsequent utilization.

The method for incorporating a non-canonical amino acid into a protein starts with a cell comprising at least one orthogonal translation system. The orthogonal translation system may comprise any of the aminoacyl-tRNA synthetase/tRNA pairs that have been disclosed elsewhere herein for the incorporation of the isopeptide-linked lysine or an analog thereof. That is, the orthogonal translation system may be used for incorporating an isopeptide-linked lysine or an analog thereof, obtained by intracellular cleavage of the compound according to the invention, into a protein of interest.

In such embodiments, the cell preferably comprises a pyrrolysyl-tRNA synthetase/tRNA pair for the incorporation of the isopeptide-linked lysine or an analog thereof. Thus, in a particular embodiment, the invention relates to the method according to the invention, wherein the orthogonal aminoacyl-tRNA synthetase/tRNA pair is a Pyrrolysyl-tRNA synthetase/tRNA pair.

When the isopeptide-linked lysine or an analog thereof is incorporated into a protein using an orthogonal translation system, it is to be understood that the isopeptide-linked lysine or analog thereof comprises an alpha amino group and an alpha carboxyl-group.

Alternatively or in addition, the cell may comprise another orthogonal translation system that does not recognize the isopeptide-linked lysine or an analog thereof, but another amino acid comprised in the compound according to the invention. That is, the compound according to the invention may not just be used to transport isopeptide-linked lysines or analogs thereof into a cell, but may also be used to transport other amino acids or analogs thereof that would otherwise not be efficiently taken up by the cell. This other amino acid or analog thereof is preferably linked to the isopeptide-linked lysine or analog thereof by a bond that can be hydrolysed inside the cell.

In certain embodiments, the analog of the other amino acid is an alpha-hydroxy acid where the alpha-amino group is replaced with a hydroxy group. Incorporation of such amino acid analogs is described, without limitation, by Owczarek et al. (Biochemistry, 2008; 47(1):301-7).

In certain embodiments, the analog of the other amino acid may be a mercapto acid where the alpha-amino group is replaced with a thiol group.

To stick with the nomenclature of the present invention, the other or second (non-canonical) amino acid would comprise the moiety B comprising the side chain Z (or Z¹ and Z²). More preferably, the other amino acid would have the structure NH₂-B-COOH once it is released from the compound according to the invention by a hydrolyzing enzyme. The other amino acid analog would preferably have the structure HO-B-COOH or HS-B-COOH once it is released from the compound according to the invention by a hydrolyzing enzyme, preferably an esterase.

In embodiments where the compound of the invention is a peptide having the structure Z-XisoK, the other amino acid would be residue Z.

Thus, in a particular embodiment, the invention relates to the method according to the invention, wherein hydrolysis of the molecule according to the invention inside the cell releases a second non-canonical amino acid (ncAA), and wherein the second ncAA is incorporated into the same or a different protein in response to a re-allocated codon.

There is no limitation regarding the other amino acid or its analog. That is, the other amino acid (Z) may, inter alia, be any non-canonical amino acid. The skilled person is capable of selecting an amino acid or analog thereof for which an orthogonal translation system is known in the art.

The cell further comprises a nucleic acid encoding a protein of interest, specifically the protein into which one or more non-canonical amino acids are to be incorporated. This nucleic acid sequence is designed to include codons that have been re-allocated for the incorporation of non-canonical amino acids by the orthogonal translation system(s). The presence of this nucleic acid ensures that the protein of interest is expressed within the cell, allowing for the precise incorporation of the desired non-canonical amino acids at specific positions.

Of note, the nucleic acid encoding the protein of interest may comprise one or more codons that have been re-allocated for the incorporation of non-canonical amino acids by the orthogonal aminoacyl-tRNA synthetase/tRNA pair(s).

That is, in certain embodiments, the nucleic acid may comprise one or more codons that have been re-allocated for the incorporation of an isopeptide-linked lysine or an analog thereof by an orthogonal aminoacyl-tRNA synthetase/tRNA pair.

In certain embodiments, the nucleic acid may comprise one or more codons that have been re-allocated for the incorporation of another amino acid or analog thereof by an orthogonal aminoacyl-tRNA synthetase/tRNA pair, wherein the other amino acid or analog thereof is also comprised in the compound according to the invention.

In certain embodiments, the nucleic acid may comprise one or more codons that have been re-allocated for the incorporation of an isopeptide-linked lysine or an analog thereof by a first orthogonal aminoacyl-tRNA synthetase/tRNA pair and one or more codons that have been re-allocated for the incorporation of another amino acid or analog thereof by a second orthogonal aminoacyl-tRNA synthetase/tRNA pair, wherein the other amino acid or analog thereof is also comprised in the compound according to the invention.

In a particular embodiment, the invention relates to the method according to the invention, wherein the nucleic acid molecule encoding the protein comprises one or more amber stop codons that have been re-allocated to incorporate the non-canonical amino acid.

As described herein above, the amber stop codon is well established for the incorporation of non-canonical amino acids. However, also other codons may be re-allocated for the incorporation of non-canonical amino acids, including the ochre stop codon, quadruplet codons or liberated sense codons, as described in more detail elsewhere herein.

In embodiments where the cell comprises two orthogonal translation systems, the nucleic acid encoding the protein of interest may comprise two re-allocated codons for the incorporation of non-canonical amino acids. This allows for the simultaneous incorporation of different non-canonical amino acids into a single protein, thereby enabling the creation of proteins with novel properties and functionalities.

Alternatively, the cell may comprise two separate nucleic acids encoding different proteins of interest. In this case, the first nucleic acid may comprise one or more codons that are re-allocated for the incorporation of a non-canonical amino acid by a first orthogonal translation system, while the second nucleic acid may comprise one or more codons that are re-allocated for the incorporation of a non-canonical amino acid by a second orthogonal translation system. This configuration allows for the independent expression and incorporation of distinct non-canonical amino acids into different proteins within the same cell, thereby expanding the range of possible protein modifications and applications.

The protein encoded by the nucleic acid may be any protein, including: an antibody or antibody fragment (including nanobodies), a fluorescent protein (such as sfGFP), Ubiquitin, SUMO1, SUMO2, a histone (such as Histone H3), a Proliferating cell nuclear antigen (PCNA), Calmodulin, β-lactamase, Hsp82, a cytokine (such as interleukin-2, IFN-β or IFN- γ), or a hormone (such as Human growth hormone (hGH)).

Antibodies may include full length antibodies such as, without limitation Trastuzumab. An antibody fragment is a portion of an antibody that retains the ability to bind to an antigen, and is preferably a single-chain fragment. Single-chain fragments, such as single-chain variable fragments (scFvs) and nanobodies, are engineered to consist of the variable regions of the heavy (VH) and light (VL) chains connected by a short flexible linker, allowing them to retain the antigen-binding specificity of the original antibody while being smaller and more stable. Examples of single-chain fragments include scFvs, which are fusion proteins of the VH and VL regions, and nanobodies, also known as VHHs or single-domain antibodies, which are derived from the unique heavy-chain-only antibodies found in camelids (such as camels, llamas, and alpacas). Nanobodies are particularly noted for their small size, high stability, and strong binding affinity, making them valuable in therapeutic treatments, diagnostic assays, and research applications. Antibody fragments may bind to any target including, without limitation, eGFP or HER2. In certain embodiments, the antibody fragment is a Fab fragment. The Fab fragment may be further PEGylated.

The cell may be any type of cell, as explained for the other methods disclosed herein. However, it is preferred herein that the cell is a prokaryotic cell, more preferably a bacterial cell, most preferably an E. coli cell. Thus, in a particular embodiment, the invention relates to the method according to the invention, wherein the cell is a prokaryotic cell, in particular an E. coli cell.

It has been demonstrated herein that endogenous peptide transporters can import the compound according to the invention. Thus, the method of the invention is not restricted to cells comprising an engineered peptide-binding protein, such as any one of the engineered peptide-binding proteins disclosed herein.

The most promising endogenous peptide transporters for the uptake of the compound according to the invention are the dipepitde permease (Dpp) and, in particular, the oligopeptide permease (Opp). Thus, in a particular embodiment, the invention relates to the method according to the invention, wherein the cell expresses a dipeptide permease and/or an oligopeptide permease

However, the inventors have observed that the compound according to the invention competes with linear peptides for endogenous peptide transporters. Therefore, it may be desirable to utilize peptide transporters that have been engineered for enhanced affinity and/or selectivity for the compound according to the invention.

Accordingly, in a particular embodiment, the invention relates to the method according to the invention, wherein the dipeptide permease and/or the oligopeptide permease comprises a peptide-binding protein that has been engineered for increased affinity and/or selectivity for the compound according to invention.

Methods for obtaining engineered peptide-binding proteins with increased affinity and/or selectivity for the compound of the invention are disclosed herein.

In particular, the cell may comprise any one of the engineered OppA variants disclosed herein. That is, in a particular embodiment, the invention relates to a method according to the invention, wherein the engineered peptide-binding protein is OppA of E. coli (SEQ ID NO:1) comprising mutations in one or more of the following positions: L78, T173, V193, D221, W222, I303, K307, K333, N337, K371, R439, T429, S460, and/or V482.

To avoid lengthy repetition, it is to be understood that all combinations of positions and/or specific mutations in OppA disclosed elsewhere herein can be used for the method of incorporating non-canonical amino acids into a protein.

In a particularly preferred embodiment, the cell is a cell expressing an oligopeptide permease comprising a wild-type or engineered peptide-binding protein OppA.

In an even more preferred embodiment, the cell is a cell expressing an oligopeptide permease comprising a wild-type or engineered peptide-binding protein OppA from E. coli.

Of note, the cell may comprise a heterologous OppA variant, meaning an OppA variant derived from a different organism. For example, a wild-type or engineered *E. coli* OppA variant may be heterologously expressed in another organism to enhance the uptake of the compound of the invention. In other embodiments, the entire oligopeptide permease system of *E. coli,* including a wild-type or engineered *E. coli* OppA variant, may be heterologously expressed in another organism to further improve the uptake of the compound of the invention.

Alternatively, a OppA or OppA variant from another organism that has been engineered using the method disclosed herein herein may be heterologously expressed in E. coli cells to facilitate the uptake of the compound according to the invention.

The cell is then contacted with the compound according to the invention as described elsewhere herein. Contacting the cell with the compound according to the invention will result in uptake of the compound according to the invention into the cell and the subsequent hydrolysis of the compound according to the invention to release an isopeptide-linked lysine or an analog thereof and, optionally, another non-canonical amino acid.

Once released inside the cell, the isopeptide-linked lysine or analog thereof and/or the other non-canonical amino acid may be incorporated into one or more proteins of interest using orthogonal translation systems.

Successful incorporation of non-canonical amino acids into proteins may be confirmed by methods known in the art, including mass spectrometry.

### BRIEF DESCRIPTION OF THE DRAWINGS

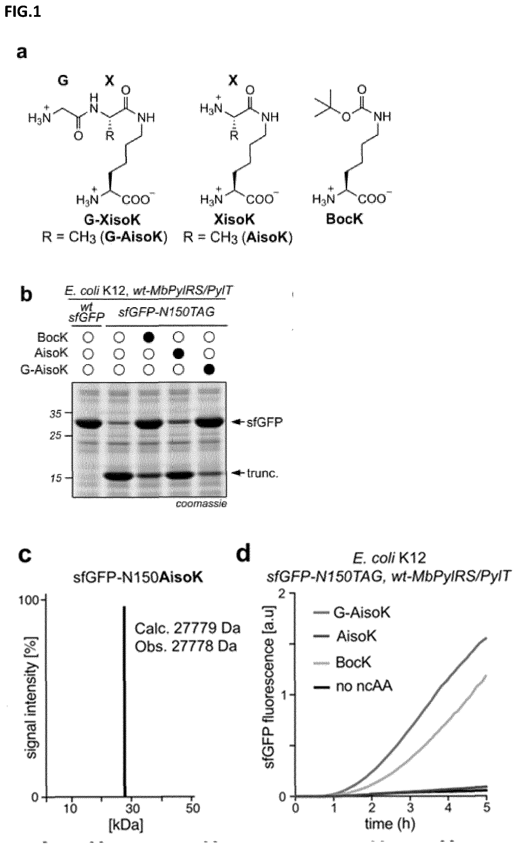

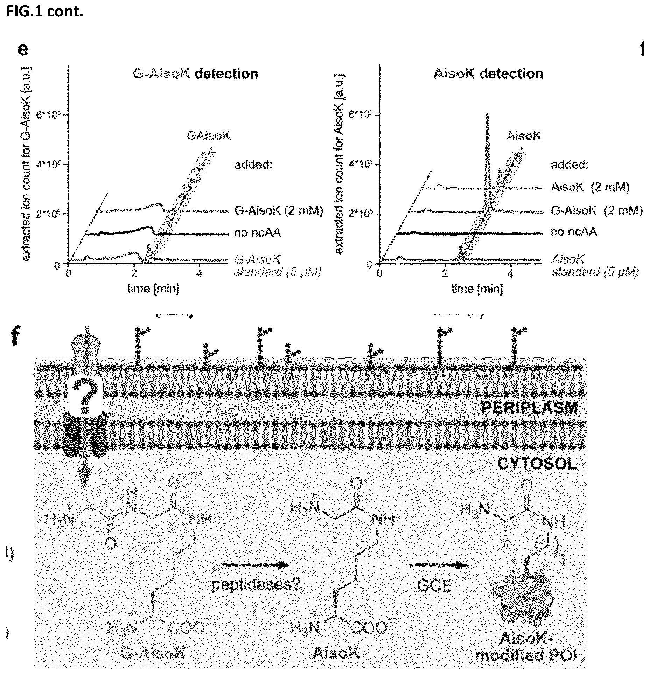

**Figure 1**. Isopeptide-linked tripeptides are privileged scaffolds for efficient *E. coli* uptake **a.** Chemical structures for G-XisoK, XisoK and BocK. X is alanine for G-AisoK and AisoK. **b.** SDS-PAGE analysis of sfGFP-N150TAG expression using wt-*Mb*PyIRS/PyIT in the presence of 2 mM BocK, AisoK or G-AisoK. (trunc. denotes truncated protein). **c.** LC-MS analysis of sfGFP-N150AisoK. **d.** Time course measurements of sfGFP fluorescence from cultures expressing sfGFP-N150TAG and wt-MbPyIRS/PyIT in the presence of G-AisoK, AisoK or BocK, or grown without ncAAs. **e.** Extracted ion chromatograms for determining intracellular concentrations of G-AisoK (teal) and AisoK (pink) by an LC-MS assay, performed on K12 cell extracts. Intracellular G-AisoK concentrations in K12 cells grown in the presence of 2 mM G-AisoK are negligible (dark grey, left). Intracellular AisoK concentrations in K12 cells grown in the presence of 2 mM G-AisoK (dark grey, right) are 5-10-fold higher than when grown in the presence of 2 mM AisoK (light grey, right). **f.** Proposed model for increased AisoK incorporation in the presence of G-AisoK. The tripeptide G-AisoK is actively taken up by K12 cells via a transporter. Within the cytoplasm G-AisoK is processed to AisoK, which is a substrate forwt-*Mb*PyIRS/PyIT and is incorporated site-specifically into a protein of interest (POI).

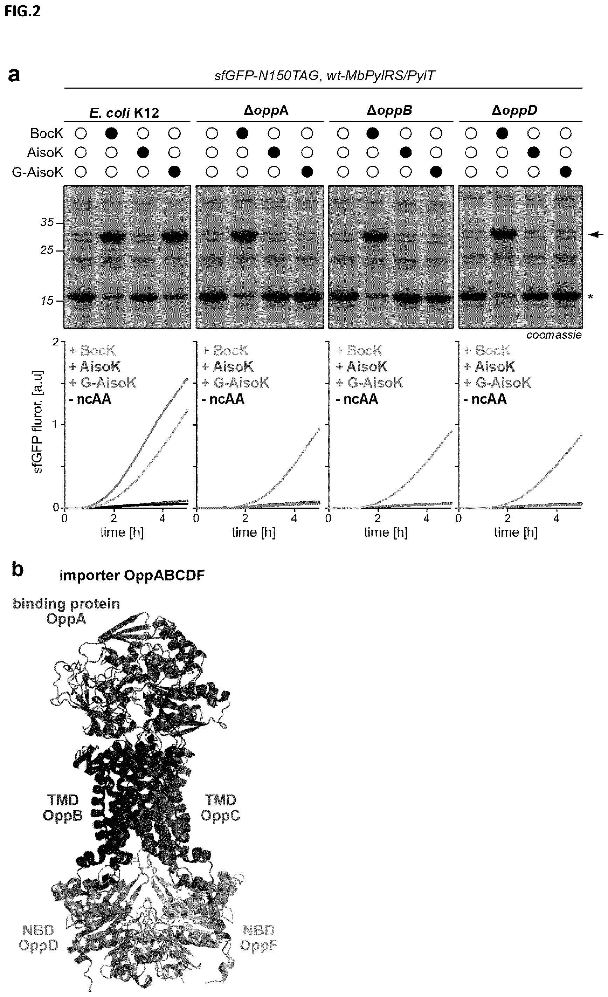

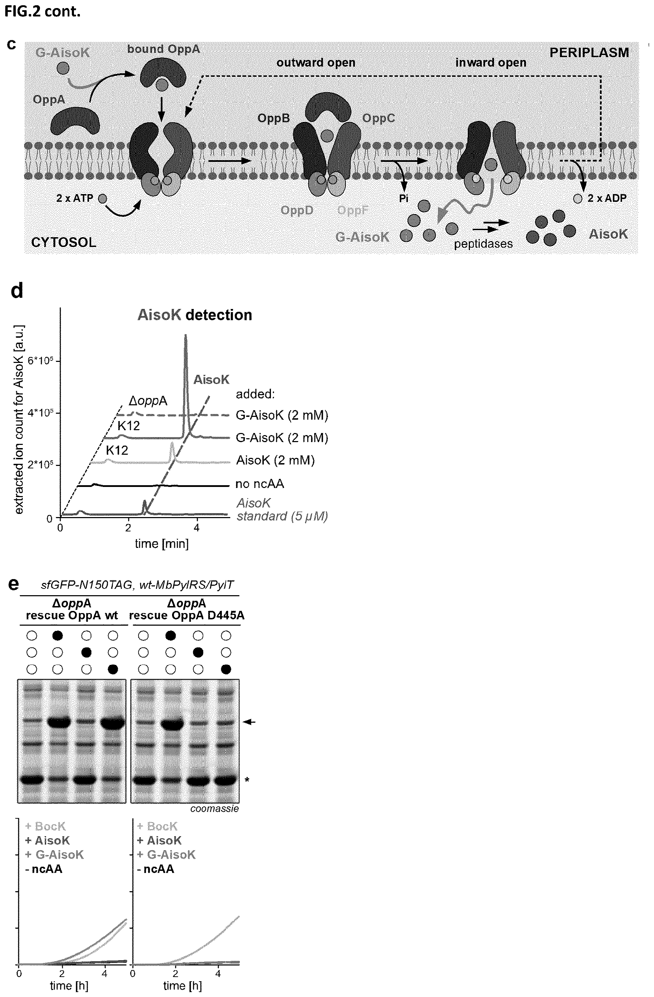

**Figure 2****.** The Opp transporter is responsible for efficient G-AisoK uptake. **a.** SDS-PAGE analysis and time course fluorescence measurements of sfGFP-N150TAG expression in the presence of BocK, AisoK or G-AisoK in K12 cells and in DoppA, DoppB or DoppD knockouts indicating that the Opp transporter is responsible for G-AisoK uptake. Results for other knockouts can be found in Fig. S3a. **b.** AlphaFold2(*40*) predicted structure of the Opp transporter consisting of the periplasmic peptide binding protein OppA, two transmembrane domains (TMDs) OppB,C and two nucleotide binding domains (NBDs) OppD,F. **c.** Proposed mechanism of Opp-mediated uptake. G-AisoK binds to OppA in the periplasm and is shuttled to membrane-bound OppB,C. The tripeptide is actively transported into the cytosol in an ATP dependent manner where it is cleaved by endogenous peptidases to AisoK. OppA in its apo-form is released from the TMDs to allow for binding of new G-AisoK. **d.** Extracted ion chromatograms of *E. coli* K12 lysates for determining intracellular AisoK concentrations in K12 versus D*opp*A K12 cells. Genomic deletion of *oppA* results in undetectable AisoK concentrations when growing cells in the presence of 2 mM G-AisoK (grey, dashed). **e.** SDS-PAGE analysis and time course fluorescence measurements of sfGFP-N150TAG expression in Δ*opp*A cells, constitutively expressing OppA variants (left: wt, right: D445A). Plasmid-based constitutive expression of wt-OppA rescues full-length sfGFP expression in presence of G-AisoK, while this is not the case for OppA-D445 expression, indicating that D445 is essential for G-AisoK binding and uptake. BocK dependent sfGFP expression remains unchanged in both cases. Arrow indicates full-length sfGFP, asterisk indicates truncated protein).

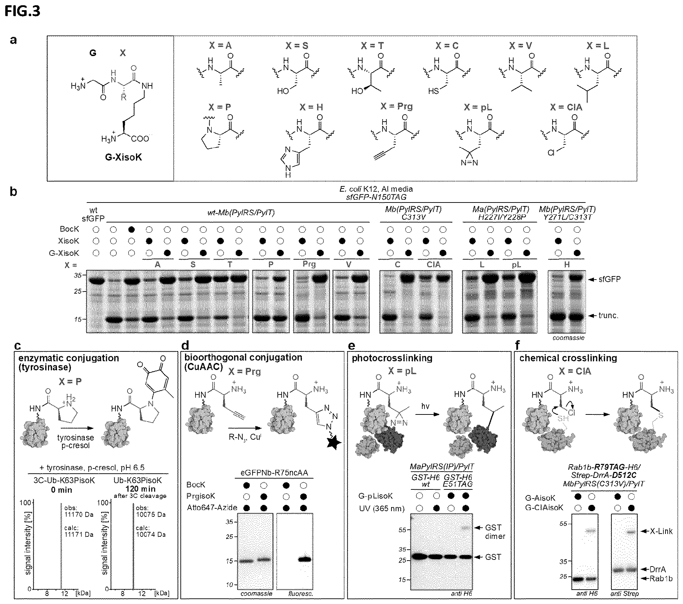

**Figure 3**. A versatile G-XisoK toolbox. **a.** Structures of all functionalities incorporated into the G-XisoK scaffold. **b.** SDS-PAGE analysis of sfGFP-N150TAG expression in the presence of either 2 mM XisoK or G-XisoK. All G-XisoK derivatives show high levels of full-length sfGFP expression using the corresponding PyIRS/PyIT pairs. Cells grown in the presence of XisoK derivatives, on the contrary, show much lower or no expression of full-length sfGFP. LC-MS analyses of purified sfGFP and Ub variants, confirming incorporation of XisoK derivatives are shown in Figs. S5, S6 and S9. c. LC-MS analysis of tyrosinase-mediated labelling of 3C-Ub bearing PisoK at K63 with p-cresol shows quantitative conversion. Conditions: 20 µM 3C-UbK63PisoK, 100 µM p-cresol, 0.4 µM tyrosinase at pH 6.5, 120 min (for details and full data see Fig. S8) **d.** SDS-PAGE analysis of CuAAC labeling of purified eGFPNb-R75PrgisoK with an Atto647-Azide fluorophore. No labeling is observed for eGFPNb-R75BocK, as expected (for details and full data see Fig. S10a). **e.** Western blot analysis of GST-dimer crosslinking for GST-E51pLisoK upon UV_{365 nm} illumination. No crosslink is observed for GST-wt, as expected (for details and full data see Fig. S10b). **f.** Western blot analysis of proximity-induced chemical crosslinking between Rab1b-R79CIAisoK and its interactor DrrA-D512C₃₃₉₋₅₂₂ (55). Cells expressing both binding partners in the presence of G-CIAisoK show a higher molecular weight band corresponding to the crosslinked complex in both anti-H6 and anti-Strep blots. No crosslinking was observed for cells grown in the presence of G-AisoK. Full data and further experiments can be found in Fig. S11.

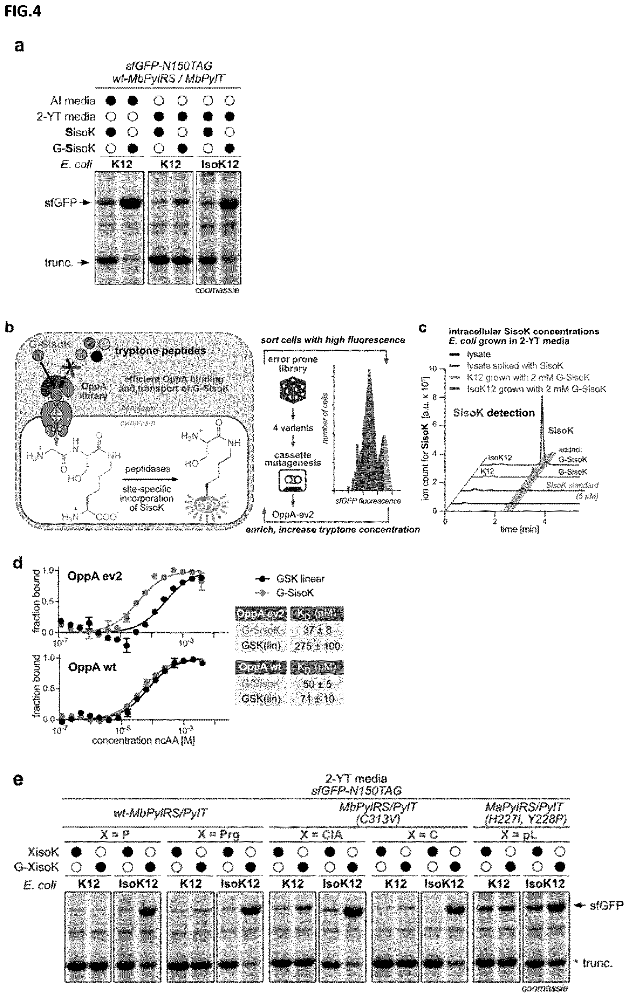

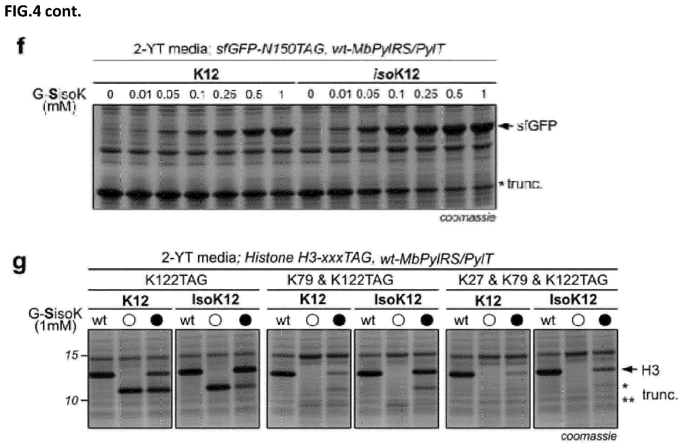

**Figure 4****.** Scalable XisoK incorporation through OppA evolution. **a.** SDS-PAGE analysis of sfGFP-N150TAG expression in the presence of SisoK or G-SisoK in AI media (left) or 2-YT (middle, right). In K12 cells full-length sfGFP expression yields are significantly reduced when using tryptone containing 2-YT (middle) media due to competition between tryptic peptides and G-SisoK for OppA binding. sfGFP expression in 2-YT is recovered when using the engineered lsoK12 strain (right). **b.** Scheme for OppA evolution for better G-SisoK uptake in tryptone containing media. OppA libraries were screened for increased G-SisoK uptake under increasing tryptone concentrations via amber suppression of sfGFP-N150TAG by sorting for fluorescent cells. Initial screening of an error-prone library yielded 4 variants which were used as basis for creating a cassette mutagenesis library, whose screening yielded OppA-ev2. **c.** Extracted ion chromatograms to determine intracellular SisoK concentrations. IsoK12 cells (purple) grown in 2-YT show 7-10-fold higher intracellular SisoK concentrations in comparison to K12 cells (grey) when adding 2 mM G-SisoK to 2-YT media. **d.** Affinity measurements of G-SisoK and a linear GSK peptide (GSK(lin)) towards OppA using microscale thermophoresis. **e.** SDS-PAGE analysis of sfGFP-N150TAG expression in wt-K12 cells versus IsoK12 cells grown in 2-YT media in presence of G-XisoK derivatives with X = P, Prg, CIA, C, pL. Full-length sfGFP expression is severely increased in IsoK12 cells for all X functionalities. Full gels as well as expression gels for other G-XisoK derivatives can be found in Fig. S12. **f.** SDS-PAGE analysis of sfGFP-N150TAG expression in 2xYT, comparing K12 with lsoK12 cells at different G-SisoK concentrations. **g.** SDS-PAGE analysis of Histone H3 with single (K122TAG), double (K79TAG, K122TAG) and triple (K27TAG, K79TAG, K122TAG) amber suppression in presence of 1 mM G-SisoK comparing K12 with lsoK12 cells grown in 2-YT media.

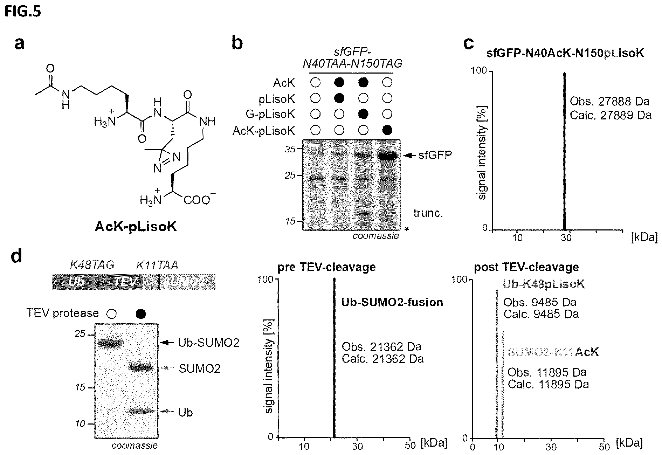

**Figure 5**. Dual stop codon suppression using a single isopeptide-linked tripeptide. **a.** Chemical structure of the tripeptide AcK-pLisoK. **b.** SDS-PAGE analysis of sfGFP-N40TAA-N150TAG expression in the presence of either AcK and G-pLisoK (or pLisoK) separately added to media or in the presence of tripeptide AcK-pLisoK in lsoK12 cells grown in AI media. **c.** LC-MS analysis of purified sfGFP confirms dual ncAA incorporation (AcK and pLisoK) upon addition of tripeptide AcK-pLisoK. **d.** Left: Scheme of Ub-SUMO fusion construct used to confirm dual and orthogonal incorporation of pLisoK and AcK. SDS-PAGE analysis shows purified full-length construct and products of TEV protease cleavage. Right: LC-MS analysis of full-length construct and cleaved products confirming pLisoK and AcK incorporation at the intended positions.

### EXAMPLES

### Example 1: Isopeptide-linked tripeptides are privileged scaffolds for efficient E. coli uptake

Previous work in our group has pioneered the use of transpeptidases in combination with genetic code expansion to generate defined protein-protein conjugates. Through site-specific incorporation of an ncAA bearing an azide-caged dipeptidic nucleophile acceptor (AzGGisoK) and subsequent Staudinger reduction, GGisoK-modified proteins can engage in transpeptidation with donor proteins modified at their C-terminus with an appropriate recognition sequence. We have leveraged these approaches for successfully generating ubiquitin (Ub)- and Ub-like modifier (Ubl)-POI conjugates using sortase or *Oα*AEP1 as transpeptidases (*30-32*). In our quest to diversify the linker sequence in the generated protein conjugates, we explored the site-specific incorporation of ncAAs resembling a general G-XisoK scaffold (Fig. 1a). While AzGGisoK required intensive PylRS engineering, we were not able to identify a PyIRS-variant for direct GGisoK incorporation(30). In contrast, addition of the alanine-bearing G-XisoK tripeptide (G-AisoK, Fig. 1a) to growing *E. coli* cells (K12 strain), transformed with plasmids coding for the wild-type *Methanosarcina barkeri* Pyrrolysyl-tRNA synthetase/tRNA pair (wt-*Mb*PyIRS/PyIT) and super-folder green fluorescence protein bearing an amber codon at position 150 (sfGFP-N150TAG), led to highly efficient sfGFP expression, comparable to wt-sfGFP production and amber suppression yields using the gold-standard ncAA BocK (Fig. 1a,b). Interestingly, MS-analysis of purified sfGFP expressed in presence of G-AisoK revealed site-specific incorporation of AisoK (Fig 1c). This suggests that the N-terminal glycine is cleaved off intracellularly, either on the free ncAA, or co/post-translationally. On the other hand, supplementing K12 with AisoK instead of G-AisoK showed almost no visible amber suppression and sfGFP production (Fig. 1b). This is corroborated by live-cell sfGFP-fluorescence measurements in the presence of different ncAAs. Cells grown in the presence of AisoK showed minimal fluorescence, while fluorescence is observed at earlier timepoints and reaches higher values with G-AisoK when compared to BocK (Fig. 1d). Similarly, G-AisoK-mediated AisoK incorporation was observed for other amber codon-bearing target proteins. Intrigued by this observation, we set out to determine intracellular levels of the corresponding ncAAs, using LC-MS based uptake assays (*18, 33*)*.* Upon G-AisoK addition, we could not observe any intracellular G-AisoK, but AisoK accumulated at 5-10-fold higher concentrations compared to supplementing K12 cells with AisoK itself (Fig 1e).

This observation led us to formulate the hypothesis that a specific transport mechanism may actively pump G-AisoK into cells. Within the cytosol G-AisoK is then enzymatically processed to AisoK, which accumulates in high concentrations and serves as a substrate for wt-PyIRS, leading to efficient AisoK encoding (Fig. 1f).

### Example 2: A bacterial ABC-transporter facilitates tripeptide uptake

In gram-negative organisms such as *E. coli,* small peptidic substrates gain access to the periplasm via diffusion through outer membrane pore-forming proteins known as porins (*34*). Depending on the mode of action there are two major classes of active peptide transporters located in the inner membrane that shuffle specific peptides from the periplasm to the cytosol: (1) proton-dependent oligopeptide transporters (POT) that leverage a proton gradient for peptide import and (2) ABC-transporters that use ATP hydrolysis as energy source for peptide import. Bacterial POTs are monomeric, multi-transmembrane (TM) helix-containing proteins that typically import various di- or tripeptides (*35*). Bacterial ABC-transporters associated with peptide uptake (e.g. Dpp-, Ddp- and Opp-transporters) require a periplasmic binding protein that delivers the captured peptidic substrate to a multi-subunit transmembrane complex for ATP-dependent promiscuous di- and oligopeptide transport.

In order to identify a potential uptake system for G-AisoK, we used single gene knockouts (*36*), which have individual transporter domains deleted, for amber suppression of sfGFP-N150TAG in the presence of the wt-PyIRS/PyIT pair and G-AisoK. We hypothesized that amber suppression of sfGFP would be abolished or diminished, if a potentially involved transporter system was absent. Deletion of the POT-family members and components of the *ddp* or *dpp* tranporters did not have any influence on efficient sfGFP production. In contrast, individual knockouts of genes constituting the *opp* operon led to complete abolishment of sfGFP expression in the presence of G-AisoK (Fig. 2a). The Opp transporter shares its general domain organization with other ABC transporters (*37*): it consists of the periplasmic binding protein (OppA), two transmembrane domains (TMDs, OppB and OppC) that span the periplasmic membrane and two cytoplasmic nucleotide-binding domains (NBDs, OppD and OppF, Fig. 2b) that bind and hydrolyze ATP. Docking of peptide-bound OppA to the TMDs triggers ATP binding and dimerization of the NBDs, leading to uptake of the substrate into the translocation channel. Hydrolysis of ATP results in dissociation of the NBD-dimerization interface and flips the TMDs to trigger release of the substrate into the cytosol. ADP-ATP exchange re-induces NBD-dimerization and the TMDs can again bind to OppA (Fig. 2c).

Individual deletions of either the binding protein OppA, or any of the two TMDs or NBDs led to complete loss of amber suppression and sfGFP fluorescence in the presence of G-AisoK, while no differences in sfGFP-N150BocK expression yields were observed, indicating that the Opp transporter may be specifically involved in G-AisoK uptake (Fig. 2a). Confirming these expression results, uptake assays showed unchanged intracellular BocK concentrations between K12 cells and D*opp*A-K12 cells, while AisoK, which accumulated in millimolar concentrations in K12 cells upon addition of G-AisoK, was not detectable when the *opp*A gene was deleted (Fig. 2d).

Remarkably, plasmid-based constitutive expression of OppA in D*opp*A-K12 cells completely rescued amber suppression in the presence of G-AisoK (Fig. 2e).

OppA is known to promiscuously bind to 2-5 amino acid long peptides and shows some preference for tri- and tetrapeptides containing positively charged side chains. Previously reported crystal structures of the apo and the liganded form of OppA have elucidated its binding mechanism: OppA shows a three-domain architecture and upon ligand binding, the N- and C-terminal domains rearrange in a Venus fly-trap mechanism to adopt a closed conformation, in which the bound peptide is completely engulfed inside the protein (38). Furthermore, available structural information of a OppA:tripeptide complex indicates that the OppA binding cavity provides large hydrated pockets for various side chains of the tripeptide. Direct interactions between the peptidic substrate and OppA target the backbone and its termini. The N-terminus of the bound tripeptide makes multiple interactions with OppA residues and is kept in place by an extensive network of hydrogen bonds and salt bridges. Specifically, the protonated a-amine of the bound tripeptide forms a salt bridge with OppA residue D445, which is kept in its deprotonated form by hydrogen-bonding to Y135 and H187.

The C-terminus of the tripeptide forms a salt bridge with R439. To experimentally verify if these interactions are also necessary for recognition of G-AisoK, we constitutively co-expressed OppA-D445A or OppA-R439A in D*opp*A-K12 cells. Expression of the OppA-R439A variant did not impact G-AisoK dependent amber suppression of sfGFP, implying that R439 may not be involved in interacting with the C-terminus of isopeptide-linked G-AisoK. In stark contrast, overexpression of the OppA-D445A variant did not lead to any sfGFP expression in the presence of G-AisoK, indicating- that the N-terminal amine of G in G-AisoK is interacting with OppA in a similar way as the one of a linear tripeptide (Fig. 2e).

To identify a potential peptidase responsible for G-AisoK to AisoK processing within the *E. coli* cytosol, we performed amber suppression experiments in single-gene knockouts (*36*) that had individual peptidases or proteases deleted in the presence of G-AisoK. We reasoned that lack of N-terminal glycine processing should lead to diminished protein expression yields. We could however not identify one specific peptidase solely responsible for G-AisoK to AisoK processing and speculate that G-AisoK may be a substrate for several promiscuous aminopeptidases (*39*).

### Example 3: A versatile toolbox for efficient encoding of diverse chemical functionalities into POIs

As OppA shows little sequence specificity for bound peptidic substrates, we envisioned to leverage our propeptide strategy for the efficient incorporation of a range of functionalities typically available for GCE, including moieties for site-specific protein conjugation and crosslinking (Fig. 3a). To test the scope of a potential G-XisoK-based toolbox for protein modification, we first synthesized a panel of G-XisoK derivatives bearing natural amino acids serine, threonine, cysteine, valine, leucine, proline or histidine at position X. Supplementing K12 cells with these G-XisoK derivatives, led to efficient suppression of sfGFP-N150TAG and Ub-K63TAG using wt-*Mb*PyIRS/PyIT or appropriate variants thereof that were identified through screening of a large panel of PyIRS-variants available in the lab, with almost no truncation product visible. Importantly, in all cases, MS-analysis confirmed site-specific incorporation of the corresponding XisoK dipeptides and severely reduced protein expression yields were observed when cells were supplemented with XisoK derivatives (Fig. 3b). Efficient encoding of such XisoK derivatives is interesting, as lysine aminoacylation, in which the e-amino group of lysine is covalently linked to the **α**-carboxyl of any of the 20 naturally occurring amino acids, was recently identified as a reversible PTM (*41*, *42*). As previous attempts at directly encoding S/T/P/CisoK derivatives via GCE proved very inefficient(*19*, *41, 43-45*) (Fig. 4b), functional studies on these PTMs are elusive.

Furthermore, CisoK-modified proteins are ideally suited for native chemical ligation approaches, as has been shown for generating Ub-conjugates (*46*, *47*). Comparing CisoK-incorporation efficiencies obtained via our propeptide strategy with published CisoK-incorporation yields using specifically evolved PylRS-variants, impressively shows the advantage of actively pumping G-CisoK into bacterial cells (*19*). This indicates that intracellular ncAA concentration may be more crucial for efficient ncAA incorporation than finely tuning and evolving PyIRS-variants.

Site-specific incorporation of XisoK derivatives, in which X is a natural amino acid, offers also the unique possibility to introduce a specific natural amino acid with an N-terminal **α**-amine within a POI sequence, equipping a protein with a second, artificial N-terminus. This allows internal protein labeling by applying various bioconjugation strategies that have been developed for targeting specific N-termini. (*48*)

By efficient encoding of PisoK (via uptake of G-PisoK, Fig. 3b), we introduce proline bearing a free **α**-amine at an internal site of a target protein and show that this allows tyrosinase-mediated labeling with phenol derivatives. (*49*) Tyrosinase oxidizes p-cresol to the corresponding o-quinone intermediate that can oxidatively couple to the free **α**-amine of proline in PisoK. We show specific and quantitative labeling of PisoK-modified Ub, establishing a solid chemoenzymatic labeling approach that is applicable to any user-chosen site in various POIs (Fig. 3c).

When screening for PyIRS-variants for incorporation of XisoK derivatives bearing aromatic side chains at position X, such as in HisoK (Fig. 3a), we could not identify any positive hits upon supplementing cells with G-HisoK. To verify if this stems from poor G-HisoK recognition by OppA or lack of appropriate PyIRS-variants for HisoK, we created a custom-designed *Mb*PyIRS library with five randomized positions in the active site and subjected this library to alternating rounds of positive and negative selection in K12 cells combined with fluorescence readout in the presence of G-HisoK. Gratifyingly, a novel *Mb*PyIRS-variant supported HisoK incorporation in the presence of G-HisoK, but not when supplementing cells with HisoK (Fig. 3b), indicating that OppA also binds and delivers G-XisoK amino acids with bulky and aromatic X-side chains. Efficient incorporation of synthetically easily accessible histidine-containing ncAAs may prove useful for expanding the range of genetically encoded metal coordination environments and may advance the generation and engineering of metalloenzymes with optimized properties and novel activities. (*50, 51*)

Importantly, also non-canonical side chains can be incorporated at position X to further expand the G-XisoK-toolbox for efficient incorporation of diverse functionalities. We explored the uptake of G-XisoK derivatives, where X contained functionalities for bioorthogonal labeling (*52*), as well as for light-induced and proximity-induced chemical crosslinking (*53*, *54*). As a considerable advantage of our propeptide strategy, G-XisoK derivatives can be easily synthesized at large scales via SPPS with many non-canonical side chains commercially available as amino acid building blocks. In standard GCE approaches, apart from limiting incorporation yields, the synthetic accessibility of ncAAs often presents a considerable challenge. We first explored incorporation of functionalities that would be amenable for bioorthogonal protein labeling via Cu(I)-catalyzed azide alkyne cycloaddition (CuAAC). A G-XisoK derivative, bearing propargyl-glycine at position X, allowed very efficient installation of PrgisoK into diverse target proteins (Fig. 3b), including an eGFP-specific nanobody (eGFPNb-R75TAG), which was successfully labeled with a fluorophore-conjugated azide moiety via CuAAC (Fig. 3d). As seen for all other tested derivatives, PrgisoK incorporation was successful only when cells were supplemented with G-PrgisoK, but not when supplemented with PrgisoK (Fig. 3b). Similarly, PrgisoK incorporation yields using the corresponding G-bearing tripeptide, compare very favorably to recently reported PrgisoK incorporation efficiencies using a specific PyIRS-variant identified for PrgisoK incorporation (*41*).

To expand the G-XisoK-toolbox to ncAAs useful for mapping and trapping PPIs, we explored efficient incorporation of different crosslinker moieties using our propeptide strategy. We integrated commercially available photoleucine (pL) as residue X in the G-XisoK scaffold. Supplementing K12 cells with this tripeptide, enabled efficient encoding of a diazirine moiety into a POI using an *Methanomethylophilus alvus (Ma)* PyIRS-derived variant (Fig. 3b) and UV-induced crosslinking of PPIs, as exemplified by crosslinking of glutathione-S-transferase (GST) dimer, as well as sfGFP dimer (Fig. 3e). As pL is commercially available, synthesis of the needed tripeptide via SPPS is much easier, more efficient, and scalable than synthetic access to other diazirine-bearing ncAAs reported for GCE and photo-crosslinking (*53*-*55*), highlighting an important advantage of our strategy.

For trapping transient PP!s in a proximity-induced manner, we designed a G-XisoK scaffold, bearing the finely tuned electrophile chloroalanine (CIA) at position X. Incubation of K12 cells with G-CIAisoK led to efficient incorporation of CIAisoK into different POIs (Fig. 3b). When CIAisoK is placed in proximity to a nucleophilic amino acid in an interacting protein, an S_{N}2 nucleophilic reaction can take place, covalently stabilizing the protein complex by a stable thioether bridge (Fig. 3f). By pairwise incorporation of CIAisoK and cysteine residues at protein-protein interfaces, we covalently stabilized a variety of low-affinity protein complexes with K_{D}s in the micromolar to low millimolar range in living cells, such as a sfGFP homodimer, the interaction between affibody and protein Z (56) as well as the ternary GDP-bound complex between small G-protein Rab1b and the guanine nucleotide exchange factor (GEF) domain of DrrA (57) (Fig. 3f). Distances of 8-12 Å between the corresponding Ca atoms of cysteine and the lysine of CIAisoK could be efficiently crosslinked. Apart from cysteine, also histidine and glutamate showed successful covalent homodimer formation with CIAisoK-modified sfGFP. In vitro crosslinking using purified affibody- and protein-Z-variants confirmed that crosslinking is specific for ClAisoK, while no crosslinking was observed for AisoK-bearing affibody, as expecte.

### Example 4: Scalable and cheap XisoK incorporation through OppA evolution

For all tested G-XisoK tripeptides, we observed highly efficient protein production in chemically-defined auto induction (Al) media (58). Amber suppression in nutrient-rich media, such as lysogeny broth (LB) or 2-YT, did, however, not give the desired yields of protein expression (Fig. 4a). We hypothesized that G-XisoK tripeptides may compete for OppA-binding with short linear peptides that are abundant in nutrient-rich expression media based on peptone or tryptone. This was confirmed by uptake assays determining intracellular SisoK concentrations, which upon G-SisoK supplementation, were sixfold lower in 2-YT compared to AI media, indicating that OppA-mediated active transport is impaired in such conditions. This drastically impacts the implementation of our versatile G-XisoK toolbox for cheap and scalable protein production, as chemically-defined AI media is much more expensive and cumbersome to prepare than regular nutrient-rich growth media and does not support as high cell-density cultures leading to decreased biomass and lower overall expression yields of ncAA-modified proteins.

We therefore aimed at evolving an engineered OppA-variant that preferentially binds G-SisoK over short linear peptides as present in peptone or tryptone-based media. We developed a fluorescence-activated cell sorting (FACS)-based screening platform to alter the binding preference and selectivity of OppA using directed evolution. Our screening system couples uptake of the G-SisoK tripeptide to sfGFP fluorescence by suppressing the amber codon in sfGFP-N150TAG (Fig. 4b). We co-transformed an error-prone OppA-library together with the wt-MbPyIRS/PyIT pair and sfGFP-N150TAG into DoppA-K12 cells in AI media and subjected these cells to subsequent rounds of enrichment from low (1g/L) to high (16g/L) tryptone concentrations. We identified four converging OppA-variants each harboring 4-5 mutations distributed all over the OppA-fold. Mutations that were present in more than one variant or were structurally close to each other, were identified as hotspot positions and selected for saturation mutagenesis on all four OppA-variants and wt-OppA. The obtained library was again subjected to multiple FACS-based enrichment steps in LB and 2-YT media. The final selected OppA-variant (OppA-ev2) contains a total of seven mutations, distributed over the entire OppA-fold, with only one mutation (R439Q) being closer than 4 Å to the binding site. We introduced these mutations into the K12 genome via lambda red-mediated homologous recombination to create a K12-derived engineered *E. coli* strain for scalable and cheap production of XisoK-bearing proteins in peptide containing media, which we dubbed lsoK12. Doubling times for lsoK12 and its parent strain were comparable both in AI and 2-YT media. With the lsoK12 strain in hand, we first measured intracellular SisoK concentrations in presence of G-SisoK in 2-YT media. Gratifyingly, intracellular SisoK concentrations substantially increased (7-10-fold) when compared to K12 cells (Fig. 4c), while BocK concentrations did not differ between the two *E. coli* strains, indicating that OppA-ev2 indeed preferentially binds and transports G-SisoK over linear peptides present in 2-YT (Fig. 4c). Importantly, the observed elevated SisoK concentrations in lsoK12 cells led to efficient amber suppression of TAG-containing constructs in 2-YT media, exhibiting similar efficiencies as observed for K12 cells grown in defined AI media (Fig. 4a).

To confirm OppA-ev2 binding preference, we measured K_{D}s of wt-OppA and OppA-ev2 towards G-SisoK, a linear GSK tripeptide (mimicking linear tryptone peptides) and SisoK via microscale thermophoresis (Fig. 4d). While both wt-OppA and OppA-ev2 showed similarly low binding affinities towards SisoK (~300 µM), affinity of G-SisoK towards OppA-ev2 is slightly increased compared to wt-OppA (37 µM) versus 50 µM). Interestingly, binding of linear GSK was four-fold decreased for OppA-ev2 versus wt-OppA (275 µM versus 71 µM), corroborating the *in cellulo* data that G-SisoK is able to compete with linear tryptone-derived peptides in the presence of OppA-ev2. In an attempt to structurally rationalize this binding behavior, we mapped the seven mutations found in OppA-ev2 onto a published OppA structure liganded to a linear tripeptide (PDB ID: 3TCF)(*38*). Most of the mutations are not directly contacting the bound tripeptide, but interestingly residue R439 that is important for keeping the C-terminus of the linear tripeptide in place is mutated to glutamine in OppA-ev2. The R439Q mutation may therefore be responsible for the observed lowered affinity for linear GSK, while the binding affinity of G-SisoK, which does not display a carboxy group at this position, is not affected by this mutation.

As the OppA-ev2-variant was evolved in the presence of G-SisoK, we wondered if preferential binding and uptake was extendable to other G-XisoK derivatives. Indeed, all of the tested G-XisoK derivatives (X=A, S, T, C, V, L, P, H, Prg, pL and CIA) led to very efficient amber suppression of sfGFP-N150TAG, with hardly any amounts of truncated side-product when expressed in lsoK12 cells grown in 2-YT media, resembling protein yields obtained in K12 cells grown in chemically-defined and peptide-free AI media (Fig. 4e). In fact, tripeptide uptake was so efficient in lsoK12 cells that G-SisoK concentrations as low as 50-100 µM led to similar incorporation yields as observed in K12 cells, supplemented with 1 mM G-SisoK, thereby decreasing necessary ncAA concentrations by 10-fold (Fig. 4f). We show that our propeptide strategy combined with engineered lsoK12 enables high-yielding XisoK-incorporation at various positions in diverse target proteins spanning sizes from 7 to 85 kDa. Amber suppression of PCNA, β-lactamase, SUMO2, Calmodulin, eGFPNb, and Hsp82 attest to the wide applicability of the G-XisoK/lsoK12 combination resulting in highly efficient production of XisoK-modified proteins matching or even exceeding wt expression yields. Interestingly, in many cases G-XisoK uptake was also improved when lsoK12 cells were grown in AI media. Preparative large-scale production and purification of a GFP nanobody with an ncAA bearing a propargyl moiety showed that the G-PrgisoK/lsoK12 combination resulted in similar purified protein yields (44 mg/L) as obtained for wt expression (41 mg/L) and exceeded yields from a previously reported optimized alkyne-bearing ncAA/PyIRS combination. Increasing intracellular ncAA concentrations via efficient tripeptide uptake facilitated also multi-site amber suppression within one target protein. We introduced TAG-codons at up to three positions into Histone H3 (K27, K79, K122) and expressed the corresponding variants in the presence of G-SisoK. lsoK12 outperformed K12 in single, double and triple amber suppression with expression yields rivaling wt-H3 production, while only minute amounts of doubly- and triply-suppressed protein were obtained with the gold-standard ncAA BocK (Fig. 4g).

### Example 5: Concomitant uptake strategy for efficient encoding of two different ncAAs

Efficient G-XisoK uptake and its processing leads to high intracellular concentrations not only of dipeptide XisoK, but also of the N-terminal glycine that is cleaved off. As OppA is quite promiscuous in regard to bound peptide sequences, we hypothesized that also other side chains, including those of ncAAs, might be accepted at this position and corresponding non-canonical tripeptides might be efficiently transported into the *E. coli* cytosol. If ncAAs at this N-terminal position were also efficiently cleaved by peptidases, this could present a general tool to increase the intracellular concentration of two encodable ncAAs with one single, easily synthesizable tripeptide. We first synthesized a K-SisoK tripeptide, with lysine as N-terminal amino acid instead of glycine. Interestingly, supplementation of K12 or lsoK12 cells with K-SisoK led to similarly efficient amber suppression of sfGFP-N150TAG in the presence of wt-MbPyIRS/PyIT as observed for G-SisoK. LC-MS analysis of purified sfGFP confirmed incorporation of SisoK, proving that indeed K-SisoK must be efficiently transported into cells and N-terminally processed within the *E. coli* cytosol to K and SisoK.

In order to leverage such a concomitant uptake strategy for the site-specific incorporation of two different ncAAs into a single protein, we designed a tripeptide that contains a PTM-bearing ncAA and a photocrosslinker ncAA. Dual site-specific incorporation of such moieties into the same POI represents an ideal tool to investigate PTM-specific protein interactors or to chemically stabilize transient POI-reader complexes (*59*). pLisoK was shown to be incorporated successfully into target proteins using a *Ma*PyIRS-variant and supplementing cells with G-pLisoK (Fig. 3b). Acetyl-lysine (AcK), an abundant PTM of both prokaryotic and eukaryotic proteins can be specifically incorporated into target proteins by using the previously published *Mb*PyIRS-variant AcKRS3 (*60*). Importantly, the two PyIRS-variants are orthogonal to each other (*61*) with AcK not being a substrate for the *Ma*PyIRS-variant and vice-versa. We synthesized the tripeptide AcK-pLisoK (Fig. 5a) and showed its efficient uptake and cleavage leading to high intracellular concentrations of AcK and pLisoK, which allowed efficient dual suppression of a TAA codon by AcK and a TAG codon by pLisoK within the same target protein (sfGFP-N40Ack-N150pLisoK, Fig. 5b) using the corresponding orthogonal PyIRS/PyIT pairs. Importantly, higher protein yields were achieved by supplementing cells with 0.5 mM of AcK-pLisoK, rather than adding 0.5 mM of both AcK and G-pLisoK to the medium, attesting to efficient uptake and cleavage (Fig 5b). LC-MS analysis of full-length sfGFP confirmed dual incorporation of AcK and pLisoK (Fig. 5c). To confirm true mutual orthogonality and absence of misincorporation of the two ncAAs and their respective PyIRS-variants, we designed an Ub-SUMO2 fusion construct with a TEV protease recognition sequence linking the two target proteins (Ub-K48TAG-TEV-SUMO-K11TAA, Fig. 5d). Expression, purification and subsequent TEV cleavage of the construct, followed by LC-MS analysis identified specific incorporation of pLisoK according to the TAG codon at position 48 of Ub and encoding of AcK in response to the TAA codon at position 11 of SUMO2 (Fig. 5d).

### Conclusion and discussion

Low protein production yields combined with difficult chemical access to ncAAs are longstanding and critical challenges in genetic code expansion that have hampered widespread and straightforward implementation of such approaches for generating proteins with potential therapeutic and biotechnological significance. In this study we successfully demonstrate the potential of leveraging a propeptide strategy together with the engineering of a bacterial membrane transporter to enhance intracellular concentrations of ncAAs and thereby boost expression yields of proteins bearing site-specifically incorporated ncAAs to rival those of wt-proteins. Isopeptide-linked G-XisoK tripeptides serve as trojan horses that are easily synthesized from commercially available building blocks via SPPS, and are privileged ligands for the periplasmic binding protein OppA, enabling active, ATP-driven transport into the cytosol. Once inside the cell, the N-terminal glycine is enzymatically processed, leading to intracellular accumulation of isopeptide-linked XisoK ncAAs that serve as excellent substrates for orthogonal PyIRS/PyIT pairs. We show very efficient encoding of eleven different XisoK ncAAs, seven of which have not been incorporated into proteins before, greatly enhancing the chemical space of recombinantly generated proteins via GCE. Among these functionalities are ncAAs bearing bioorthogonal handles, novel PTMs, chemical crosslinker and photocrosslinker moieties, as well as functionalities for chemoenzymatic ligations and we show proof-of-principle applications for all examples. XisoK ncAAs have positively charged side chains under physiological conditions, making them ideal moieties for applications like protein labeling, where site-specific incorporation at surface-exposed positions is required. This avoids aggregation and misfolding associated with large hydrophobic ncAAs, as typically used for labeling purposes(*62*, *63*). With these advantages, we foresee that the XisoK-toolbox can be further developed through aaRS engineering to accommodate functionalities not addressed here such as moieties for inverse-electron demand Diels-Alder cycloadditions or spectroscopic probes.

To make our approach amenable to protein production in nutrient-rich, peptide-containing expression media and to evolve an OppA variant that preferentially shuffles isopeptide-linked tripeptides over linear tripeptides into the *E. coli* cytosol, we have developed a FACS-based evolution platform for screening OppA libraries. The high-throughput evolution of periplasmic binding proteins holds untapped potential for expanding the scope of ncAA-peptide substrates for which transport could be engineered. We envision that this may lead to the development of a generalizable system for ncAA transport, allowing for the design and incorporation of ncAAs without the need to design for passive membrane permeability. Such advancements will further expand the chemical space available for genetic code expansion.

Engineering transport systems could facilitate the discovery and evolution of orthogonal aaRS enzymes for charging and encoding ncAAs. This is exemplified by XisoK ncAAs, where previous attempts at incorporation required extensive screening or evolution for moderate efficiencies. In contrast, with G-XisoK-based uptake, efficient incorporation for a variety of new ncAAs was achieved with minimal aaRS engineering. We anticipate that screening and evolving orthogonal aaRS enzymes under increased ncAA regimes as obtainable via active uptake may yield even more successful outcomes.

Beyond increasing incorporation efficiencies, active uptake offers an economical strategy for producing ncAA-bearing proteins. With active transporters such as Opp, significantly lower concentrations of ncAA can be used in media without compromising on protein yields. We envision that Opp-based active uptake can work synergistically with other efforts to improve GCE, including the use of release factor deficient strains(*13*, *14*), or genomically recoded *E. coli* for incorporating multiple ncAAs(*16*, *17*). Excitingly, we have shown that Opp-based uptake and enzymatic processing of corresponding isopeptide-linked tripeptides can be adapted for dual ncAA incorporation and could be expanded further for multi ncAA incorporation in combination with discovery and evolution of corresponding uptake systems and tailoring enzymes.

Adapting our propeptide strategy combined with discovery and engineering of transport systems in other model organisms/cell lines such as yeast or mammalian cells may prove a game changer for scalable expression of ncAA-containing proteins that cannot be expressed in *E. coli,* allowing efficient generation of homogenously modified antibody-drug conjugates.

### Methods

### General methods: Plasmids and Reagents

Genes encoding GST-E51TAG, tyrosinase and Histone H3 mutants were ordered as DNA string from Twist biosciences and cloned into the pBAD vector via standard restriction cloning with enzymes from New England Biolabs. Single point mutants of OppA as well as insertion of a 3C site on UbK63 were done via Site-directed, Ligase-Independent Mutagenesis (SLIM) (J. Chiu, P. E. March, R. Lee, D. Tillett, Site-directed, Ligase-Independent Mutagenesis (SLIM): a single-tube methodology approaching 100% efficiency in 4 h. Nucleic Acids Res 32, e174 (2004)). Recombination plasmid pSIJ8 was purchased from Addgene (Addgene ID: 68122). Oligonucleotide primers were ordered from Microsynth AG unless otherwise specified. General solvents and chemical reagents were purchased from Sigma Aldrich, cslabs, Fisher Scientific, Carbolution or Acros Organics. Fmoc building blocks of photoleucine, propagylglycine and chloroalanine were purchased from Iris Biotech. All reagents were used without further purification.

SDS-PAGE gels (Bolt^{™} Bis-Tris Plus Mini Protein Gels, 4-12%, Invitrogen) were run on a Bolt^{™} Mini Gel Tank (Invitrogen) system (165 V for 40 minutes) and stained with Quick Coomassie Stain (Generon). As a marker, PageRuler Prestained Plus Protein Ladder 10-250 kDa (ThermoFisher) was used. Proteins were transferred onto a nitrocellulose membrane using a Bio-Rad Trans-blot Turbo Transfer System. After transfer, the membrane was blocked with 5% skim milk in TBS-T buffer for 1 h at RT and incubated with the appropriate HRP-coupled antibody. Imaging of western blots was performed on an Amersham ImageQuant^{™} 800 using Immobilon^{®} Forte Western HRP Substrate (Millipore).

Peptide and protein LC-MS was done using an Agilent Technologies 1260 Infinity LC-MS system with a 6310 Quadrupole spectrometer. Proteins were measured on a Phenomenex Jupiter C4 300 A LC Column (150 × 2 mm, 5 µm) and peptides, on a Luna Omega PS C18 (100 × 2.1 mm, 3 µm). The solvent system consisted of 0.1% formic acid in water (solvent A) and 0.1% formic acid in acetonitrile (solvent B). Both proteins and peptides were analyzed in positive mode.

**Table 3: Plasmids used in this study**

| **Plasmid** | **Resistance** | **Description** |
|---|---|---|
| pBAD_sfGFP_N150TAG_H6 | Ampicillin | sfGFP-N150TAG with a C-terminal His6 tag under an arabinose promoter |
| pBAD_sfGFP_wt_H6 | Ampicillin | sfGFP wt with a C-terminal His6 tag under an arabinose promoter |
| pBAD_Ub_K63TAG_H6 | Ampicillin | Ubiquitin-K63TAG with a C-terminal His6 tag under an arabinose promoter |
| pEVOL_*Mb*PyIRS | Chloramphenicol | Two copies of *Mb*PyIRS wt under an arabinose promotor and a glnS |
| | | promotor and *Mb* tRNA_{CUA} under a proK promoter. |
| pEVOL_*Mb*PyIRS_C313V | Chloramphenicol | Two copies of *Mb*PyIRS C313V under an arabinose promotor and a glnS promotor and *Mb* tRNA_{CUA} under a proK promoter |
| pEVOL_*Ma*PylRS_IP | Chloramphenicol | Two copies of *Ma*PyIRS H227I, Y228P under an arabinose promotor and a glnS promotor and *Ma* tRNA_{CUA} under a proK promoter |
| pEVOL_HisoKRS | Chloramphenicol | Two copies of *Mb*PyIRS Y271L C313T under an arabinose promotor and a glnS promotor and *Mb* tRNA_{CUA} under a proK promoter |
| pEVOL_DiazKRS | Chloramphenicol | Two copies of DiazKRS under an arabinose promotor and a glnS promotor and *Mm* tRNA_{CUA} under a proK promoter |
| pBAD_eGFP-NB_wt_H6 | Ampicillin | eGFP nanobody wt with a C-terminal His6 tag under an arabinose promoter |
| pBAD_eGFP-NB_R17TAG_H6 | Ampicillin | eGFP nanobody-R17TAG with a C-terminal His6 tag under an arabinose promoter |
| pBAD_eGFP-NB_R75TAG_H6 | Ampicillin | eGFP nanobody-R75TAG with a C-terminal His6 tag under an arabinose promoter |
| pBAD_GST_E51TAG_H6 | Ampicillin | GST-E51TAG with a C-terminal His6 tag under an arabinose promoter |
| pBAD_3C_Ub_K63TAG_H6 | Ampicillin | Ub-K63TAG with a N-terminal 3C cleavage site and a C-terminal His6 tag under an arabinose promoter |
| pPyIT_CAT_111TAG | Tetracycline | Chloramphenicol acetyl transferase with a TAG stop codon at position 111 |
| pBK_*Mb*PyIRS_lib | Ampicillin | *Mb*PyIRS library with positions A276, Y271, L274, C313 and M315 randomized with NNK primers under a *gInS* promoter |
| pYOBB_Barnase_3TAG | Chloramphenicol | Barnase with a TAG stop codon at position 3 |
| pPylT_sfGFP150TAG_ CAT_111TAG | Tetracycline | sfGFP with a TAG stop codon at position 150 under an arabinose promoter and Chloramphenicol acetyl transferase with a TAG stop codon at position 111 |
| pEVOL_*Mb*PyIRS_*opp*A_wt | Chloramphenicol | *Mb*PyIRS under an arabinose promotor and *opp*A wt under a *gIn*S promoter |
| pEVOL_*Mb*PyIRS_*opp*A_D446A | Chloramphenicol | *Mb*PyIRS under an arabinose promotor and *oppA* D446A under a *gInS* promoter |
| pEVOL_*Mb*PyIRS_*opp*A_R439A | Chloramphenicol | *Mb*PyIRS under an arabinose promotor and *opp*A R439A under a *gIn*S promoter |
| pEVOL_*Mb*PyIRS_*opp*A_EP_lib | Chloramphenicol | *Mb*PyIRS under an arabinose promotor and a error prone library of oppA under a glnS promoter |
| pEVOL_*Mb*PyIRS_*opp*A_ trimer_lib | Chloramphenicol | *Mb*PyIRS under an arabinose promotor and a site saturation library of oppA under a glnS promotor |
| pEVOL_*Mb*PyIRS_*opp*A_ev2 | Chloramphenicol | *Mb*PyIRS under an arabinose promotor and a site saturation library of oppA ev2 under a glnS promoter |
| pBAD_oppA_wt_H6 | Ampicillin | *oppA* wt with a C-terminal His6 tag under an arabinose promoter |
| pBAD_oppA_ev2_H6 | Ampicillin | *oppA* ev2 with a C-terminal His6 tag under an arabinose promoter |
| pBAD_PCNA_wt_H6 | Ampicillin | PCNA wt with a C-terminal His6 tag under an arabinose promoter |
| pBAD_PCNA_K164TAG_H6 | Ampicillin | PCNA-K164TAG with a C-terminal His6 tag under an arabinose promoter |
| pBAD_β-lactamase wt_H6 | Ampicillin | β-lactamase wt with a C-terminal His6 tag under an arabinose promoter |
| pBAD_β-lactamase_ K221TAG_H6 | Ampicillin | β-lactamase-K221TAG with a C-terminal His6 tag under an arabinose promoter |
| pBAD_SUMO2_wt_H6 | Ampicillin | SUMO2 wt with a C-terminal His6 tag under an arabinose promoter |
| pBAD_SUMO_K45TAG_H6 | Ampicillin | SUMO2-K45TAG with a C-terminal His6 tag under an arabinose promoter |
| pBAD_Calmodulin_wt_strep | Ampicillin | Calmodulin wt with a C-terminal Strep tag under an arabinose promoter |
| pBAD Calmodulin_ G40TAG_strep | Ampicillin | Calmodulin-G40TAG with a C-terminal Strep tag under an arabinose promoter |
| pBAD_H3_wt_H6 | Ampicillin | Histone H3 wt with a C-terminal His6 tag under an arabinose promoter |
| pBAD_H3_K122TAG_H6 | Ampicillin | Histone H3-K122TAG with a C-terminal His6 tag under an arabinose promoter |
| pBAD_H3_K79TAG_ K122TAG_H6 | Ampicillin | Histone H3K79TAG K122TAG with a C-terminal His6 tag under an arabinose promoter |
| pBAD_H3_K27TAG_ K79TAG_K122TAG_H6 | Ampicillin | Histone H3 K27TAG K79TAG K122TAG with a C-terminal His6 tag under an arabinose promoter |
| pBAD_Hsp82_wt_H7 | Ampicillin | Hsp82 wt with a C-terminal His7 tag under an arabinose promoter |
| pBAD_Hsp82_D452TAG_H7 | Ampicillin | Hsp82-D452TAG with a C-terminal His7 tag under an arabinose promoter |
| pET28_tyrosinase_H6 | Kanamycin | Tyrosinase with a C-terminal His6 tag under a T7 promoter |
| pSIJ8 | Ampicillin | Addgene ID: 68122 |
| pEVOL_AcKRS3(TAA)_ RBS_MaPyIRS_IP(TAG) | Chloramphenicol | AcKRS3 and MaPyIRS_IP under a arabinose promoter polycistronically and *Mb* tRNA_{UUA}, *Ma* tRNA_{CUA} under separate proK promoters |
| pBAD_sfGFP_N40TAA_ N150TAG_H6 | Ampicillin | sfGFP with N40TAA, N150TAG mutations and a C-terminal His6 tag under an arabinose promoter. |
| pBAD_U bK48TAA_TEV_ SUMO2K11TAG_H6 | Ampicillin | Ubiquitin with K48TAA mutation followed by a TEV protease cleavage |
| | | sequenced and SUMO2 with mutation K11TAG and a C-terminal His6 tag under an arabinose promoter. |

**Table 4: Primers used in this study**

| **Primers** | **Sequence** |
|---|---|
| OppA_EP_fwd | CGCTTTGAGGAATCCCATATGGGG (SEQ ID NO:12) |
| OppA_EP_rev | GAAACTGCAGTTAATGGTGATGATGATGGTGC (SEQ ID NO:13) |
| OppA_D221X_W22 2X_fwd | CGTAAGAAGACCCTTAAAA**X01X01**GTCGTAAACGAACGAATC (SEQ ID NO:14) |
| OppA_D221X_W22 2X_rev | CGTTAGAAGACTTTTAAGGTATAGGCACCG (SEQ ID NO:15) |
| OppA_R439X_fwd | CGAATGAAGACATGTGGCC**X01**GCAGGCTGGTGTGCTGAC (SEQ ID NO:16) |
| OppA_R439X_rev | GCATAGAAGACGGCCACATCAAAAGTACCCTGGTGAC (SEQ ID NO:17) |
| OppA_S460X_fwd | GATATGAAGACTTTCGAAC**X01**TCGATGAATACCGCGC (SEQ ID NO:18) |
| OppA_S460X_rev | GCTTAGAAGACGTTCGAAAGCATGGTGTTC (SEQ ID NO:19) |
| OppA_genomic_fw d | TACACATGCTGGTTAATACCAGTAATTATAATGAGGGAGTCCAAAAAACA ATGACCAACATCACCAAGAGAAG (SEQ ID NO:20) |
| OppA_genomic_rev | AAAATCAGACACCGTGGAGCAGGACACTCCTGCCCCACGTATTGCCATTA CTTCACAATGTACATATTCCGGG (SEQ ID NO:21) |

**Table 5: Primers used for SLIM cloning**

| **Construct** | **Primer** | **Sequence** |
|---|---|---|
| pEVOL_*Mb*P ylRS_*opp*A_ D446A | Short fwd | TACAACGAACCAACTTCC (SEQ ID NO:22) |
| | Short rev | ACGGGCCACATCAAAAGTAC (SEQ ID NO:23) |
| | Tail fwd | GCAGGCTGGTGTGCTGCTTACAACGAACCAACTTCC (SEQ ID NO:24) |
| | Tail rev | AGCAGCACACCAGCCTGCACG (SEQ ID NO:25) |
| pEVOL_*Mb*P ylRS_*opp*A_ R439A | Short fwd | GCAGGCTGGTGTGCTGAC (SEQ ID NO:26) |
| | Short rev | CTGGTGACGGGTGTCGAG (SEQ ID NO:27) |
| | Tail fwd | GGTACTTTTGATGTGGCCGCGGCAGGCTGGTGTGCTG (SEQ ID NO:28) |
| | Tail rev | CGCGGCCACATCAAAAGTACCCTGG (SEQ ID NO:29) |

### Protein Sequences

sfGFP N150TAG H6 (SEQ ID NO: 30): 
MPSKGEELFTGVVPILVELDGDVNGHKFSVRGEGEGDATNGKLTLKFICTTGKLPVPWPTLVTTLTYGVQ

CFSRYPDHMKRHDFFKSAMPEGYVQERTISFKDDGTYKTRAEVKFEGDTLVNRIELKGIDFKEDGNILGHK

LEYNFNSH*VYITADKQKNGIKANFKIRHNVEDGSVQLADHYQQNTPIGDGPVLLPDNHYLSTQSVLSKD

PNEKRDHMVLLEFVTAAGITHGMDELYKGSHHHHHH
UbK63TAG H6 (SEQ ID NO:31): 
MQIFVKTLTGKTITLEVEPSDTIENVKAKIQDKEGIPPDQQRLIFAGKQLEDGRTLSDYNIQ*ESTLHLVLRL
RGGHHHHHH
*Mb*PyIRS (SEQ ID NO:2): 
MDKKPLDVLISATGLWMSRTGTLHKIKHHEVSRSKIYIEMACGDHLVVNNSRSCRTARAFRHHKYRKTCK
RCRVSDEDINNFLTRSTESKNSVKVRVVSAPKVKKAMPKSVSRAPKPLENSVSAKASTNTSRSVPSPAKST
PNSSVPASAPAPSLTRSQLDRVEALLSPEDKISLNMAKPFRELEPELVTRRKNDFQRLYTNDREDYLGKLER
DITKFFVDRGFLEIKSPILIPAEYVERMGINNDTELSKQIFRVDKNLCLRPMLAPTLYNYLRKLDRILPGPIKIF
EVGPCYRKESDGKEHLEEFTMVNFCQMGSGCTRENLEALIKEFLDYLEIDFEIVGDSCMVYGDTLDIMHG
DLELSSAVVGPVSLDREWGIDKPWIGAGFGLERLLKVMHGFKNIKRASRSESYYNGISTNL
MaPyIRS H227I Y228P (SEQ ID NO:6): 
MTVKYTDAQIQRLREYGNGTYEQKVFEDLASRDAAFSKEMSVASTDNEKKIKGMIANPSRHGLTQLMN
DIADALVAEGFIEVRTPIFISKDALARMTITEDKPLFKQVFWIDEKRALRPMLAPNLYSVMRDLRDHTDGP
VKIFEMGSCFRKESHSGMHLEEFTMLNLVDMGPRGDATEVLKNYISVVMKAAGLPDYDLVQEESDVYKE
TIDVEINGQEVCSAAVGPIPLDAAHDVHEPWSGAGFGLERLLTIREKYSTVKKGGASISYLNGAKIN
DiazKRS (SEQ ID NO:5): 
MDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPV
STQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQIF
RVDKNFCLRPMLAPNLMNYARKLDRALPDPIKIFEIGPCYRKESDGKEHLEEFTMLNFAQMGSGCTRENL
ESIITDFLNHLGIDFKIVGDSCMVYGDTLDVMHGDLELSSAVVGPIPLDREWGIDKPWIGAGFGLERLLKV
KHDFKNIKRAARSESYYNGISTNL
eGFP-NB wt (SEQ ID NO:32): 
MKYLLPTAAAGLLLLAAQPAMAQVQLVESGGALVQPGGSLRLSCAASGFPVNRYSMRWYRQAPGKERE
WVAGMSSAGDRSSYEDSVKGRFTISRDDARNTVYLQMNSLKPEDTAVYYCNVNVGFEYWGQGTQVTV
SSKKKHHHHHH
eGFP-NB R17TAG H6 (SEQ ID NO:33): 
MKYLLPTAAAGLLLLAAQPAMAQVQLVESGGALVQPGGSL*LSCAASGFPVNRYSMRWYRQAPGKERE
WVAGMSSAGDRSSYEDSVKGRFTISRDDARNTVYLQMNSLKPEDTAVYYCNVNVGFEYWGQGTQVTV
SSKKKHHHHHH
eGFP-NB R75TAG H6 (SEQ ID NO:34): 
MKYLLPTAAAGLLLLAAQPAMAQVQLVESGGALVQPGGSLRLSCAASGFPVNRYSMRWYRQAPGKERE
WVAGMSSAGDRSSYEDSVKGRFTISRDDA*NTVYLQMNSLKPEDTAVYYCNVNVGFEYWGQGTQVTV
SSKKKHHHHHH
GST E51TAG H6 (SEQ ID NO:35): 
MSPILGYWKIKGLVQPTRLLLEYLEEKYEEHLYERDEGDKWRNKKFELGL*FPNLPYYIDGDVKLTQSMAII
RYIADKHNMLGGCPKERAEISMLEGAVLDIRYGVSRIAYSKDFETLKVDFLSKLPEMLKMFEDRLCHKTYL
 
NGDHVTHPDFMLYDALDVVLYMDPMCLDAFPKLVCFKKRIEAIPQIDKYLKSSKYIAWPLQGWQATFGG
GDHPPKHHHHHH
3C-Ub-K63TAG H6 (SEQ ID NO:36): 
MENLLEVLFQGPGGGGSMQIFVKTLTGKTITLEVEPSDTIENVKAKIQDKEGIPPDQQRLIFAGKQLEDGR

TLSDYNIQ*ESTLHLVLRLRGGHHHHHH
OppA H6 (SEQ ID NO:37): 
MGTNITKRSLVAAGVLAALMAGNVALAADVPAGVTLAEKQTLVRNNGSEVQSLDPHKIEGVPESNISRD
LFEGLLVSDLDGHPAPGVAESWDNKDAKVWTFHLRKDAKWSDGTPVTAQDFVYSWQRSVDPNTASPY
ASYLQYGHIAGIDEILEGKKPITDLGVKAIDDHTLEVTLSEPVPYFYKLLVHPSTSPVPKAAIEKFGEKWTQP
GNIVTNGAYTLKDWVVNERIVLERSPTYWNNAKTVINQVTYLPIASEVTDVNRYRSGEIDMTNNSMPIEL
FQKLKKEIPDEVHVDPYLCTYYYEINNQKPPFNDVRVRTALKLGMDRDIIVNKVKAQGNMPAYGYTPPYT
DGAKLTQPEWFGWSQEKRNEEAKKLLAEAGYTADKPLTINLLYNTSDLHKKLAIAASSLWKKNIGVNVKL
VNQEWKTFLDTRHQGTFDVARAGWCADYNEPTSFLNTMLSNSSMNTAHYKSPAFDSIMAETLKVTDE
AQRTALYTKAEQQLDKDSAIVPVYYYVNARLVKPWVGGYTGKDPLDNTYTRNMYIVKHHHHHH
OppA ev2 H6 (SEQ ID NO:38): 
MGTNITKRSLVAAGVLAALMAGNVALAADVPAGVTLAEKQTLVRNNGSEVQSLDPHKIEGVPESNISRD
LFEGLLVSDLDGHPAPGVAESWDNKDAKVWTFHLRKDAKWSDGTPVTAQDFVYSWQRSVDPNTASPY
ASYLQYGHIAGIDEILEGKKPITDLGVKAIDDHTLEVTLSEPVPYFYKLLVHPSTSPAPKAAIEKFGEKWTQP
GNIVTNGAYTLKLAVVNERIVLERSPTYWNNAKTVINQVTYLPIASEVTDVNRYRSGEIDMTNNSMPIELF
QKLKKEIPDEVHVDPYLCTYYYEVNNQKPPFNDVRVRTALKLGMDRDIIVNKVKAQGDMPAYGYTPPYT
DGAKLTQPEWFGWSQEKRNEEAKKLLAEAGYTADKPLTINLLYNTSDLHKKLAIAASSLWKKNIGVNVKL
VNQEWKTFLDTRHQGTFDVAQAGWCADYNEPTSFLNTMLSNHSMNTAHYKSPAFDSIMAETLKVTDE
AQRTALYTKAEQQLDKDSAIVPVYYYVNARLVKPWVGGYTGKDPLDNTYTRNMYIVKHHHHHH
β-lactamase wt H6 (SEQ ID NO:39): 
MGADLADRFAELERRYDARLGVYVPATGTTAAIEYRADERFAFCSTFKAPLVAAVLHQNPLTHLDKLITYT
SDDIRSISPVAQQHVQTGMTIGQLCDAAIRYSDGTAANLLLADLGGPGGGTAAFTGYLRSLGDTVSRLDA
EEPELNRDPPGDERDTTTPHAIALVLQQLVLGNALPPDKRALLTDWMARNTTGAKRIRAGFPADWKVID
 
KTGTGDYGRANDIAVVWSPTGVPYVVAVMSDRAGGGYDAEPREALLAEAATCVAGVLAGSGGSGHHH
HHH
β-lactamase K221TAG H6 (SEQ ID NO:40): 
MGADLADRFAELERRYDARLGVYVPATGTTAAIEYRADERFAFCSTFKAPLVAAVLHQNPLTHLDKLITYT
SDDIRSISPVAQQHVQTGMTIGQLCDAAIRYSDGTAANLLLADLGGPGGGTAAFTGYLRSLGDTVSRLDA
EEPELNRDPPGDERDTTTPHAIALVLQQLVLGNALPPDKRALLTDWMARNTTGA*RIRAGFPADWKVID
KTGTGDYGRANDIAVVWSPTGVPYVVAVMSDRAGGGYDAEPREALLAEAATCVAGVLAGSGGSGHHH
HHH
SUMO2 wt H6 (SEQ ID NO:41): 
MADEKPKEGVKTENNDHINLKVAGQDGSVVQFKIKRHTPLSKLMKAYCERQGLSMRQIRFRFDGQPINE
TDTPAQLEMEDEDTIDVFQQQTGGHHHHHH
SUMO2 K45TAG H6 (SEQ ID NO:42): 
MADEKPKEGVKTENNDHINLKVAGQDGSVVQFKIKRHTPLSKLM*AYCERQGLSMRQIRFRFDGQPINE
TDTPAQLEMEDEDTIDVFQQQTGGHHHHHH
Calmodulin wt strep (SEQ ID NO:43): 
MADQLTEEQIAEFKEAFSLFDKDGDGTITTKELGTVMRSLGQNPTEAELQDMINEVDADGNGTIDFPEFL
TMMARKMKDTDSEEEIREAFRVFDKDGNGYISAAELRHVMTNLGEKLTDEEVDEMIREADIDGDGQVN
YEEFVQMMTAKSAWSHPQFEKGGGSGGGSGGSAWSHPQFEK
Calmodulin G40TAG strep (SEQ ID NO:44): 
MADQLTEEQIAEFKEAFSLFDKDGDGTITTKELGTVMRSL*QNPTEAELQDMINEVDADGNGTIDFPEFL
TMMARKMKDTDSEEEIREAFRVFDKDGNGYISAAELRHVMTNLGEKLTDEEVDEMIREADIDGDGQVN
YEEFVQMMTAKSAWSHPQFEKGGGSGGGSGGSAWSHPQFEK
Histone H3 wt H6 (SEQ ID NO:45): 
MARTKQTARKSTGGKAPRKQLATKAARKSAPATGGVKKPHRYRPGTVALREIRRYQKSTELLIRKLPFQRL
VREIAQDFKTDLRFQSSAVMALQEASEAYLVALFEDTNLCAIHAKRVTIMPKDIQLARRIRGERARSHHHH
HH
Histone H3 K112TAG (SEQ ID NO:46): 
MARTKQTARKSTGGKAPRKQLATKAARKSAPATGGVKKPHRYRPGTVALREIRRYQKSTELLIRKLPFQRL
VREIAQDFKTDLRFQSSAVMALQEASEAYLVALFEDTNLCAIHAKRVTIMP*DIQLARRIRGERARSHHHH
HH
Histone H3 K79 K112TAG H6 (SEQ ID NO:47): 
MARTKQTARKSTGGKAPRKQLATKAARKSAPATGGVKKPHRYRPGTVALREIRRYQKSTELLIRKLPFQRL
VREIAQDF*TDLRFQSSAVMALQEASEAYLVALFEDTNLCAIHAKRVTIMP*DIQLARRIRGERARSHHHH
HH
Histone H3 K27 K79 K112TAG H6 (SEQ ID NO:48): 
MARTKQTARKSTGGKAPRKQLATKAAR*SAPATGGVKKPHRYRPGTVALREIRRYQKSTELLIRKLPFQRL
VREIAQDF*TDLRFQSSAVMALQEASEAYLVALFEDTNLCAIHAKRVTIMP*DIQLARRIRGERARSHHHH
HH
Tyrosinase H6 (SEQ ID NO:49): 
MSNKYRVRKNVLHLTDTEKRDFVRTVLILKEKGIYDRYIAWHGAAGKFHTPPGSDRNAAHMSSAFLPWH
REYLLRFERDLQSINPEVTLPYWEWETDAQMQDPSQSQIWSADFMGGNGNPIKDFIVDTGPFAAGRW
TTIDEQGNPSGGLKRNFGATKEAPTLPTRDDVLNALKITQYDTPPWDMTSQNSFRNQLEGFINGPQLHN
RVHRWVGGQMGVVPTAPNDPVFFLHHANVDRIWAVWQIIHRNQNYQPMKNGPFGQNFRDPMYP
WNTTPEDVMNHRKLGYVYDIELRKSKRSSLEHHHHHH
Hsp82 H7 (SEQ ID NO:50): 
MASETFEFQAEITQLMSLIINTVYSNKEIFLRELISNASDALDKIRYKSLSDPKQLETEPDLFIRITPKPEQKVLE
IRDSGIGMTKAELINNLGTIAKSGTKAFMEALSAGADVSMIGQFGVGFYSLFLVADRVQVISKSNDDEQYI
WESNAGGSFTVTLDEVNERIGRGTILRLFLKDDQLEYLEEKRIKEVIKRHSEFVAYPIQLVVTKEVEKEVPIPE
EEKKDEEKKDEEKKDEDDKKPKLEEVDEEEEKKPKTKKVKEEVQEIEELNKTKPLWTRNPSDITQEEYNAFY
KSISNDWEDPLYVKHFSVEGQLEFRAILFIPKRAPFDLFESKKKKNNIKLYVRRVFITDEAEDLIPEWLSFVKG
VVDSEDLPLNLSREMLQQNKIMKVIRKNIVKKLIEAFNEIAEDSEQFEKFYSAFSKNIKLGVHEDTQNRAAL
AKLLRYNSTKSVDELTSLTDYVTRMPEHQKNIYYITGESLKAVEKSPFLDALKAKNFEVLFLTDPIDEYAFTQL
KEFEGKTLVDITKDFELEETDEEKAEREKEIKEYEPLTKALKEILGDQVEKVVVSYKLLDAPAAIRTGQFGWS
 
ANMERIMKAQALRDSSMSSYMSSKKTFEISPKSPIIKELKKRVDEGGAQDKTVKDLTKLLYETALLTSGFSL
DEPTSFASRINRLISLGLNIDEDEETETAPEASTAAPVEEVPADTEMEEVDPGEQKCEEWKRRYEKEKEKN
ARLKGKVEKLEIELARWRPGSAWSHHHHHHH
Hsp82 D452TAG H7 (SEQ ID NO:51): 
MASETFEFQAEITQLMSLIINTVYSNKEIFLRELISNASDALDKIRYKSLSDPKQLETEPCLFIRITPKPEQKVLE
IRDSGIGMTKAELINNLGTIAKSGTKAFMEALSAGADVSMIGQFGVGFYSLFLVADRVQVISKSNDDEQYI
WESNAGGSFTVTLDEVNERIGRGTILRLFLKDDQLEYLEEKRIKEVIKRHSEFVAYPIQLVVTKEVEKEVPIPE
EEKKDEEKKDEEKKDEDDKKPKLEEVDEEEEKKPKTKKVKEEVQEIEELNKTKPLWTRNPSDITQEEYNAFY
KSISNDWEDPLYVKHFSVEGQLEFRAILFIPKRAPFDLFESKKKKNNIKLYVRRVFITDEAEDLIPEWLSFVKG
VVDSEDLPLNLSREMLQQNKIMKVIRKNIVKKLIEAFNEIAEDSEQFEKFYSAFSKNIKLGVHEDTQNRAAL
AKLLRYNSTKSV*ELTSLTDYVTRMPEHQKNIYYITGESLKAVEKSPFLDALKAKNFEVLFLTDPIDEYAFTQL
KEFEGKTLVDITKDFELEETDEEKAEREKEIKEYEPLTKALKEILGDQVEKVVVSYKLLDAPAAIRTGQFGWS
ANMERIMKAQALRDSSMSSYMSSKKTFEISPKSPIIKELKKRVDEGGAQDKTVKDLTKLLYETALLTSGFSL
DEPTSFASRINRLISLGLNIDEDEETETAPEASTAAPVEEVPADTEMEEVDPGEQKCEEWKRRYEKEKEKN
ARLKGKVEKLEIELARWRPGSA WSHHHHHHH
AcKRS3 (SEQ ID NO:52): 
MDKKPLDVLISATGLWMSRTGTLHKIKHHEVSRSKIYIEMACGDHLVVNNSRSCRTARAFRHHKYRKTCK
RCRVSGEDINNFLTRSTESKNSVKVRVVSAPKVKKAMPKSVSRAPKPLENSVGAKASTNTSRSVPSPAKST
PNSSVPASAPAPSLTRSQLDRVEALLSPEDKISLNMAKPFRELEPELVTRRKNDFQRLYTNDREDYLGKLER
DITKFFVDRGFLEIKSPILIPAEYVERMGINNDTELSKQIFRVDKNLCLRPMMAPTIFNYARKLDRILPGPIKIF
EVGPCYRKESDGKEHLEEFTMVNFFQMGSGCTRENLEALIKEFLDYLEIDFEIVGDSCMVYGDTLDIMHG
DLELSSAVVGPVSLDREWGIDKPWIGAGFGLERLLKVMHGFKNIKRASRSESYYNGISTNL
sfGFP N40TAA N150TAG H6 (SEQ ID NO:53): 
MPSKGEELFTGVVPILVELDGDVNGHKFSVRGEGEGDAT*GKLTLKFICTTGKLPVPWPTLVTTLTYGVQC
FSRYPDHMKRHDFFKSAMPEGYVQERTISFKDDGTYKTRAEVKFEGDTLVNRIELKGIDFKEDGNILGHKL
EYNFNSH*VYITADKQKNGIKANFKIRHNVEDGSVQLADHYQQNTPIGDGPVLLPDNHYLSTQSVLSKDP
NEKRDHMVLLEFVTAAGITHGMDELYKGSHHHHHH
Ub K48TAA TEV SUMO2K11TAG H6 (SEQ ID NO:54):

MQIFVKTLTGKTITLEVEPSDTIENVKAKIQDKEGIPPDQQRLIFAG*QLEDGRTLSDYNIQKESTLHLVLRL

RGGEDLYFQSMADEKPKEGVKTENNDHINL*VAGQDGSVVQFKIKRHTPLSKLMKAYCERQGLSMRQIR

FRFDGQPINETDTPAQLEMEDEDTIDVFQQQNGLHHHHHH

### Synthesis of peptides via solid phase peptide synthesis

All peptides were synthesized via solid phase peptide synthesis (SPPS). Reactions were performed in plastic syringes with a frit using <1g of resin. SPPS was done using the Fmoc-strategy using a 2-Chlorotrityl chloride resin. For charging, 2 eq. of Fmoc protected amino acid and 3 eq of DIPEA were dissolved in DCM and added to 1eq of resin (1.5 mmol/g maximum capacity, 100-200 mesh) and incubated for 1 h on a roller at RT. Resin was then washed 5 times with DCM and 5 times with DMF. Fmoc deprotection was performed by adding a 20% piperidine (v/v) solution in DMF and incubated for 10 min at RT. This process was performed twice for complete deprotection followed by washing the resin 5 times with DMF. For subsequent coupling of amino acids a coupling solution was prepared by dissolving 2 eq. of Fmoc protected amino acid, 1.8 eq. HATU and 3 eq. DIPEA in DMF and mixing for 5 min at RT. Coupling solution was added to the resin and incubated on a roller for 1 h at RT. This process was repeated depending on the length of peptide being synthesized.

For cleavage of the protected peptide off the resin, the resin was washed 5 times with DMF and 5 times with DCM and cleavage was performed by adding 20% HFIP in DCM (v/v) for 10 minutes at RT. This process was repeated 2 times, each time collecting the filtrate. Solvent from the filtrate was evaporated under reduced pressure.

Deprotection was performed by adding 95% TFA with 2.5% water and 2.5% TIPS and stirred at RT till deprotection was complete, monitoring the reaction via LC-MS. Deprotected peptide was subsequently precipitated in cold ether and dissolved in water. Any remaining ether was evaporated under low pressure and the resulting peptide solution was flash frozen in liquid nitrogen and lyophilized to obtain a dry powder.

**Table 6: Peptides used in this study**

| **Peptide** | **Expected mass** | **[M+H]+** |
|---|---|---|
| AisoK | 217.25 | 218.2 |
| G-AisoK | 274.32 | 275.2 |
| SisoK | 233.25 | 234.2 |
| G-SisoK | 290.32 | 291.1 |
| TisoK | 247.28 | 248.2 |
| G-TisoK | 304.35 | 305.2 |
| VisoK | 245.31 | 246.2 |
| G-VisoK | 302.38 | 303.2 |
| CisoK | 249.31 | 250.2 |
| G-CisoK | 306.38 | 307.2 |
| PisoK | 243.29 | 244.2 |
| G-PisoK | 300.36 | 301.2 |
| PrgisoK | 241.27 | 242.2 |
| G-PrgisoK | 298.34 | 299.1 |
| LisoK | 259.33 | 260.2 |
| G-LisoK | 316.4 | 317.2 |
| pLisoK | 271.3 | 272.2 |
| G-pLisoK | 328.37 | 329.2 |
| HisoK | 283.31 | 284.2 |
| G-HisoK | 340.38 | 341.2 |
| K-SisoK | 361.44 | 362.2 |
| AcK-pLisoK | 441.53 | 442.3 |

### Protein purification

### Expression and purification of OppA and variants

Chemically competent *E. coli* K12 Δ*Opp*A cells were transformed with pBAD_OppA_His6. After recovery in 1 mL SOC for 1 h at 37 °C, the cells were cultured in 50 mL of 2-YT supplemented with 100 µg/mL Ampicillin overnight at 37 °C. the overnight culture was then diluted to OD₆₀₀ 0.05 in 2-YT supplemented with 50 µg/mL Ampicillin and grown at 37 °C, 200 rpm till OD₆₀₀ 0.6. Protein expression was induced with 0.05% L-arabinose and the culture grown for 3 h at 37 °C, 200 rpm. Cells were harvested by centrifugation at 7000 × g for 20 min and pellets flash frozen in liquid nitrogen and stored at -20 °C.

Cell pellets were resuspended in lysis buffer (20 mM Tris-HCl pH 8, 300 mM NaCl, 30 mM imidazole, 1 mM PMSF) (30 ml/liter culture) and cells lysed using a cell disruptor (CF1 Constant Systems Ltd., 40 Kpsi) . Lysed cells were centrifuged at 14,000 × g, 20 minutes and cleared lysate loaded on a 5ml HisTrap HP column (Cytiva) pre-equilibrated with wash buffer (20 mM Tris-HCl pH 8, 300 mM NaCl, 30 mM imidazole). The loaded column was then washed with 5 column volumes (CV) of wash buffer. Protein was eluted with a gradient from 30 to 300 mM imidazole over 20 CV, collecting 1.4 ml fractions. Fractions containing protein were pooled and concentrated using Amicon^{®} centrifugal filter units (Millipore, 30 kDa MWCO) and then injected on a HiLoad 16/600 Superdex 75 pg size exclusion (SE) column (Cytiva) equilibrated with SE buffer (2x PBS). SE fractions containing protein were then pooled and concentrated. Any endogenously bound peptides were removed by partially unfolding OppA with 2M Guanidine hydrochloride (GdnHCl) in SE buffer and removing the peptides via dialysis (Pur-A-Lyzer^{™} Maxi, 12 kDa MWCO, Sigma-Aldrich) in SE buffer containing decreasing amounts of GdnHCl from 2 M to 0 M. resulting protein was then flash frozen in liquid nitrogen and stored at -80 °C till further use.

### Expression of XisoK bearing proteins

Chemically competent *E. coli* K12 cells were co-transformed with pBAD_POI (encodes the protein of interest (POI) with a C-terminal H6 tag) and pEVOL_PyIRS (encodes two copies of the PylRS variant and tRNA_{CUA}). After recovery in 1 mL SOC media for 1 h at 37 °C, cells were cultured in 5 ml of 2-YT supplemented with Ampicillin (100 µg/mL) and Chloramphenicol (50 µg/mL) and grown overnight at 37 °C. The overnight culture was then diluted to an OD₆₀₀ of 0.05 in either 2-YT or Autoinducing media(2) supplemented with Ampicillin (100 µg/mL), Chloramphenicol (50 µg/mL) and non-canonical amino acid. Cells grown in 2-YT were grown till an OD₆₀₀ of 0.6 and induced with 0.05% L-arabinose and grown overnight at 37 °C. Cells grown in Autoinducing media were directly grown overnight at 37 °C. Cells of the Overnight culture were harvested by centrifugation at 4000 × g for 10 min and the pellets stored at -20 °C till further use.

### Purification of His6 tagged proteins

Cell pellets were resuspended in lysis buffer (20 mM Tris-HCl pH 8, 300 mM NaCl, 30 mM imidazole, 1 mM PMSF) and the cells lysed via sonication in an ice water bath. Lysed cells were centrifuged at 14,000 × g for 20 minutes and cleared lysate was incubated with Ni Sepharose fast flow beads (1 mL slurry/ 1 L culture, Cytiva) pre-equilibrated with Ni-NTA wash buffer (20 mM Tris-HCl pH 8, 300 mM NaCl, 30 mM imidazole) and incubated on a roller for 1 h at 4 °C. Beads were then washed with 10 CV of wash buffer 3 times and protein eluted with elution buffer (20 mM Tris-HCl pH 8, 300 mM NaCl, 300 mM imidazole). Resulting protein was either directly used for mass determination or further purification was performed via gel filtration using a Superdex 75 increase 10/300 GL column (Cytiva) equilibrated with SE buffer (2xPBS pH 7 for eGFP-NB or 20 mM Potassium phosphate buffer pH 6.5, 100 mM NaCl for PisoK bearing proteins or 1x PBS pH 7.4 for Affibody and ProteinZ). Fractions containing protein were pooled and concentrated using Amicon^{®} centrifugal filter units with the appropriate MWCO. Protein was then flash frozen in liquid nitrogen and stored at -80 °C till further use. Proteins bearing CisoK were treated with methoxyamine (10 µM) protein, 100 mM methoxyamine at pH 4 ammonium acetate) and buffer exchanged in 1xPBS before storage.

### Expression and purification of Tyrosinase

Chemically competent *E. coli* BL21 (DE3) were transformed with pET28_Tyrosinase_H6 (encoding for tyrosinase from *Priesto megaterium*)*.* After recovery with 1 mLof SOC medium for 1 h at 37 °C, the cells were cultured overnight in 50 mL 2-YT medium containing kanamycin (50 µg/mL) at 37 °C, 200 rpm. The overnight culture was diluted to an OD₆₀₀ of 0.05 in 1 L of fresh 2-YT medium supplemented with kanamycin (25 µg/mL) and cultured at 37 °C with shaking (200 rpm) until OD₆₀₀ reached 0.5.

IPTG was added to a final concentration of 0.4 mM and protein expression was induced for 18 h at 37 °C. The cells were harvested by centrifugation (4000 × g, 20 min, 4 °C), resuspended in lysis buffer (20 mM Tris-HCl pH 8, 300 mM NaCl, 30 mM imidazole, 0.2 mM PMSF) and lysed via sonication in an ice water bath. The lysed cells were centrifuged (15,000 × g, 40 min, 4 °C) and the cleared lysate was applied to Ni Sepharose fast flow beads (1mL slurry/ 1L culture) pre-equilibrated with Ni-NTA wash buffer (20 mM Tris-HCl pH 8, 300 mM NaCl, 30 mM imidazole) and incubated on a roller for 1 h at 4 °C. After incubation, the mixture was transferred to an empty plastic column and washed with 10 CV of wash buffer (20 mM Tris-HCl pH 8, 300 mM NaCl, 30 mM imidazole). Tyrosinase was eluted in 1 mL fractions with wash buffer supplemented with 300 mM imidazole and 0.02 mM CuSO₄. The fractions containing the protein were pooled and buffer was exchanged to PBS pH 7.4 supplemented with 15 % glycerol and 0.02 mM CuSO4 using an Amicon^{®} centrifugal filter units with an appropriate MWCO (Millipore). Protein concentration was calculated from the measured A280 absorption (extinction coefficients were calculated with ProtParam (https://web.expasy.org/protparam/). Tyrosinase was flash frozen using liquid nitrogen and stored at -80 °C until further use.

### eGFP nanobody purification and yield determination

In order to determine yields of amber suppressed eGFPNb, expression and purification were performed as with other XisoK bearing proteins with a few modifications. Both *E. coli* K12 and isoK12 were grown in 25 mL 2xYT cultures manually inducing expression with 0.05% arabinose at an OD₆₀₀ of 0.6. 300 uL of Ni-NTA bead slurry was used for each purification and after loading the beads with lysate an additional high salt wash was done with wash buffer containing 1 M NaCl to remove non-specifically bound proteins. Protein was eluted in elution buffer till no more protein was detected in the flow-though, checking with Bradford's reagent. Eluted protein was buffer exchanged in 1xPBS, 5 times and concentrations determined using a NanoPhotometer^{®} NP60 (Implen). To check protein purity as well as if all the protein bound to the beads, eluted protein and lysate flow-though were analyzed via SDS-PAGE.

### Evolution of HisoKRS

Electrocompetent *E. coli* DH10**β** cells containing a positive selection plasmid pPyIT_CAT111TAG were transformed with a pBK_*Mb*PyIRS_lib (MbPyIRS library with positions A276, Y271, L274, C313 and M315 randomized using degenerate NNK primers). After recovery with 5 mL SOC for 1 hour at 37 °C. cells were added to 100 mL of 2-YT supplemented with Ampicillin (100 µg/mL) and tetracycline (17 µg/ml) and grown to an OD₆₀₀ of 0.6. Cells were then plated in a dilution series on 24 cm autoinduction agar plates containing 0.5 mM G-HisoK and incubated overnight at 37 °C. Plates with distict single colonies were scraped and the plasmids were isolated from the cell mass and the pBK MbPyIRS_lib was isolated on a 1% agarose gel. Electrocompetent *E. coli* DH10**β** cells containing a negative selection plasmid pYOBB_Barnase3TAG were then transformed with the isolated library and after recovery with 1 mL SOB for 1 h at 37 °C, cells were added to 100 ml of 2-YT supplemented with Ampicillin (100 µg/mL) and Chloramphenicol (50 µg/mL) and grown to an OD₆₀₀ of 0.6. For negative selection, cells were then plated on LB-agar plates supplemented with Ampicillin (100 µg/mL), Chloramphenicol (50 µg/mL) and 0.2% arabinose and grown overnight at 37 °C. Plates were scraped and library plasmid isolated for a final positive selection round using a pPyIT_GFP_N150TAG_CAT111TAG double reporter plasmid. Single colonies were then picked into a 96 deep well plate containing 1 mL non-inducing media/well and grown overnight at 37 °C. Two 96 deep well plates containing autoinducing media with and without 0.5 mM G-HisoK were inoculated with the overnight culture and grown for 24 h. sfGFP fluorescence was measured for both plates on a Varioscan Lux plate reader (Thermo Scientific). Clones with sufficient fluorescence above negative sample were sent for sanger sequencing.

### Generation of a OppA error prone library

An error prone library of *oppA* was generated using an error prone PCR kit (Jena Bioscience Cat no. PP-102). Primers OppA_EP_fwd and OppA_EP_rev **(Table 4)** were used to amplify *opp*A from pEVOL_*Mb*PyIRS_*opp*A according to manufacturer's instructions running for 25 cycles and adding 2.5 µL error prone solution. Resultant amplicon was purified on a 1% agarose gel and digested with *Nde*1 and *Pst*1-HF. Digested insert was then ligated with T4 ligase into a backbone generated by digesting pEVOL_*Mb*PyIRS_*opp*A with *Nde*1 and *Pst*1-HF followed by dephosphorylation with Antarctic Phosphatase. Electrocompetent DH10**β** cells were then transformed with the purified ligation. After recovery in SOC media for 1 h at 37 °C, cells were added to 50 ml of 2-YT supplemented with Chloramphenicol (50 µg/mL) and grown overnight at 37 °C. library plasmid from the overnight culture was purified and used for subsequent steps. The size of the library was determined by plating the freshly transformed cells in a dilution series on LB-agar plates supplemented with Chloramphenicol (50 µg/mL). The size was calculated to be 4 × 10⁶. Multiple single clones were sequenced to determine the average error rate and was optimized to be ~ 0.8% or an average of 5 amino acid mutations/gene.

### Generation of an OppA site saturation library

4 hotspot positions in OppA were selected for site saturation based on sequenced variants from selection of the error prone library. Positions were chosen if mutations occurred more than once (R439, S460) or if mutations occurred close to each other in different variants (D221, W222).

Sites were randomized using degenerate trimer primers (Ella Biotech). A mix of templates based on pEVOL_*Mb*PyIRS_*opp*A containing OppA wt and four variants from the error prone library were amplified with library primers (see primer table.). The linear amplicon was then purified on a 1% agarose gel and digested with *Bbs*1-HF and *Dpn*1*.* Digested fragment was circularized via ligation with T4 ligase and electrocompetent DH10**β** cells were transformed with the circularized library plasmid. After recovery in SOC media for 1 h at 37 °C, cells were added to 50 ml of 2-YT supplemented with Chloramphenicol (50 µg/mL) and grown overnight at 37 °C. Library plasmid was purified from the overnight culture and used as the template for the next round of amplification to randomize the next position(s). This process was repeated 3 times to give a library with positions D221, W222, R439 and S460 mutated to all 20 amino acids on oppA wt and 4 error prone variants. The library size was 8 × 10⁵.

### FACS based screening protocol

Electrocompetent *E. coli* K12 Δ*Opp*A cells containing sfGFP reporter plasmid pBAD_sfGFP_N150TAG_H6 were transformed with the OppA library (pEVOL *Mb*PyIRS *opp*A_EP_lib, pEVOL_*Mb*PyIRS_*opp*A_trimer_lib or pEVOL_*Mb*PyIRS_*opp*A_wt). After recovery in 1 mL SOC for 1 h at 37 °C, transformed cells were diluted in 50 mL of non-inducing media (auto-inducing media without arabinose) supplemented with Ampicillin (100 µg/mL) and Chloramphenicol (50 µg/mL) and grown overnight. The Overnight culture was then diluted in non-inducing media and grown to an OD₆₀₀ of 0.6. At this point, 0.05% arabinose was added to induce sfGFP expression and the culture was split into smaller cultures supplemented with +/- 0.5 mM G-SisoK and varying amounts of tryptone to apply a selection pressure towards OppA variants which preferably bound to G-SisoK. Cultures were then grown for 4 h at 37 °C to allow for sfGFP expression. To halt growth, the cultures were cooled on ice for 10 minutes, centrifuged (4000 × g, 5 min) and resuspended in ice cold PBS. The PBS cell suspension was sorted on a Sony cell sorter (SH800) using a 70 µM) chip sorting for cells with highest sfGFP fluorescence intensity. Gating was decided based on the positive control (oppA wt, 0.5 mM G-SisoK, 0 g/L tryptone) and the negative controls (oppA wt, 0.5 mM G-SisoK, Xg/L tryptone - with X being the tryptone concentration used in that round of enrichment, and oppA wt, 0 mM G-SisoK, 0 g/L tryptone). For each round cells with the top 0.5-2% sfGFP fluorescence were sorted. Sorted cells were recovered in SOC media supplemented with Ampicillin (50 µg/mL) and Chloramphenicol (25 µg/mL) overnight at 37 °C and the process repeated for further enrichment. After multiple rounds of enrichment, cells were sorted into a 96-well plate containing SOC with supplemented with Ampicillin (50 µg/mL) and Chloramphenicol (25 µg/mL) and grown overnight at 37 °C. Cultures grown from single cells were further evaluated via the fluorescence plate reader assay and variants which showed the desired phenotype were sequenced.

The error prone library was subjected to 5 rounds of enrichment, each time doubling the tryptone concentration from 1 g/L (round 1) to 16 g/L (round 5).

The site saturation library was directly grown in LB media (10 g/L tryptone) for 2 rounds of enrichment and 2xYT (16 g/L) for 3 rounds of enrichment.

**Table 7: Mutations in OppA variants**

| OppA variant | Mutations |
|---|---|
| Error-prone Variant 1 | T173N, **D221**G, K371E, **R439**H |
| Error-prone Variant 2 | L78I, **W222**R, K307R, K333N |
| Error-prone Variant 3 | T429A, **R439**L, **S460**C, V482A |
| Error-prone Variant 4 | V193A, I303V, N337D, **S460N** |
| Site-saturation variant OppA-ev2 | V193A, **D221L, W222**A, I303V, N337D, **R439**Q**, S460**H |

| | |
|---|---|
| In bold: positions targeted for site saturation mutagenesis | |

### Preparation of E. coli lysates for LC-MS based uptake assays

Uptake assay protocol adapted from previously published protocol (N. Huguenin-Dezot et al., Trapping biosynthetic acyl-enzyme intermediates with encoded 2,3-diaminopropionic acid. Nature 565, 112-117 (2019)). Relevant *E. coli* strains were transformed with a dummy pBAD plasmid to prevent contamination of cultures. After recovery in 1mL SOC for 1 h at 37 °C, cells were cultured in 5 ml of 2xYT or Autoinducing media supplemented with Ampicillin (100 µg/mL) and grown overnight at 37 °C. Overnight cultures were diluted to an OD₆₀₀ of 0.05 in 5 mL of either 2xYT or autoinducing media supplemented with Ampicillin (100 µg/mL) and non-canonical amino acid and grown overnight at 37 °C. The OD₆₀₀ of the overnight cultures was determined and 12 OD.mL were harvested by centrifugation at 4000 × g for 10 minutes. Cell pellets were then washed 3 times with 1 mL of cold media and resuspended in 400 µL of a methanol:water solution (60:40). Cells were lysed via 5 freeze thaw cycles in liquid nitrogen and a 42 °C water bath. Lysate was cleared by centrifugation at 17,900 × g for 20 min. 5 uL of cleared lysate was injected onto the LC-MS for analysis. For cultures incubated with BocK, samples were injected onto a Zorbax SB-C18 (Agilent, 4.6 × 150 mm) column and a gradient of 5-95% was used. For cultures incubated with XisoK and GXisoK peptides, samples were injected on a Poroshell 120 HILIC-Z (Agilent, 2.1 × 100 mm) column and using a gradient of 95-10% . The mass spectrometer was set to single ion mode to detect the relevant m/z for each non-canonical amino acid.

To determine intracellular concentrations, calibration points of lysate spiked with known non-canonical amino acid concentrations were measured. Ion peaks were integrated, and integral values plotted against concentration to determine a linear calibration line. Integral values of unknown samples interpolated on calibration line to determine lysate concentrations. Intracellular concentrations were estimated assuming 1 OD₆₀₀ = 8 × 10⁸ cells/mL and the volume of an *E. coli* cell = 0.6 fL.

### Determination of K_{D}s using microscale thermophoresis

Microscale thermophoresis (MST) was performed on the NanoTemper Monolith NT.115 (NanoTemper Technologies). Wt OppA and evolved variants were fluorescently labeled using the Monolith Protein Labeling Kit RED-NHS 2nd Generation (NanoTemper Technologies) and diluted to 100 nM in assay buffer (2xPBS, 0.02% tween 20). Peptides were diluted to double the highest measured concentration in assay buffer and diluted 2-fold in a dilution series to give 16 peptide concentrations. Equal volumes of protein and peptide solutions were mixed (final protein concentration of 50 nM) and incubated at room temperature for 30 minutes. Samples were loaded into capillaries (Monolith NT.115 Capillaries, NanoTemperTechnologies) and measured according to manufacturer's instructions. Three independent replicates were measured for each peptide-protein combination and data analysis was performed with MO.affinity Analysis (v3.0.5, NanoTemper Technologies).

### Generating isoK12 strain via homologous recombination

Primers with 50 bp overhangs homologous to regions upstream and downstream of the OppA locus in *E. coli* genome were used to amplify *oppA* ev2 from pEVOL_*Mb*PyIRS_*opp*A_ev2. **(Table 4)**

A single clone of *E. coli* K12 Δ*Opp*A cells transformed with a pSIJ8 plasmid was cultured in 2-YT supplemented with Ampicillin (50 µg/mL) and grown at 30°C, 200 rpm until an OD₆₀₀ of 0.3 followed by induction with 15 mM arabinose for the expression of lambda red recombineering genes . After incubation for 45 min at 37 °C the culture was cooled on ice to halt growth and made electrocompetent via a standard protocol. The resulting electrocompetent *E. coli* K12 Δ*Opp*A cells with expressed recombineering genes were then transformed with the linear DNA fragment encoding *oppA* ev2. After recovery in 1mL SOC for 2 h at 37 °C, cells were diluted in 2-YT and grown overnight at 37 °C for the curing of thermosensitive plasmid pSIJ8. The overnight culture (containing a mix of Δ*opp*A and knock-in cells) was diluted and grown to an OD₆₀₀ of 0.6 and made electrocompetent. These cells were then co-transformed with aaRS plasmid pEVOL_*Mb*PyIRS and sfGFP reporter pBAD_sfGFP_N150TAG_H6. Transformed cells were recovered in 1 mL SOC for 1 h at 37 °C and diluted in 2-YT supplemented with Ampicillin (100 µg/mL) and Chloramphenicol (50 µg/mL) and grown to an OD600 of 0.6 and sfGFP expression was induced with 0.05% arabinose and 0.5 mM G-SisoK was added. The culture was grown for 4 h, cooled on ice, centrifuged (4000 × g, 5 minutes), and resuspended in ice cold PBS. Cells with the highest sfGFP fluorescence were sorted as single cells into a 96 well plate containing 2-YT and grown overnight. Clones with the correct genomic insert were confirmed with sequencing of the genomic locus and whole genome sequencing. Once confirmed, plasmids were cured from the strain via electroporation.

### On bead CuAAC labeling of eGFP-NB with Picolyl-Azide-Sulfo-Cy5

eGFP-NB bearing either BocK, XisoK (Prg) or PraK (D. P. Nguyen et al., Genetic Encoding and Labeling of Aliphatic Azides and Alkynes in Recombinant Proteins via a Pyrrolysyl-tRNA Synthetase/tRNACUA Pair and Click Chemistry. Journal of the American Chemical Society 131, 8720-8721 (2009)) at position R75 was expressed and purified as described in 'Expression of XisoK bearing proteins' and 'Purification of His6 tagged proteins'. Purified proteins were buffer exchanged into PBS using Amicon^{®} centrifugal filter units (Millipore, 3 kDa MWCO), and 20 µM) of each eGFP-NB variant was bound to magnetic Ni-NTA beads (Cube biotech) . Beads were washed 2 times with PBS and then incubated with CuAAC mix containing 100 µM AF647-Picolyl-Azide (Jena Bioscience), 50 µM CuSO₄, 250 µM THPTA and 2.5 mM ascorbic acid in PBS for 30 minutes at room temperature. Beads were then washed 5 times with PBS and protein was eluted with 300 mM imidazole in PBS. Eluted protein and flow-through samples were run on an SDS-PAGE and labeling visualized via in-gel fluorescence (iBright^{™} FL1500, ThermoFisher Scientific).

### Photocrosslinking of diazirine bearing proteins in cells

Chemically competent *E. coli* K12 cells were co-transformed with pBAD_POI_His6 (either sfGFP or GST encoding a protein of interest with a C-terminal His6 tag and a TAG codon at the indicated position) and pEVOL MaPyIRS_IP (encoding two copies of *Ma*PyIRS H227I, Y228P and *Ma* tRNA_{CUA}) for incorporating pLisoK or pEVOL_DiazKRS (encoding two copies of DiazKRS (T. A. Nguyen, T. F. Gronauer, T. Nast-Kolb, S. A. Sieber, K. Lang, Substrate Profiling of Mitochondrial Caseinolytic Protease P via a Site-Specific Photocrosslinking Approach. Angew Chem Int Ed Engl 61, e202111085 (2022)) and *Mm* tRNA_{CUA}). After recovery in 1 mL SOC for 1 h at 37 °C, cells were added to 5 mL of 2-YT supplemented with Ampicillin (100 µg/mL) and Chloramphenicol (50 µg/mL) and grown overnight at 37 °C. Autoinducing media supplemented with Ampicillin (100 µg/mL) and Chloramphenicol (50 µg/mL) and containing either no non-canonical amino acid, 2mM BocK or 2mM G-pLisoK were inoculated with the overnight culture and grown at 37 °C overnight. The resulting culture was diluted to an OD₆₀₀ of 1 and +UV samples were irradiated with UV light (15 W, 365 nm) using a lamp (Vilber, VL-215.L) for 15 minutes. +UV and -UV samples were run on an SDS-PAGE and analyzed via western blot using an anti-His peroxidase-coupled antibody (Sigma).

### Tyrosinase-mediated labelling of PisoK bearing proteins

20 µM of protein (either Ub wt, UbK63PisoK or 3C-UbK63PisoK) was incubated with 160 µM p-cresol and 0.4 µM tyrosinase in tyrosinase buffer (20 mM potassium phosphate buffer pH 6.5, 100 mM NaCl) and incubated at room temperature for 2 hours. Reaction was quenched with 1 mM TCEP and 1 mM BCN-OH (Synaffix) for 10 minutes. Cleavage of the 3C-UbK63PisoK construct N-terminus was done with HRV 3C-protease (ThermoFischer) to obtain single-labeled protein.

### Chemical crosslinking of protein-protein complexes using CIAisoK in living E. coli

### Affibody-ProteinZ:

Chemically competent *E. coli* K12 cells were co-transformed with pBAD_Affibody_D36TAG_His6, pBAD_RSF1035_Strep_SUMO_ProteinZ_N7C and pEVOL_MbPylRS_C313V (encoding two copies of *Mb* PylRS with a C313V mutation and *Mm* tRNA_{CUA}) or pEVOL_wt_*Mb*PylRS. After recovery in 1 mL SOC for 1 h at 37 °C, cells were added to 5 mL of 2-YT supplemented with Ampicillin (100 µg/mL), Chloramphenicol (50 µg/mL) and Kanamycin (50 µg/mL) and grown overnight at 37 °C, 200 rpm. Autoinducing media (2 mL cultures in a 24-well plate) (M. Muzika et al., Chemically-defined lactose-based autoinduction medium for site-specific incorporation of non-canonical amino acids into proteins. RSC Adv 8, 25558-25567 (2018)) supplemented with Ampicillin (100 µg/mL), Chloramphenicol (50 µg/mL) and Kanamycin (50 µg/mL) and containing either no non-canonical amino acid, 2 mM G-CIAisoK or 2 mM G-AisoK were inoculated with the overnight culture and grown at 37 °C, 200 rpm overnight.

After overnight incubation, OD₆₀₀ was measured, samples were normalized and subjected to SDS-PAGE followed by either Coomassie staining or western blot analysis using an anti-His peroxidase-coupled antibody (Sigma) or antiStrep-HRP (StrepMAB-Classic HRP, IBA, Cat.No. 2-1509-001).

### sfGFP dimer:

Chemically competent *E. coli* K12 cells were co-transformed with pBAD_sfGFP_N150TAG_His6 (and mutants E173C, E173H, E173A or V207K) and pEVOL_*Mb*PyIRS_C313V (encoding two copies of *Mb* PylRS with a C313V mutation and Mm tRNA_{CUA}) or pEVOL wt_MbPyIRS. Expression and downstream analysis were performed analogously to the above-described procedure for Affibody-ProteinZ.

### Rab1b-DrrA

Chemically competent *E. coli* K12 cells were co-transformed with pBAD_Rablb_R79TAG_His6_RBS_Strep_TEV_DrrA(339-522)_D512C (encoding both target genes in a polycistronical manner) and pEVOL_*Mb*PyIRS_C313V (encoding two copies of *Mb* PylRS with a C313V mutation and Mm IRNA_{CUA}) or pEVOL_wt_*Mb*PyIRS. Expression and downstream analysis were performed analogously to the above-described procedure for Affibody-ProteinZ. Ni-NTA purification was performed as described in "Purification of His6 tagged proteins".

### Chemical crosslinking of Affibody and ProteinZ in vitro

Affibody bearing either AisoK or ClAisoK at position 36 and a SUMO-ProteinZ N7C mutant were expressed and purified as described in sections "Expression of XisoK bearing proteins" and "Purification of His6 tagged proteins". For in vitro crosslinking assays Affibody variants and the SUMO-ProteinZ N7C mutant were diluted to 50 µM each into PBS pH 7.4 supplemented with 1 mM TCEP and incubated at 37 °C. At denoted time points, 2 µL samples were taken and quenched by the addition of 8 µL 4× SDS loading buffer. After boiling at 95 °C for 10 min, the samples were loaded on SDS-PAGE and visualized by Coomassie staining.

### Determination of doubling times of isoK12 and K12 in Al and 2-YT media

Single colonies of *E. coli* K12 and GXK were cultured in 20 ml of 2-YT and grown to an OD₆₀₀ of 0.6 at 37 °C, 200 rpm. These culture were then diluted to an OD₆₀₀ of 0.05 in pre-warmed 2-YT or autoinduction media and cultured at 37 °C, 200 rpm while monitoring OD₆₀₀ at regular intervals. To determine doubling times the slope of time (minutes) vs log2(OD₆₀₀) at the exponential phase was calculated. Analysis was done using GraphPad Prism Version 10).

### Platereader based sfGFP fluorescence measurements

sfGFP fluorescence time courses of *E. coli* cultures were measured on a Tecan Spark^{®} Multimode Microplate Reader with a humidity cassette in order to prevent evaporation. E. *coli* cultures containing the appropriate plasmids were diluted to an OD₆₀₀ of 0.05 in the relevant media and grown to an OD₆₀₀ of 0.6 and sfGFP expression was induced with 0.05% arabinose and non-natural amino acid added to the media. 200 uL of this culture was added to the well of a 96-well microplate with a clear bottom (Greiner Bio-One µClear^{™} Cat.no. 655096) and the plate sealed with a breathe-EASIERä (Diversified Biotech) membrane. sfGFP fluorescence was measured from the bottom every 10 minutes while shaking in-between reads at 37 °C.

### Dual stop codon suppression for incorporation fo AcK and pLisoK into proteins

Chemically competent *E. Coli* K12 cells were co-transformed with pBAD_POI (either pBAD_sfGFP_N40TAA_N150TAG_H6 or
pBAD_Ub_K48TAA_TEV_SUMO2_K11TAG_H6) with a C-terminal His6 tag) and pEVOL AcKRS3(TAA)_RBS_MaPyIRS_IP(TAG) (encodes AcKRS3 and MaPyIRS_IP polycistronically and their respective tRNAs). After recovery in 1 mL SOC media for 1 h at 37 °C, cells were cultured in 5 ml of 2-YT supplemented with Ampicillin (100 µg/mL) and Chloramphenicol (50 µg/mL) and incubated overnight at 37 °C, 200 rpm. The overnight culture was then diluted to an OD₆₀₀ of 0.05 in autoinducing media supplemented with Ampicillin (100 µg/mL), Chloramphenicol (50 µg/mL) and respective ncAA and/or peptide. Cells were grown overnight at 37 °C and the overnight culture was harvested by centrifugation at 4000 x g for 10 minutes and the pellets were stored at -20 °C till further use.

Ub-K48pLisoK-TEV-SUMO2-K11AcK-H6 was purified as described in 'Purification of His6 tagged proteins'. For cleavage, Ub-K48pLisoK-TEV-SUMO2-K11AcK-H was diluted into buffer (PBS with 3 mM DTT) and incubated with TEV protease (0.1 mg/ml) for 30 minutes at RT.

### References

1. L. S. Icking et al., iNClusive: a database collecting useful information on non-canonical amino acids and their incorporation into proteins for easier genetic code expansion implementation. Nucleic Acids Res 52, D476-D482 (2024).
2. H. B. Yi et al., Cellular and Biophysical Applications of Genetic Code Expansion. Chemical Reviews 124, 7465-7530 (2024).
3. N. G. Koch, N. Budisa, Evolution of Pyrrolysyl-tRNA Synthetase: From Methanogenesis to Genetic Code Expansion. Chemical Reviews, (2024).
4. T. Umehara et al., N-acetyl lysyl-tRNA synthetases evolved by a CcdB-based selection possess N-acetyl lysine specificity in vitro and in vivo. FEBS Lett 586, 729-733 (2012).
5. P. O'Donoghue, J. Ling, Y. S. Wang, D. Soll, Upgrading protein synthesis for synthetic biology. Not Chem Biol 9, 594-598 (2013).
6. T. S. Young, I. Ahmad, J. A. Yin, P. G. Schultz, An enhanced system for unnatural amino acid mutagenesis in E. coli. J Mol Biol 395, 361-374 (2010).
7. Y. Ryu, P. G. Schultz, Efficient incorporation of unnatural amino acids into proteins in Escherichia coli. Not Methods 3, 263-265 (2006).
8. D. Jewel et al., Virus-assisted directed evolution of enhanced suppressor tRNAs in mammalian cells. Not Methods 20, 95-103 (2023).
9. J. Guo, C. E. Melancon, 3rd, H. S. Lee, D. Groff, P. G. Schultz, Evolution of amber suppressor tRNAs for efficient bacterial production of proteins containing nonnatural amino acids. Angew Chem Int Ed Engl 48, 9148-9151 (2009).
10. D. I. Bryson et al., Continuous directed evolution of aminoacyl-tRNA synthetases. Not Chem Biol 13, 1253-1260 (2017).
11. K. Wang, H. Neumann, S. Y. Peak-Chew, J. W. Chin, Evolved orthogonal ribosomes enhance the efficiency of synthetic genetic code expansion. Nat Biotechnol 25, 770-777 (2007).
12. H. Neumann, K. Wang, L. Davis, M. Garcia-Alai, J. W. Chin, Encoding multiple unnatural amino acids via evolution of a quadruplet-decoding ribosome. Nature 464, 441-444 (2010).
13. T. Mukai et al., Highly reproductive Escherichia coli cells with no specific assignment to the UAG codon. Sci Rep 5, 9699 (2015).
14. D. B. Johnson et al., RF1 knockout allows ribosomal incorporation of unnatural amino acids at multiple sites. Not Chem Biol 7, 779-786 (2011).
15. M. J. Lajoie et al., Genomically recoded organisms expand biological functions. Science 342, 357-360 (2013).
16. J. Fredens et al., Total synthesis of Escherichia coli with a recoded genome. Nature 569, 514-518 (2019).
17. W. E. Robertson et al., Sense codon reassignment enables viral resistance and encoded polymer synthesis. Science 372, 1057-1062 (2021).
18. M. S. Zhang et al., Biosynthesis and genetic encoding of phosphothreonine through parallel selection and deep sequencing. Not Methods 14, 729-736 (2017).
19. J. Tai et al., Pyrrolysine-Inspired in Cellulo Synthesis of an Unnatural Amino Acid for Facile Macrocyclization of Proteins. J Am Chem Soc 145, 10249-10258 (2023).
20. J. A. Marchand et al., Discovery of a pathway for terminal-alkyne amino acid biosynthesis. Nature 567, 420-424 (2019).
21. Y. Chen et al., Unleashing the potential of noncanonical amino acid biosynthesis to create cells with precision tyrosine sulfation. Not Commun 13, 5434 (2022).
22. R. A. Mehl et al., Generation of a bacterium with a 21 amino acid genetic code. J Am Chem Soc 125, 935-939 (2003).
23. P. Zhu et al., PermaPhos (Ser) : autonomous synthesis of functional, permanently phosphorylated proteins. bioRxiv, (2021).
24. W. Ko, R. Kumar, S. Kim, H. S. Lee, Construction of Bacterial Cells with an Active Transport System for Unnatural Amino Acids. ACS Synth Biol 8, 1195-1203 (2019).
25. E. Rodriguez-Robles et al., Rational design of a bacterial import system for new-to-nature molecules. Metabolic Engineering 85, 26-34 (2024).
26. T. E. Fickel, C. Gilvarg, Transport of impermeant substances in E. coli by way of oligopeptide permease. Not New Biol 241, 161-163 (1973).
27. B. N. Ames, G. F. Ames, J. D. Young, D. Tsuchiya, J. Lecocq, Illicit transport: the oligopeptide permease. Proc Natl Acad Sci U S A 70, 456-458 (1973).
28. X. Luo et al., Genetically encoding phosphotyrosine and its nonhydrolyzable analog in bacteria. Not Chem Biol 13, 845-849 (2017).
29. A. Moussatova, C. Kandt, M. L. O'Mara, D. P. Tieleman, ATP-binding cassette transporters in Escherichia coli. Biochim Biophys Acta 1778, 1757-1771 (2008).
30. M. Fottner et al., Site-specific ubiquitylation and SUMOylation using genetic-code expansion and sortase. Not Chem Biol 15, 276-284 (2019).
31. M. Fottner et al., Site-Specific Protein Labeling and Generation of Defined Ubiquitin-Protein Conjugates Using an Asparaginyl Endopeptidase. Journal of the American Chemical Society 144, 13118-13126 (2022).
32. M. Fottner et al., A modular toolbox to generate complex polymeric ubiquitin architectures using orthogonal sortase enzymes. Not Commun 12, 6515 (2021).
33. N. Huguenin-Dezot et al., Trapping biosynthetic acyl-enzyme intermediates with encoded 2,3-diaminopropionic acid. Nature 565, 112-117 (2019).
34. J. Vergalli et al., Porins and small-molecule translocation across the outer membrane of Gram-negative bacteria. Not Rev Microbiol 18, 164-176 (2020).
35. A. L. Davidson, J. Chen, ATP-binding cassette transporters in bacteria. Annu Rev Biochem 73, 241-268 (2004).
36. T. Baba et al., Construction of Escherichia coli K-12 in-frame, single-gene knockout mutants: the Keio collection. Mol Syst Biol 2, 2006 0008 (2006).
37. C. Thomas, R. Tampe, Structural and Mechanistic Principles of ABC Transporters. Annu Rev Biochem 89, 605-636 (2020).
38. M. M. Klepsch et al., Escherichia coli peptide binding protein OppA has a preference for positively charged peptides. J Mol Biol 414, 75-85 (2011).
39. C. G. Miller, Peptidases and proteases of Escherichia coli and Salmonella typhimurium. Annu Rev Microbiol 29, 485-504 (1975).
40. J. Jumper et al., Highly accurate protein structure prediction with AlphaFold. Nature 596, 583-589 (2021).
41. J. Zang et al., Genetic code expansion reveals aminoacylated lysine ubiquitination mediated by UBE2W. Not Struct Mol Biol 30, 62-71 (2023).
42. X. D. He et al., Sensing and Transmitting Intracellular Amino Acid Signals through Reversible Lysine Aminoacylations. Cell Metab 27, 151-166 e156 (2018).
43. A. Gran-Scheuch, E. Bonandi, I. Drienovská, Expanding the Genetic Code: Incorporation of Functional Secondary Amines via Stop Codon Suppression. ChemCatChem 16, e202301004 (2024).
44. R. L. Brabham et al., Rapid sodium periodate cleavage of an unnatural amino acid enables unmasking of a highly reactive alpha-oxo aldehyde for protein bioconjugation. Org Biomol Chem 18, 4000-4003 (2020).
45. M. M. Lee et al., Pyrrolysine-inspired protein cyclization. Chembiochem 15, 1769-1772 (2014).
46. X. Li, T. Fekner, J. J. Ottesen, M. K. Chan, A pyrrolysine analogue for site-specific protein ubiquitination. Angew Chem Int Ed Engl 48, 9184-9187 (2009).
47. H. Zhou et al., Linkage-Specific Synthesis of Di-ubiquitin Probes Enabled by the Incorporation of Unnatural Amino Acid ThzK. Chembiochem 23, e202200133 (2022).
48. C. B. Rosen, M. B. Francis, Targeting the N terminus for site-selective protein modification. Not Chem Biol 13, 697-705 (2017).
49. J. C. Maza et al., Enzymatic Modification of N-Terminal Proline Residues Using Phenol Derivatives. J Am Chem Soc 141, 3885-3892 (2019).
50. A. P. Green, T. Hayashi, P. R. E. Mittl, D. Hilvert, A Chemically Programmed Proximal Ligand Enhances the Catalytic Properties of a Heme Enzyme. Journal of the American Chemical Society 138, 11344-11352 (2016).
51. H. Huang et al., Genetically encoded Ndelta-vinyl histidine for the evolution of enzyme catalytic center. Not Commun 15, 5714 (2024).
52. K. Lang, J. W. Chin, Cellular incorporation of unnatural amino acids and bioorthogonal labeling of proteins. Chem Rev 114, 4764-4806 (2014).
53. J. W. Chin, A. B. Martin, D. S. King, L. Wang, P. G. Schultz, Addition of a photocrosslinking amino acid to the genetic code of Escherichiacoli. Proc Natl Acad Sci USA 99, 11020-11024 (2002).
54. T. A. Nguyen, M. Cigler, K. Lang, Expanding the Genetic Code to Study Protein-Protein Interactions. Angew Chem Int Ed Engl 57, 14350-14361 (2018).
55. M. Cigler et al., Proximity-Triggered Covalent Stabilization of Low-Affinity Protein Complexes In Vitro and In Vivo. Angew Chem Int Ed Engl 56, 15737-15741 (2017).
56. M. Hogbom, M. Eklund, P. A. Nygren, P. Nordlund, Structural basis for recognition by an in vitro evolved affibody. Proc Natl Acad Sci USA 100, 3191-3196 (2003).
57. J. Du et al., Rab1-AMPylation by Legionella DrrA is allosterically activated by Rab1. Not Commun 12, 460 (2021).
58. M. Muzika et al., Chemically-defined lactose-based autoinduction medium for site-specific incorporation of non-canonical amino acids into proteins. RSCAdv 8, 25558-25567 (2018).
59. Y. Zheng, M. J. Gilgenast, S. Hauc, A. Chatterjee, Capturing Post-Translational Modification-Triggered Protein-Protein Interactions Using Dual Noncanonical Amino Acid Mutagenesis. ACS Chem Biol 13, 1137-1141 (2018).
60. H. Neumann et al., A method for genetically installing site-specific acetylation in recombinant histones defines the effects of H3 K56 acetylation. Mol Cell 36, 153-163 (2009).
61. J. C. W. Willis, J. W. Chin, Mutually orthogonal pyrrolysyl-tRNA synthetase/tRNA pairs. Not Chem 10, 831-837 (2018).
62. K. Lang et al., Genetic Encoding of bicyclononynes and trans-cyclooctenes for site-specific protein labeling in vitro and in live mammalian cells via rapid fluorogenic Diels-Alder reactions. J Am Chem Soc 134, 10317-10320 (2012).
63. H. S. Jang, S. Jana, R. J. Blizzard, J. C. Meeuwsen, R. A. Mehl, Access to Faster Eukaryotic Cell Labeling with Encoded Tetrazine Amino Acids. J Am Chem Soc 142, 7245-7249 (2020).

## Claims

1. A compound comprising an isopeptide-linked lysine or an analogue thereof, the compound being a compound of formula (I): 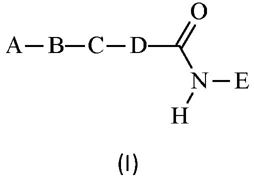 or a salt thereof, wherein:
A is selected from -NH₂, -OH, -SH, -NH(C₁₋₅ alkyl), -N(C₁₋₅ alkyl)(C₁₋₅ alkyl), -N⁺(C₁₋₅ alkyl)(C₁₋₅ alkyl)(C₁₋₅ alkyl),-NH-CO-(C₁₋₅ alkyl), -COOH, -SO₃H, -SO₂H, -N₃, and -NO₂, or A is a peptidyl group;
B is selected from: 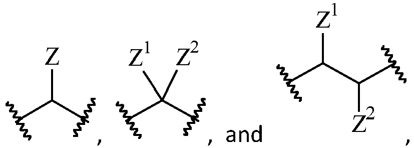 wherein the left empty valence is connected to A and the right empty valence is connected to C, wherein:
Z is hydrogen or a side chain of an amino acid, in particular wherein Z is selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{Z}, -(C₁₋₆ alkylene)-S-R^{Z}, -(C₁₋₆ alkylene)-N(R^{Z})-R^{ZZ}, -(C₁₋₆ alkylene)-CO-R^{Z}, -(C₁₋₆ alkylene)-COO-R^{Z}, -(C₁₋₆ alkylene)-O-CO-(C₁₋₆ alkyl), -(C₁₋₆ alkylene)-CO-N(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CO-N(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z groups are each optionally substituted with one or more groups selected from -OH, -SH and Hal, further wherein one -CH₂- group in said alkyl may be replaced with 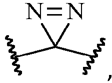 and further wherein each R^{Z} is independently selected from hydrogen and C₁₋₆ alkyl and wherein R^{ZZ} is selected from hydrogen, C₁₋₆ alkyl, -CHO, -CO(C₁₋₅ alkyl), -CO(C₁₋₅ alkenyl), -CO(C₁₋₅ alkynyl), -CO(C₁₋₅ alkylene)-COOH, -CO(C₁₋₅ alkenylene)-COOH, -CO(Co-s alkylene)-carbocyclyl, -CO(Co-s alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S},
Z¹ and Z² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{Z}, -(C₁₋₆ alkylene)-S-R^{Z}, -(C₁₋₆ alkylene)-N(R^{Z})-R^{ZZ}, -(C₁₋₆ alkylene)-COR^{Z}, -(C₁₋₆ alkylene)-COO-R^{Z}, -(C₁₋₆ alkylene)-O-CO-(C₁₋₆ alkyl), -(C₁₋₆ alkylene)-CON(R^{Z})-R^{Z}, -(C₁₋₆ alkylene)-N(R^{Z})-CO-(C₁₋₆ alkyl), -(C₁₋₆ alkylene)-CO-N(R^{Z})-O-R^{Z}, -(C₁₋₆ alkylene)-O-CO-N(R^{Z})-R^{Z}, -(C₁₋₆ alkylene)-N(R^{Z})-CO-N(R^{Z})-R^{Z}, -(C₁₋₆ alkylene)-N(R^{Z})-C(=N-R^{Z})-N(R^{Z})-R^{Z}, -(C₁₋₆ alkylene)-SO₃-R^{Z}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned Z¹ and Z² groups are each optionally substituted with one or more -OH, -SH or Hal, wherein each R^{Z} is independently selected from hydrogen and C₁₋₆ alkyl, and R^{ZZ} is selected from hydrogen, C₁₋₆ alkyl, -CHO, -CO(C₁₋₅ alkyl), -CO(C₁₋₅ alkenyl), -CO(C₁₋₅ alkynyl), -CO(C₁₋₅ alkylene)-COOH, -CO(C₁₋₅ alkenylene)-COOH, -CO(Co-s alkylene)-carbocyclyl, -CO(C₀₋₅ alkylene)-heterocyclyl, -CO-O-(C₁₋₅ alkyl), -CO-O-(C₁₋₅ alkenyl), -CO-O-(C₁₋₅ alkynyl), -CO-O-(C₀₋₅ alkylene)-carbocyclyl, -CO-O-(C₀₋₅ alkylene)-heterocyclyl, wherein said carbocyclyl and said heterocyclyl are each optionally substituted with one or more groups selected from R^{S}, and further wherein one - CH₂-group in said alkyl may be replaced with 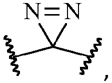
provided that at least one of Z¹ and Z² is not hydrogen, further provided that if Z¹ and Z² are connected to the same carbon atom, then both Z¹ and Z² are not hydrogen,
or Z¹ and Z² are joined together to form, together with carbon atom(s) that otherwise carry Z¹ and Z², a non-aromatic carbocyclic ring or non-aromatic heterocyclic ring, wherein said non-aromatic carbocyclic ring and said non-aromatic heterocyclic ring are each optionally substituted with one or more R^{S}; -C-D- are defined as follows:
C is selected from -CO-NH-, -CO-O-, -CO-S- and -CO-N(C₁₋₅ alkyl)-, wherein the left empty valence is connected to B and the right empty valence is connected to D, and
D is selected from 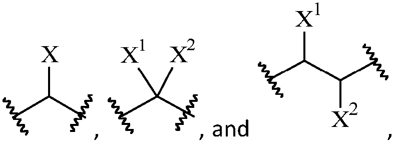
wherein the left empty valence is connected to C and the right empty valence is connected to -CO-NH-, wherein:
X is a hydrogen or a side chain of an amino acid, in particular wherein X is selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{X}, -(C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(C₁₋₆ alkyl), -(C₁₋₆ alkylene)-CO-N(R^{X})-R^{X}, -(C₁₋₆ alkylene)-N(R^{X})-CO-(C₁₋₆ alkyl), -(C₁₋₆ alkylene)-CO-N(R^{X})-O-R^{X}, -(C₁₋₆ alkylene)-O-CO-N(R^{X})-R^{X}, -(C₁₋₆ alkylene)-N(R^{X})-CO-N(R^{X})-R^{X}, -(C₁₋₆ alkylene)-N(R^{X})-C(=N-R^{X})-N(R^{X})-R^{X}, -(C₁₋₆ alkylene)-SO₃-R^{x}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X groups are each optionally substituted with one or more -OH, -SH or -Hal, wherein each R^{X} is independently selected from hydrogen and C₁₋₆ alkyl, and further wherein
one -CH₂- group in said alkyl may be replaced with 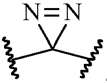
X¹ and X² are each independently selected from hydrogen, C₁₋₈ alkyl, C₂₋₈ alkenyl, C₂₋₈ alkynyl, -(C₁₋₆ alkylene)-N₃, -(C₁₋₆ alkylene)-CN, -(C₁₋₆ alkylene)-Hal, -(C₁₋₆ alkylene)-O-R^{X}, -(C₁₋₆ alkylene)-S-R^{X}, -(C₁₋₆ alkylene)-N(R^{X})-R^{X}, -(C₁₋₆ alkylene)-CO-R^{X}, -(C₁₋₆ alkylene)-COO-R^{X}, -(C₁₋₆ alkylene)-O-CO-(C₁₋₆ alkyl), - (C₁₋₆ alkylene)-CO-N(R^{X})-R^{X}, -(C₁₋₆ alkylene)-N(R^{X})-CO-(C₁₋₆ alkyl), -(C₁₋₆ alkylene)-CO-N(R^{X})-O-R^{X}, -(C₁₋₆ alkylene)-O-CO-N(R^{X})-R^{X}, -(C₁₋₆ alkylene)-N(R^{X})-CO-N(R^{X})- R^{X}, -(C₁₋₆ alkylene)-N(R^{X})-C(=N-R^{X})-N(R^{X})-R^{X}, -(C₁₋₆ alkylene)-SO₃-R^{X}, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R^{S}, wherein said alkyl, said alkenyl, said alkynyl, and any alkylene group comprised in any of the aforementioned X¹ and X² groups are each optionally substituted with one or more -OH, -SH or Hal (preferably with one or more -OH), wherein each R^{X} is independently selected from hydrogen and C₁₋₆ alkyl, further wherein one -CH₂- group in said alkyl may be replaced with 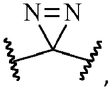 provided that at least one of X¹ and X² is not hydrogen, further provided that if X¹ and X² are connected to the same carbon atom, then both X¹ and X² are not hydrogen, or X¹ and X² are joined together to form, together with carbon atom(s) that otherwise carry X¹ and X², a non-aromatic carbocyclic ring or non-aromatic heterocyclic ring, wherein said non-aromatic carbocyclic ring and said non-aromatic heterocyclic ring are each optionally substituted with one or more R^{S};
or -C-D- taken together is -CO-(N-heterocycloalkylene)-, wherein said N-heterocycloalkylene is connected to said -CO- group of -C-D- through its nitrogen atom and wherein said N-heterocycloalkylene is optionally substituted with one or more R^{S};
E is -(C₁₋₆ alkylene)-CHY¹Y², wherein Y¹ is selected from hydrogen, -SH, -OH and -NH₂, and wherein Y² is hydrogen or COOH, provided that at least one of Y¹ and Y² is not hydrogen, further wherein said alkylene is optionally substituted with an -OH group; and
each R^{S} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-NOz, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-CO-NH(C₁₋₅ alkylene)-CN, -(C₀₋₃ alkylene)-CO-N(C₁₋₅ alkyl)(C₁₋₅ alkylene)-CN, -(C₀₋₃ alkylene)-NH-CO-(C₁₋₅ alkylene)-CN, -(C₀₋₃ alkylene)-N(C₁₋₅ alkyl)-CO-(C₁₋₅ alkylene)-CN, -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SOz-NHz, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO₂-(C₁₋₅ alkyl), -(C₀₋₃ alkylene)-SO-(C₁₋₅ alkyl), -OSO₂F, -B(OH)₂, -PO₄H, -PO₃H, - SO₄H, -SO₃H, -L-(C₀₋₃ alkylene)-carbocyclyl, and -L-(C₀₋₃ alkylene)-heterocyclyl, wherein L is selected from -(C₀₋₃ alkylene)-NH-, -(C₀₋₃ alkylene)-O-, -(C₀₋₃ alkylene)-CO-, -(C₀₋₃ alkylene)-NH-CO-, -(C₀₋₃ alkylene)-CO-NH-, -(C₀₋₃ alkylene)-NH-CO-O-, -(C₀₋₃ alkylene)-O-CO-NH-, -(C₀₋₃ alkylene)-NH-CO-NH- and -(C₀₋₃ alkylene)-NH-CO-NH-, and wherein the carbocyclyl moiety in said -L-(C₀₋₃ alkylene)-carbocyclyl and the heterocyclyl moiety in said -L-(C₀₋₃ alkylene)-heterocyclyl are each optionally substituted with one or more groups independently selected from C₁₋₄ alkyl, halogen, -CN, -NOz, -OH, -O-(C₁₋₄ alkyl), - SH, -S-(C₁₋₄ alkyl), -NH₂, -NH(C₁₋₄ alkyl), -N(C₁₋₄ alkyl)(C₁₋₄ alkyl), -COOH, -COO(C₁₋₄ alkyl), -CONH₂, -CONH(C₁₋₄ alkyl), -CON(C₁₋₄ alkyl)(C₁₋₄ alkyl), -NHCO(C₁₋₄ alkyl) and -N(C₁₋₄ alkyl)-CO(C₁₋₄ alkyl).

2. The compound of claim 1, wherein A is selected from -NH₂, -OH, -SH, -NH(C₁₋₅ alkyl), - N(C₁₋₅ alkyl)(C₁₋₅ alkyl), -NH-CO-(C₁₋₅ alkyl), -COOH, -SO₃H, -SOzH, -N₃, and -NO₂, preferably wherein A is selected from -NH₂, -OH, -SH, -NH(C₁₋₅ alkyl), -N(C₁₋₅ alkyl)(C₁₋₅ alkyl), and -N₃,
more preferably wherein A is -NH₂,
and/or
wherein B is 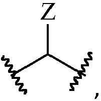 preferably wherein B is 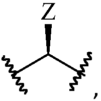 wherein the left empty valence is connected to A and the right empty valence is connected to C,
preferably wherein Z is selected from hydrogen, -(C₁₋₆ alkylene)-N(R^{Z})-R^{ZZ}, and -(C₁₋₆ alkylene)-N(R^{Z})-CO-(C₁₋₆ alkyl), more preferably wherein Z is selected from hydrogen, - CH₂CH₂CH₂CH₂-NH₂ and -CH₂CH₂CH₂CH₂-NH-CO-CH₃, even more preferably wherein Z is hydrogen,
and/or
wherein C is selected from -CO-NH- and -CO-O-, wherein the left empty valence is connected to B and the right empty valence is connected to D,
preferably wherein C is -CO-NH-, wherein the left empty valence is connected to B and the right empty valence is connected to D,
and
wherein D is 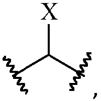 wherein the left empty valence is connected to C and the right empty valence is connected to -CO-NH- group,
preferably wherein X is selected from C₁₋₈ alkyl, C₂₋₈ alkenyl, C₂₋₈ alkynyl, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-N₃, -(C₁₋₆ alkylene)-Cl, -(C₁₋₆ alkylene)-NH₂, -(C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R^{S}, wherein said alkyl, said alkenyl, said alkynyl, and any alkylene group in any of the aforementioned groups are each optionally substituted with one or more -OH, and further wherein one -CH₂- group in said alkyl may be replaced with 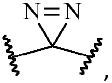
more preferably wherein X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl,
or
wherein -C-D- is a moiety according to formula 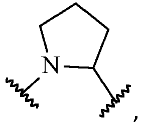
wherein the left empty valence is connected to B, and the right empty valence is connected to -CO-NH- group,
and/or
wherein E is -CH₂CH₂CH₂CH₂-CH(-NH₂)-COOH.

3. The compound of claim 1 or 2, wherein the compound is a compound according to formula (II): 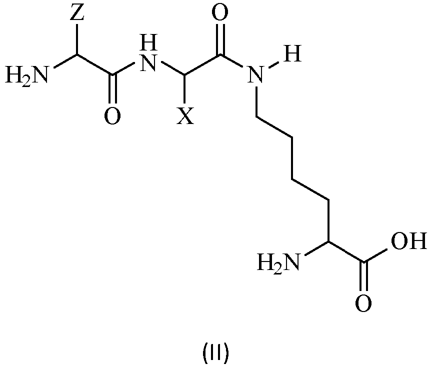
or its salt, preferably wherein the compound of formula (II) is preferably a compound of formula (IIb): 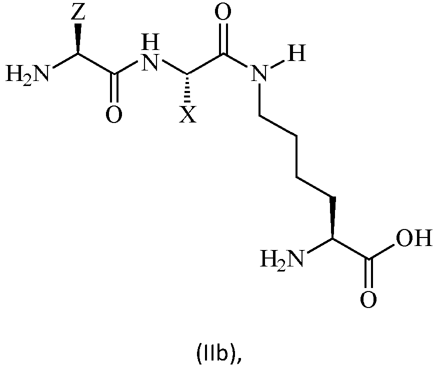
or its salt, wherein Z and X are as defined in claim 1 or 2,
preferably wherein Z is selected from hydrogen, -(C₁₋₆ alkylene)-N(R^{Z})-R^{ZZ}, and -(C₁₋₆ alkylene)-N(R^{Z})-CO-(C₁₋₆ alkyl), more preferably wherein Z is selected from hydrogen, - CH₂CH₂CH₂CH₂-NH₂ and -CH₂CH₂CH₂CH₂-NH-CO-CH₃, even more preferably wherein Z is hydrogen,
preferably wherein X is selected from methyl, ethyl, isopropyl, sec-butyl, isobutyl, hydroxymethyl, 2-hydroxypropyl, thiomethyl, propargyl, chloromethyl, (3-methyl-diazirin-3-yl)methyl, azidomethyl, aminomethyl, (imidazol-4-yl)methyl and benzyl;
or wherein the compound is a compound of formula (III): 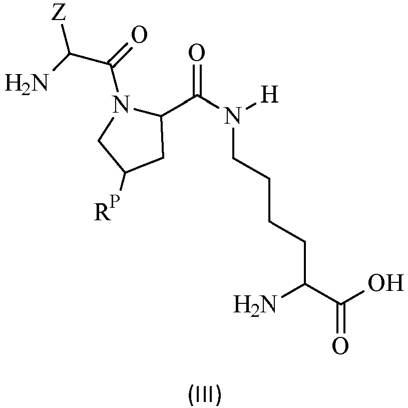
or its salt, preferably wherein the compound of formula (III) is a compound of formula (Illa): 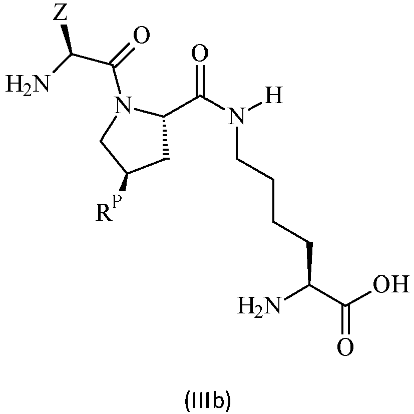
or its salt, wherein R^{P} is hydrogen or -OH, and wherein Z is as defined in any one of claims 1 to 14, preferably wherein is selected from hydrogen, -(C₁₋₆ alkylene)-N(R^{Z})-R^{ZZ}, and -(C₁₋₆ alkylene)-N(R^{Z})-CO-(C₁₋₆ alkyl), more preferably wherein Z is selected from hydrogen, -CH₂CH₂CH₂CH₂-NH₂ and -CH₂CH₂CH₂CH₂-NH-CO-CH₃, even more preferably wherein Z is hydrogen.

4. A method for identifying an engineered peptide-binding protein that promotes uptake of the compound according to any one of claims 1 to 3, the method comprising the steps of:
a) providing a nucleic acid encoding a peptide-binding protein comprising one or more mutations;
b) expressing the nucleic acid of step (a) in a cell comprising an orthogonal translation system, wherein the orthogonal translation system comprises an orthogonal aminoacyl-tRNA synthetase/tRNA pair specific for an isopeptide-linked lysine or an analog thereof, and a reporter protein gene comprising one or more codons that have been re-allocated for the incorporation of the isopeptide-linked lysine or the analog thereof by the orthogonal aminoacyl-tRNA synthetase/tRNA pair;
c) contacting the cell of step (b) with a compound according to any one of claims 1 to 3, wherein the compound is transported into the cell and hydrolyzed inside the cell to release the isopeptide-linked lysine or an analog thereof;
d) detecting the expression of the reporter protein gene by the cell, wherein the expression is dependent on the successful uptake of the compound in step (c); and
e) identifying the engineered peptide-binding protein that promotes uptake of the compound according to any one of claims 1 to 3 based on the expression of the reporter protein gene detected in step (d).

5. The method according to claim 4, wherein the peptide-binding protein is the peptide-binding protein of an oligopeptide permease (OppA), in particular wherein the peptide-binding protein is the peptide-binding protein of the E. coli oligopeptide permease (OppA; SEQ ID NO:1); in particular wherein in step (a) one or more mutations are introduced at positions L78, T173, V193, D221, W222, I303, K307, K333, N337, K371, R439, T429, S460, and/or V482 of SEQ ID NO:1.

6. The method according to any one of claims 4 or 5, wherein the mutations introduced in step (a) are introduced using error-prone PCR and/or saturated mutagenesis and/or continuous directed evolution, and/or
wherein the cell in step (b) is a prokaryotic cell, in particular an E. coli cell; and/or
wherein the orthogonal aminoacyl-tRNA synthetase/tRNA pair is a Pyrrolysine-tRNA synthetase/tRNA pair; and/or
wherein the reporter protein gene in step (b) comprises one or more amber stop codons that have been re-allocated to incorporate the isopeptide-linked lysine or the analog thereof; and/or
wherein in step (c), the cell is contacted with a compound according to any one of claims 1 to 3 in the presence of a peptide that competes for binding to the peptide-binding protein; and/or
wherein the reporter protein is a fluorescent protein, in particular wherein the detection of the expression of the reporter protein gene in step (d) is performed using fluorescence-activated cell sorting (FACS); or wherein the detection of the expression of the reporter protein gene in step (d) is performed using antibiotic resistance screening and/or using growth-based selection; and/or
wherein identifying the engineered peptide-binding protein as having increased specificity for the compound in step (e) comprises a step of determining the sequence of the nucleic acid encoding the peptide-binding protein and/or determining specific mutations in the nucleic acid encoding the peptide-binding protein.

7. The method according to any one of claims 3 to 6, wherein the isopeptide-linked lysine or the analog thereof is obtained by intracellular cleavage of the compound of any one of claims 1 to 3, in particular wherein the isopeptide-linked lysine or the analog thereof has the following structure: 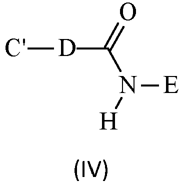 wherein C' is -NH₂ or -OH, and wherein D and E is defined as in any one of claims 1 to 3, wherein the left empty valence in D is connected to C'.

8. An engineered variant of an oligopeptide-binding protein OppA from E. coli (SEQ ID NO:1) comprising mutations in one or more of the following positions: L78, T173, V193, D221, W222, I303, K307, K333, N337, K371, R439, T429, S460, and/or V482.

9. The engineered variant of claim 8, wherein the one or more mutations are located in positions: T173, V193, D221, W222, I303, N337, R439, and/or S460, in particular wherein the one or more mutations are located in positions: D221, W222, N337, R439, and/or S460, in particular wherein the one or more mutations are located in positions: D221, W222, R439, and/or S460, in particular wherein the one or more mutations are located in positions: R439, and/or S460.

10. A nucleic acid encoding the engineered variant of claim 8 or 9.

11. A vector comprising the nucleic acid according to claim 10.

12. A cell comprising the nucleic acid according to claim 10 or the vector according to claim 11, in particular wherein the cell is a prokaryotic cell, in particular an E. coli cell.

13. A method for incorporating a non-canonical amino acid, or an analog thereof, into a protein, the method comprising the steps of:
a) providing a cell comprising at least one orthogonal translation system, wherein the at least one orthogonal translation system comprises an orthogonal aminoacyl-tRNA synthetase/tRNA pair specific for a non-canonical amino acid, or an analog thereof, comprised in the compound according to an one of claims 1 to 3, and a nucleic acid molecule encoding the protein, wherein the nucleic acid molecule encoding the protein comprises one or more codons that have been re-allocated for the incorporation of the non-canonical amino acid, or an analog thereof, by the orthogonal aminoacyl-tRNA synthetase/tRNA pair;
b) contacting the cell of step (a) with the compound according to any one of claims 1 to 3, wherein the compound is transported into the cell and hydrolyzed inside the cell to release an isopeptide-linked lysine or an analog thereof and, optionally, another non-canonical amino acid or an analog thereof;
c) incorporating (i) the isopeptide-linked lysine or an analog thereof and/or (ii) the other non-canonical amino acid or an anlaog thereof into the protein in response to the one or more re-allocated codon using the orthogonal translation system.

14. The method according to claim 13, wherein the cell is a prokaryotic cell, in particular an E. coli cell; and/or
wherein the cell expresses an oligopeptide permease, in particular wherein the oligopeptide permease comprises a peptide-binding protein that has been engineered for increased specificity for the compound according to any one of claims 1 to 3, in particular wherein the engineered peptide-binding protein is OppA of E. coli (SEQ ID NO:1) comprising mutations in one or more of the following positions: L78, T173, V193, D221, W222, I303, K307, K333, N337, K371, R439, T429, S460, and/or V482; and/or
wherein the orthogonal aminoacyl-tRNA synthetase/tRNA pair is a Pyrrolysyl-tRNA synthetase/tRNA pair; and/or
wherein the nucleic acid molecule encoding the protein comprises one or more amber stop codons that have been re-allocated to incorporate the non-canonical amino acid or an analog thereof; and/or
wherein hydrolysis of the molecule according to any one of claims 1 to 3 inside the cell releases a second non-canonical amino acid (ncAA) or analog thereof, and wherein the second ncAA or analog thereof is incorporated into the same or a different protein in response to a re-allocated codon.

15. The method according to any one of claims 13 or 14, wherein the isopeptide-linked lysine or the analog thereof has the following structure: 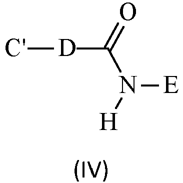 wherein C' is -NH₂ or -OH, and wherein D and E is defined as in any one of claims 1 to 3, wherein the left empty valence in D is connected to C'.
